(19) Europäisches Patentamt
European Patent Office
Office européen des brevets

(11) Publication number: **0 042 154**
**B1**

(12) **EUROPEAN PATENT SPECIFICATION**

(45) Date of publication of patent specification: **15.04.87**

(21) Application number: **81104520.2**

(22) Date of filing: **12.06.81**

(51) Int. Cl.[4]: **C 07 D 501/46,** A 61 K 31/545 // C07D285/08, C07D213/81, C07D209/48

(54) **New cephem compounds and processes for preparation thereof.**

(30) Priority: **18.06.80 US 160904**
**01.12.80 GB 8038456**
**31.12.80 GB 8041636**
**09.04.81 GB 8111164**

(43) Date of publication of application:
**23.12.81 Bulletin 81/51**

(45) Publication of the grant of the patent:
**15.04.87 Bulletin 87/16**

(84) Designated Contracting States:
**BE CH DE FR GB IT LI LU NL SE**

(56) References cited:
**EP-A-0 013 762**
**EP-A-0 025 017**
**EP-A-0 027 599**
**EP-A-0 047 977**
**FR-A-2 137 899**
**US-A-4 165 430**

**The file contains technical information submitted after the application was filed and not included in this specification**

(73) Proprietor: **Fujisawa Pharmaceutical Co., Ltd.**
**3, Doshomachi 4-chome Higashi-ku**
**Osaka-shi, Osaka 541 (JP)**

(72) Inventor: **Teraji, Tsutomu**
**No. 6-20-6, Kofudai Toyono-cho**
**Toyono-gun Osaka-fu (JP)**
Inventor: **Sakane, Kazuo**
**No. 6-60-5, Higashi-sonoda-cho**
**Amagasaki (JP)**
Inventor: **Goto, Jiro**
**A-401 Senridai-sukai-taun No. 21 Kashikiriyama**
**Suita (JP)**

(74) Representative: **Türk, Gille, Hrabal**
**Bruckner Strasse 20**
**D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 042 154 B1**

**Description**

Cephem compounds are known from FR—A—2 137 899, EP—A—13 762 and EP—A—27 599. The present compounds are a selection from the broad range of compounds disclosed in FR—A—2 137 899. The compounds of EP—A—13 762 and EP—A—27 599 are structurally similar to the present compounds.

The present invention relates to new cephem compounds and pharmaceutically acceptable salts thereof. More particularly, it relates to new cephem compounds and pharmaceutically acceptable salts thereof, which have antimicrobial activities and to processes for the preparation thereof, to pharmaceutical composition comprising the same, which can be used therapeutically in the treatment of infectious diseases in human beings and animals.

Accordingly, it is one object of the present invention to provide new cephem compounds and pharmaceutically acceptable salts thereof, which are active against a number of pathogenic miroorganisms.

Another object of the present invention is to provide processes for the preparation of new cephem compounds and pharmaceutically acceptable salts thereof.

A further object of the preent invention is to provide pharmaceutical composition comprising, as active ingredients, said new cephem compounds and pharmaceutically acceptable salts thereof, which can be used for the treatment of infectious diseases caused by pathogenic bacteria in human beings and animals.

The object new cephem compounds are novel and can be represented by the following general formula (I).

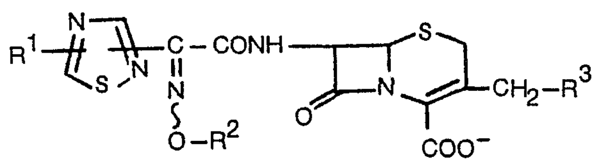

(I)

wherein $R^1$ is amino or a protected amino group,

$R^2$ is $(C_{1-6})$aliphatic hydrocarbon group which may be substituted with substituent(s) selected from the group consisting of carboxy and protected carboxy, cyclo$(C_{3-6})$alkyl or cyclo$(C_{3-6})$alkenyl, and

$R^3$ is a thiazolio group which may be substituted with substituent(s) selected from the group consisting of $(C_{1-6})$alkyl and hydroxy$(C_{1-6})$alkyl:

a pyridinio group substituted with substituent(s) selected from the group consisting of halogen, cyano, hydroxy, amino protected in a usual manner by acyl, $(C_{1-6})$alkanoyl, hydroxycarbamoyl, $(C_{1-14})$alkylcarbamoyl, protected carboxy, $(C_{1-6})$alkyl, hydroxy$(C_{1-6})$alkyl, amino$(C_{1-6})$alkyl, protected amino$(C_{1-6})$alkyl, carboxy$(C_{1-6})$alkyl and hydroxymino$(C_{1-6})$alkyl,

or a pyridinio group substituted with amino: with proviso that, when $R^2$ is $(C_{1-6})$alkyl, $R^3$ is not a pyridinio group substituted with carboxy$(C_{1-6})$alkyl; and when $R^2$ is 2-carboxyprop-2-yl, $R^3$ is not carboxymethyl pyridinio; or pharmaceutically acceptable salts thereof.

compounds (I) can be prepared by various processes which are illustrated in the following scheme.

Process 1

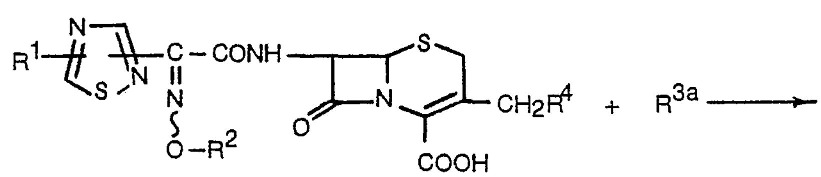

(II) (III)

or a salt thereof

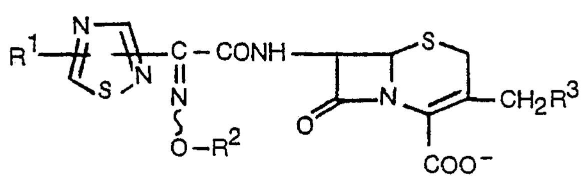

(I)

or a salt thereof

Process 2

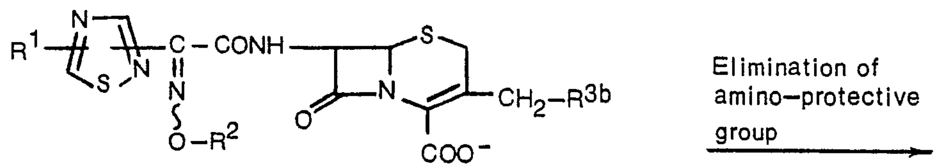


(Ia)

or a salt thereof

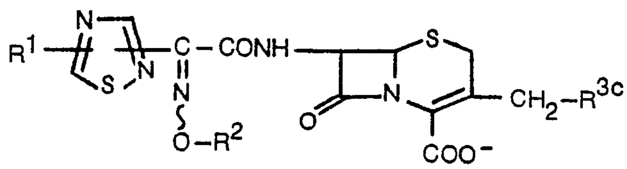

(Ib)

or a salt thereof

Process 3

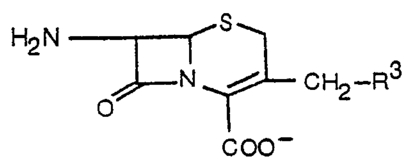

+

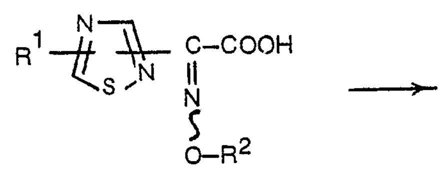

(IV)

or its reactive derivative at the amino group or a salt thereof.

(V)

or its reactive derivative at the carboxy group or a salt thereof

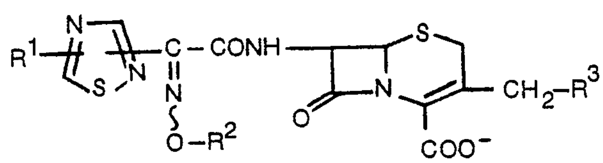

(I)

or a salt thereof

Process 4

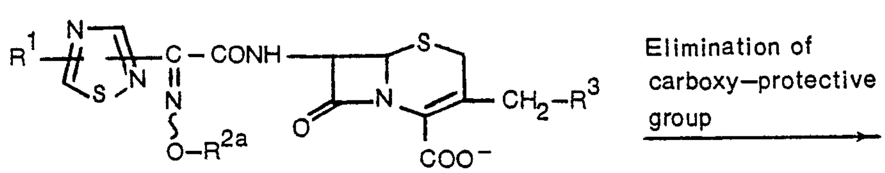


(Ic)

or a salt thereof

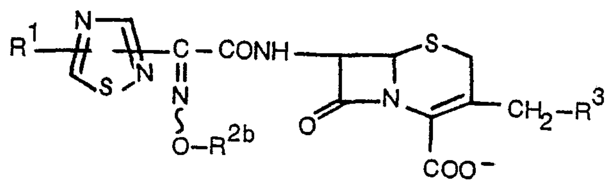

(Id)

or a salt thereof

Process 5

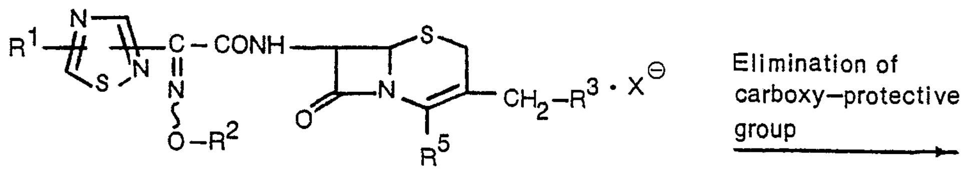


(VI)

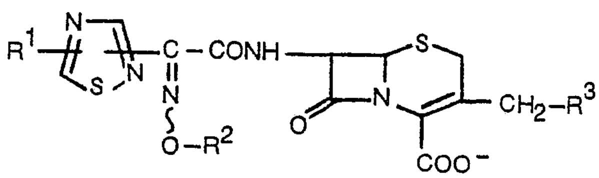

(I)

or a salt thereof

wherein $R^1$, $R^2$ and $R^3$ are each as defined above;

$R^4$ is a group which can be replaced by a group of the formula: $R^3$ wherein $R^3$ is as defined above;

$R^{3a}$ is thiazole which may be substituted with substituent(s) selected from the group consisting of $C_1$ to $C_6$alkyl and hydroxy ($C_1$ to $C_6$)alkyl; pyridine substituted with substituent(s) selected from the group consisting of halogen, cyano, hydroxy, amino protected in a usual way by acyl, $C_1$ to $C_6$alkanoyl, hydroxycarbamoyl, ($C_1$ to $C_{14}$)alkylcarbamoyl, protected carboxy, $C_1$ to $C_6$alkyl, hydroxy($C_1$ to $C_6$)alkyl, protected amino($C_1$ to $C_6$)alkyl, amino($C_1$ to $C_6$)alkyl, carboxy($C_1$ to $C_6$)alkyl and hydroxyimino($C_1$ to $C_6$)alkyl; or pyridine substituted with amino; with proviso that, when $R^2$ is ($C_1$ to $C_6$)alkyl, $R^{3a}$ is not pyridine substituted with carboxy($C_1$ to $C_6$)alkyl and when $R^2$ is carboxyprop-2-yl,

$R^{3a}$ is not 3-carboxymethylpyridine;

$R^{3b}$ is a pyridinio group substituted with protected amino($C_1$ to $C_6$)alkyl;

$R^{3c}$ is a pyridinio group substituted with amino($C_1$ to $C_6$)alkyl;

$R^{2a}$ is protected carboxy ($C_1$ to $C_6$)alkyl;

$R^{2b}$ is carboxy ($C_1$ to $C_6$)alkyl;

$R^5$ is a protected carboxy group; and

X is an acid residue.

Among the starting compounds of the present invention, the compound (VI) is novel and can be prepared by the following methods.

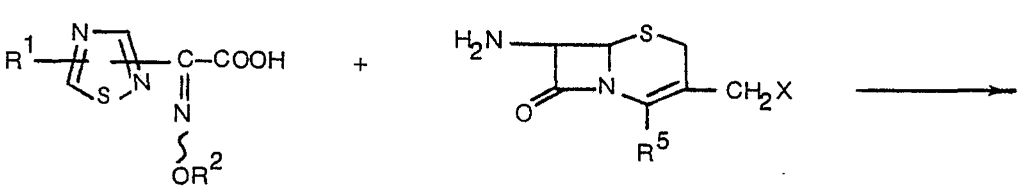

(V) (VII)

or its reactive derivative at the carboxy group or a salt thereof

or its reactive derivative at the amino group or a salt thereof

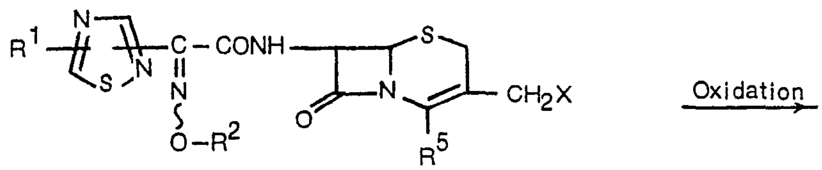


(VIII)

or a salt thereof

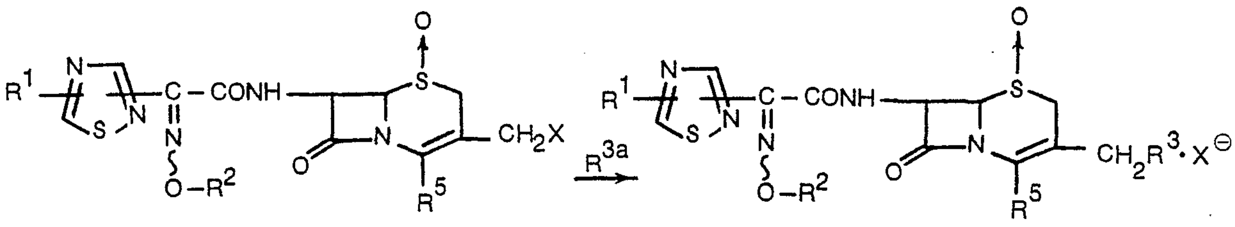


(IX)

or a salt thereof

(X)

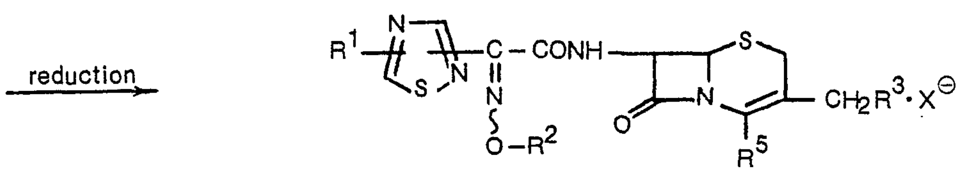


(VI)

wherein $R^1$, $R^2$, $R^3$, $R^{3a}$, $R^5$ and X are each as defined above.

Regarding the object compounds (I), (Ia), (Ib), (Ic) and (Id) and the starting compounds (II), (V), (VIII), (VI), (IX) and (X), it is to be understood that said object and starting compounds include syn isomer, anti isomer and a mixture thereof. For example, with regard to the object compound (I), syn isomer means one geometrical isomer having the partial structure represented by the following formula:

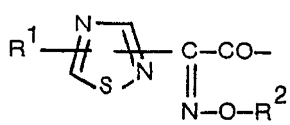

(wherein $R^1$ and $R^2$ are each as defined above) and anti-isomer means the other geometrical isomer having the partial structure represented by the following formula:

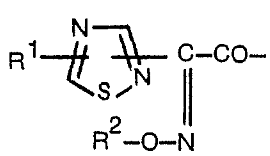

(wherein $R^1$ and $R^2$ are each as defined above).

Regarding the other object compounds and starting compounds as mentioned above, th syn isomer and the anti isomer can also be referred to the same geometrical isomers as illustrated for the compound (I).

Suitable pharmaceutically acceptable salts of the object compounds (I) are conventional non-toxic salts and include e.g. a metal salt such as an alkali metal salt (e.g. sodium salt, potassium salt) and an alkaline earth metal salt (e.g. calcium salt, magnesium salt), an ammonium salt, an organic base salt (e.g. trimethylamine salt, triethylamine salt, pyridine salt, picoline salt, dicyclohexylamine salt, N,N'-dibenzylethylenediamine salt), an organic acid salt (e.g. acetate, maleate, tartrate, methanesulfonate, benzenesulfonate, formate, toluenesulfonate), an inorganic acid salt (e.g. hydrochloride, hydrobromide, hydroiodide, sulfate, phosphate), or a salt with an amino acid (e.g. arginine, aspartic acid, glutamic acid).

In the above and subsequent descriptions of the present specification, suitable examples and illustrations of the various definitions which the present invention include within the scope thereof are explained in details as follows.

The term "lower" is intended to mean 1 to 6 carbon atoms, unless otherwise indicated.

Suitable "protected amino" for $R^1$ and "protected amino" moiety in the term "protected amino(lower)alkyl" may include an acylamino ro an amino group substituted by a conventional protecting group such as ar(lower)alkyl which may have at least one suitable substituent(s), (e.g. benzyl, trityl).

Suitable acyl moiety in the term "amino, protected in a usual manner by acyl" may include carbamoyl, aliphatic acyl group and acyl group containing an aromatic or heteroxyclic ring. And suitable examples of the said acyl may be lower alkanoyl (e.g. formyl, acetyl, propionyl, butyryl, isobutyryl, valeryl, isovaleryl, oxalyl, succinyl, pivaloyl); lower alkoxycarbonyl having 2 to 7 carbon atoms (e.g. methoxycarbonyl, ethoxycarbonyl, propoxycarbonyl 1-cyclopropylethoxycarbonyl, isopropoxycarbonyl, butoxycarbonyl, tertiarybutoxycarbonyl, pentyloxycarbonyl, hexyloxycarbonyl);
lower alkanesulfonyl (e.g. mesyl, ethanesulfonyl, propanesulfonyl, isopropanesulfonyl, butanesulfonyl); arenesulfonyl (e.g. benzenesulfonyl, tosyl); aroyl (e.g. benzoyl, toluoyl, xyloyl, naphthoyl, phthaloyl, indancarboxyl);
ar(lower)alkanoyl (e.g. phenylacetyl, phenylpropionyl); and
ar(lower)alkoxycarbonyl (e.g. benzyloxycarbonyl phenethyloxycarbonyl). The acyl moiety as stated above may have at least one suitable substituent(s) such as halogen (chlorine, bromine, fluorine and iodine) or lower alkanoyl.

Suitable lower aliphatic hydrocarbon group may include e.g. lower alkyl, lower alkenyl and lower alkynyl.

Suitable "cyclo(($C_{3-6}$)alkyl" for $R^2$ may include for example, cyclopropyl, cyclobutyl, cyclopentyl or cyclohexyl, preferably one having 4 to 6 carbon atoms.

Suitable "cyclo($C_{3-6}$)alkenyl" for $R^2$ may include, for example, cyclopentenyl or cyclohexenyl, preferably one having 5 to 6 carbon atoms.

Suitable "$C_1$ to $C_6$ alkyl" and "$C_1$ to $C_6$ alkyl" moiety in the terms "hydroxy($C_1$ to $C_6$)alkyl", "protected amino ($C_1$ to $C_6$)alkyl", "protected carboxy($C_1$ to $C_6$)alkyl" and "carboxy($C_1$ to $C_6$)alkyl" may include e.g. methyl, ethyl, propyl, isopropyl, butyl, isobutyl, tert-butyl, pentyl, tert-pentyl, hexyl, and preferably one having 1 to 4 carbon atom(s).

Suitable "lower alkenyl" is one having 2 to 6 carbon atoms and may include e.g. vinyl, allyl, isopropenyl, 1-propenyl, 2-butenyl and 3-pentenyl.

Suitable "lower alkynyl" is one having 2 to 6 carbon atoms and may include e.g. ethynyl, 2-propynyl, 2-butynyl, 3-pentynyl and 3-hexynyl.

The lower aliphatic hydrocarbon group as mentioned above may be substituted e.g. with 1 to 3 substituent(s) selected from carboxy, protected carboxy as mentioned below or halogen (e.g. chlorine, bromine).

Suitable substituent(s) on a thiazolio group for $R^3$ and thiazole for $R^{3a}$ may include e.g. methyl, ethyl, propyl, isopropyl, butyl, isobutyl, tert-butyl, pentyl, tert-pentyl, hexyl, hydroxymethyl, hydroxyethyl hydroxypropyl and hydroxybutyl, and the number of the substituent(s) may be 1 to 3.

A pyridinio group for $R^3$ or pyridine for $R^{3a}$ is substituted with 1 to 3 substituent(s) selected from the group consisting of halogen, cyano, hydroxy, amino protected in a usual manner by acyl, ($C_{1-6}$)alkanoyl, hydroxycarbamoyl; ($C_{1-14}$)alkylcarbamoyl, protected carboxy, ($C_{1-6}$)alkyl, hydroxy($C_{1-6}$)alkyl, amino($C_{1-6}$)alkyl, protected amino($C_{1-6}$)alkyl, carboxy($C_{1-6}$)alkyl, hydroxyimino($C_{1-6}$)alkyl and amino.

Suitable "halogen" may include chlorine, bromine, fluorine and iodine.

Suitable "lower alkanoyl" can be referred to the ones as exemplified for aforesaid "acyl".

Suitable "alkyl" moiety in the term "($C_{1-14}$)alkylcarbamoyl" may include e.g. methyl, ethyl, propyl, isopropyl, butyl, isobutyl, t-butyl, pentyl, t-pentyl, hexyl, heptyl, octyl, nonyl, decyl, undecyl, dodecyl, tridecyl, tetradecyl and preferably one having 1 to 12 carbon atoms(s).

Suitable "protected carboxy" and "protected carboxy" moiety in the term "protected carboxy($C_{1-6}$)alkyl" may include e.g. esterified carboxy in which said ester may be the ones such as alkyl ester (e.g. methyl ester, ethyl ester, propyl ester, isopropyl ester, butyl ester, isobutyl ester, t-butyl ester, pentyl ester, t-pentyl ester, hexyl ester, heptyl ester, octyl ester, nonyl ester, decyl ester, undecyl ester, dodecyl ester, hexadecyl ester);

lower alkenyl ester (e.g. vinyl ester, allyl ester); lower alkynyl ester (e.g. ethynyl ester, propynyl ester); mono(or di or tri)-halo(lower)alkyl ester (e.g., 2-iodoethyl ester, 2,2,2-trichloroethyl ester); lower alkanoyloxy(lower)alkyl ester (e.g. acetoxymethyl ester, propionyloxymethyl ester, 1-acetoxypropyl ester, valeryloxymethyl ester, pivaloyloxymethyl ester, hexanoyloxymethyl ester, 1-acetoxyethyl ester, 2-propionyloxyethyl ester, 1-isobutyryloxyethyl ester); lower alkanesulfonyl(lower)alkyl ester (e.g., mesylmethyl ester, 2-mesylethyl ester); ar(lower)alkyl ester, for example, phenyl(lower)alkyl ester which may be substituted with one or more suitable substituent(s) (e.g. benzyl ester, 4-methoxybenzyl ester, 4-nitrobenzyl ester, phenethyl ester, trityl ester, diphenylmethyl ester, bis(methoxyphenyl)methyl ester, 3,4-dimethoxybenzyl ester, 4-hydroxy-3,5-ditertiarybutylbenzyl ester);

lower alkoxycarbonyloxy(lower)alkyl ester (e.g. methoxycarbonyloxymethyl ester, ethoxycarbonyloxymethyl ester, ethoxycarbonyloxyethyl ester) which may be substituted with azido;

a heterocyclic ester, preferably benzotetrahydrofuryl ester which may be substituted with oxo group, more preferably phthalidyl ester;
aroyloxy(lower)alkyl ester (e.g. benzoyloxymethyl ester, benzoyloxyethyl ester, toluoyloxyethyl ester); and aryl ester which may have one or more suitable substituent(s) (e.g. phenyl ester, tolyl ester, tertiarybutylphenyl ester, xylyl ester, mesityl ester, cumenyl ester).

Preferable example of "protected carboxy" may be alkoxycarbonyl (e.g. methoxycarbonyl, ethoxycarbonyl, propoxycarbonyl, isopropoxycarbonyl, butoxycarbonyl, t-butoxycarbonyl, pentyloxycarbonyl, hexyloxycarbonyl, heptyloxycarbonyl, octyloxycarbonyl, nonyloxycarbonyl, decyloxycarbonyl, undecyloxycarbonyl, dodecyloxycarbonyl, hexadecyloxycarbonyl).

Suitable $R^4$ may include an acid residue such as acyloxy, azido or halogen, wherein acyl moiety in the term "acyloxy" and halogen can be referred to the ones as exemplified above.

Suitable X may include acid residues as above.

Preferred embodiments of the object compound (I) are as follows.

Preferred embodiment of $R^1$ is amino;

$R^2$ is $C_{1-6}$ alkyl (most preferably ethyl), cyclo $(C_{3-6})$alkyl (most preferably cyclopentyl), cyclo$(C_{3-6})$alkenyl (most preferably cyclopentenyl), carboxy$(C_{1-6})$alkyl or esterified carboxy$(C_{1-6})$alkyl (more preferably $C_{1-6}$ alkoxycarbony$(C_{1-6})$alkyl);

$R^3$ is thiazolio, thiazolio substituted with $(C_{1-6})$alkyl and hydroxy$(C_{1-6})$alkyl, or pyridinio substituted with 1 to 2 substituent(s) selected from the group consisting of halogen, cyano, hydroxy, $(C_{1-6})$ alkanoyl (most preferably acetyl), amino protected in a usual manner by acyl (more preferably $(C_{1-6})$alkanesulfonylamino, most preferably mesylamino), amino hydroxycarbamoyl, $(C_{1-14})$alkylcarbamoyl, $(C_{1-6})$alkoxycarbonyl, $(C_{1-6})$alkyl, hydroxy$(C_{1-6})$alkyl, carboxy$(C_{1-6})$alkyl, amino$(C_{1-6})$alkyl, $(C_{1-6})$alkoxycarbonylamino$(C_{1-6})$alkyl, and hydroxyimino$(C_{1-6})$alkyl.

The processes for preparing the object compounds of the present invention are explained in details in the following.

Process 1

The object compound (I) or a salt thereof can be prepared by reacting the compound (II) or a salt thereof with the compound (III).

Suitable salts of the compound (II) can be referred to the ones exemplified for the compound (I).

The present reaction may be carried out in a solvent such as water, phosphate buffer, acetone, chloroform, acetonitrile, nitrobenzene, methylene chloride, ethylene chloride, formamide, dimethylformamide, methanol, ethanol, ether, tetrahydrofuran, dimethylsulfoxide, or any other organic solvent which does not adversely affect the reaction, preferably in ones having strong polarities. Among the solvents, hydrophilic solvents may be used in a mixture with water. The reaction is preferably carried out in around neutral medium. When the compound (II) is used in a free form, the reaction is preferably conducted in the presence of a base, for example, inorganic base such as alkali metal hydroxide, alkali metal carbonate, alkali metal bicarbonate and organic base such as trialkylamine. The reaction temperature is not critical, and the reaction is usually carried out at ambient temperature, under warming or under heating. The present reaction is preferably carried out in the presence of e.g. alkali metal halide (e.g. sodium iodide, potassium iodide) and alkali metal thiocyanate (e.g. sodium thiocyanate, potassium thiocyanate).

Process 2

The object compound (Ib) or a salt thereof can be prepared by subjecting the compound (Ia) or a salt thereof to elimination reaction of the protective group of amino.

Suitable salts of the compounds (Ia) and (Ib) can be referred to the ones exemplified for the compound (I).

The present elimination reaction is carried out in accordance with a conventional method such as hydrolysis; reduction; or a method by reacting the compound (Ia) wherein the protective group is acyl group with iminohalogenating agent and then with iminoetherifying agent, and, if necessary, subjecting the resulting compound to hydrolysis. The hydrolysis may include e.g. a method using an acid or base or hydrazine. These methods may be selected depending on the kind of the protective groups to be eliminated.

Among these methods, hydrolyssis using an acid is one of the common and preferable method for eliminating the protective group such as substituted or unsubstituted alkoxycarbonyl (e.g. t-pentyloxycarbonyl, t-butoxycarbonyl), alkanoyl (e.g. formyl), cycloalkoxycarbonyl, substituted or unsubstituted aralkoxycarbonyl (e.g. benzyloxycarbonyl, substituted, benzyloxycarbonyl) and ar(lower)alkyl (e.g benzyl, trityl).

Suitable acids may include an organic or an inorganic acid, for example, formic acid, trifluoroacetic acid, benzenesulfonic acid, p-toluenesulfonic acid, hydrochloric acid and preferred acids are, for example, formic acid, trifluoroacetic acid and hydrochloric acid. The acid suitable for the reaction can be selected according to the kind of protective group to be eliminated. When the elimination reaction is conducted with the acid, it can be carried out in the presence or absence of a solvent. Suitable solvent may include a conventional organic solvent, water or a mixture thereof. When trifluoroacetic acid is used, the elimination

reaction may preferably be carried out in the presence of anisole.

The hydrolysis using hydrazine is commonly applied for eliminating the protective group, for example, succinyl or phthaloyl.

The hydrolysis with a base is preferably applied for eliminating an acyl group, for example, haloalkanoyl (e.g. dichloroacetyl, trifluoroacetyl). Suitable base may include, for example, an inorganic base such as an alkali metal hydroxide (e.g. sodium hydroxide, potassium hydroxide), alkaline earth metal hydroxide (e.g. magnesium hydroxide, calcium hydroxide), alkali metal carbonate (e.g. sodium carbonate, potassium carbonate), alkaline earth metal carbonate (e.g. magnesium carbonate, calcium carbonate), alkali metal bicarbonate (e.g. sodium bicarbonate, potassium bicarbonate), alkali metal acetate (e.g. sodium acetate, potassium acetate,), alkaline earth metal phosphate (e.g. magnesium phosphate, calcium phosphate) or alkali metal hydrogen phosphate (e.g. disodium hydrogen phosphate, dipotassium hydrogen phosphate), and an organic base such as a trialkylamine (e.g. trimethylamine, triethylamine), picoline, N-methylpyrrolidine, N-methylmorpholine, 1,5-diazabicyclo[4,3,0]-non-5-ene, 1-4-diazabicyclo[2,2,2]octane or 1,5-diazabicyclo[5,4,0]undecene-5. The hydrolysis using a base is often carried out in water, a conventional organic solvent or a mixture thereof.

Among the protective groups, the acyl group can be generally eliminated by hydrolysis as mentioned above or by the other conventional hydrolysis. In case that the acyl group is halogen substituted-alkoxycarbonyl or 8-quinolyloxycarbonyl, they are eliminated by treating with a heavy metal such as copper, zinc or the like.

The reductive elimination is generally applied for eliminating the protective group, for example, haloalkoxycarbonyl (e.g. trichloroethoxycarbonyl), substituted or unsubstituted aralkoxycarbonyl (e.g. benzyloxycarbonyl, substituted benzyloxycarbonyl), 2-pyridylmethoxycarbonyl. Suitable reduction may include, for example, reduction with an alkali metal borohydride (e.g. sodium borohydride).

The reaction temperature is not critical and may be suitably selected in accordance with the kind of the protective group of the amino group and the elimination method as mentioned above, and the present reaction is preferably carried out under a mild condition such as under cooling, at ambient temperature or slightly elevated temperature.

The present reaction includes, within its scope, the cases that the protected amino group for $R^1$ is transformed into the free amino group in the course of the elimination reaction as mentioned above or in the post-treatment of the reaction mixture of reaction product.

Process 3

The object compound (I) or a salt thereof can be prepared by reacting the compound (IV) or its reactive derivative at the amino group or a salt thereof with the compound (V) or its reactive derivative at the carboxy group or a salt thereof.

Suitable reactive derivatives at the amino group of the compound (IV) may include a conventional reactive derivative used in amidation, for example, Schiff's base type imino or its tautomeric enamine type isomer formed by the reaction of the compound (IV) with a carbonyl compound; a silyl derivative formed by the reaction of the compound (IV) with a silyl compound such as bis(trimethylsilyl)acetamide, trimethylsilylacetamide or the like; a derivative formed by reaction of the compound (IV) with phosphorus trichloride or phosgene.

Suitable salts of the compound (IV) may include, e.g., an acid addition salt such as an organic acid salt (e.g. acetate, maleate, tartrate, benzenesulfonate, toluenesulfonate) or an inorganic acid salt (e.g. hydrochloride, hydrobromide, sulfate, phosphate); a metal salt (e.g. sodium salt, potassium salt, calcium salt, magnesium salt); ammonium salt; and an organic amine salt (e.g. triethylamine salt, dicylohexylamine salt).

Suitable reactive derivatives at the carboxy group of the compound (V) may include e.g. an acid halide, an acid anhydride, an activated amide and an activated ester. The suitable example may be an acid chloride; an acid azide; a mixed acid anhydride with an acid such as substituted phosphoric acid (e.g. dialkylphosphoric acid, phenylphosphoric acid, diphenylphosphoric acid, dibenzylphosphoric acid, halogenated phosphoric acid), dialkylphosphorous acid, sulfurous acid, thiosulfuric acid, sulfuric acid, alkylcarbonic acid, aliphatic carboxylic acid (e.g. pivalic acid, pentanoic acid, isopentanoic acid, 2-ethylbutyric acid, acetic acid or trichloroacetic acid) or aromatic carboxylic acid (e.g. benzoic acid); a symmetrical acid anhydride; an activated amide with imidazole, dimethylpyrazole, triazole or tetrazole; or an activated ester (e.g. cyanomethyl ester, methoxymethyl ester, dimethyliminomethyl $[(CH_3)_2\overset{+}{N}=CH-]$ ester, vinyl ester, propargyl ester, p-nitrophenyl ester, 2,4-dinitrophenyl ester, trichlorophenyl ester, pentachlorophenyl ester, mesyl phenyl ester, phenylazophenyl ester, phenyl thioester, p-nitrophenyl thioester, p-cresyl thioester, carboxymethyl thioester, pyranyl ester, phyridyl ester, piperidyl ester, 8-quinolyl thioester, or an ester with N,N-dimethylhydroxylamine, 1-hydroxy-2-(1H)-pyridone, N-hyroxysuccinimide, N-hydroxyphthalimide or 1-hydroxy-6-chloro-1H-benzotriazole. These reactive derivatives can be optionally selected from them according to the kind of the compound (V) to be used.

The salts of the compound (V) may be salts with an inorganic base such as an alkali metal salts (e.g. sodium or potassium salt), or an alkaline earth metal salt (e.g. calcium or magnesium salt), a salt with an

organic base such as trimethylamine, triethylamine, pyridine or a salt with an acid (e.g. hydrochloric acid or hydrobromic acid).

The reaction is usually carried out in a conventional solvent such as water, acetone, dioxane, acetonitrile, chloroform, methylene chloride, ethylene chloride, tetrahydrofuran, ethyl acetate, N,N-dimethylformamide, pyridine or any other organic solvent which does not adversely influence to the reaction. Among these solvents, hydrophilic solvents may be used in a mixture with water.

When the compound (V) is used in free acid form or its salt form in the reaction, the reaction is preferably carried out in the presence of a conventional condensing agent such as N,N-dicyclohexylcarbodiimide; N-cyclohexyl-N'-morpholinoethylcarbodiimide; N-cyclohexyl-N'-(4-diethylaminocyclohexyl)carbodiimide; N,N-diethylcarbodiimide; N,N,diisopropylcarbodiimide; N-ethyl-N'-(3-dimethylaminopropyl)carbodiimide; N,N-carbonylbis(2-methylimidazole); pentamethylene-ketene-N-cyclohexylimine; diphenylketene-N-cyclohexylimine; ethoxyacetylene; ethyl polyphosphate; isopropyl polyphosphate; diethyl phosphorochloridite; phosphorus oxychloride; phosphorus trichloride; phosphorus pentachloride; thionyl chloride; oxalyl chloride; triphenylphosphine; N-ethyl-7-hydroxybenzisoxazolium fluoroborate; N-ethyl-5-phenylisoxazolium-3'-sulfonate; 1-(p-chlorobenzenesulfonyloxy)-6-chloro-1H-benzotriazole; so-called Vilsmeier reagnet, for example (chloromethylene) dimethylammonium chloride produced by the reaction of dimethylformamide with thionyl chloride or phosgene or a compound produced by the reaction of dimethylformamide with e.g. phosphorus oxychloride.

The reaction may be also carried out in the presence of an inorganic or an organic base such as an alkali metal hydroxide, an alkali metal bicarbonate, alkali metal carbonate, alkali metal acetate, tri(lower)alkylamine, pyridine, N-(lower)alkylmorpholine, N,N-di(lower)alkylbenzylamine or N,N-di(lower)alkylaniline as exemplified below. When the base or the condensing agent is in liquid, it can be used also as a solvent. The reaction temperature is not critical, and the reaction is usually carried out under cooling or at ambient temperature.

In the present reaction, a syn-isomer of the object compound (I) can be obtained preferably by conducting the reaction of the compound (IV) with a synisomer of the starting compound (V).

Process 4

The object compound (Id) or a salt thereof can be prepared by subjecting the compound (Ic) or a salt thereof to elimination reaction of the protective group of carboxy.

Suitable salts of the compounds (Ic) and (Id) are the ones exemplified for the compound (I).

The present reaction is carried out in accordance with a conventional method such as hydrolysis or reduction.

In the case that the protective group is an ester, the protective group can be eliminated by hydrolysis. Hydrolysis is preferably carried out in the presence of a base or an acid. Suitable bases may include an inorganic base and an organic base such as an alkali metal (e.g. sodium, potassium), an alkaline earth metal (e.g. magnesium, calcium), the hydroxide or carbonate or bicarbonate thereof, trialkylamine (e.g. trimethylamine, triethylamine), picoline, 1,5-diazabicyclo[4,3,0]none-5-ene, 1,4-diazabicyclo[2,2,2]octane or 1,8-diazabicyclo[5,4,0]undecene-7. Suitable acids may include an organic acid (e.g. formic acid, acetic acid, propionic acid, trifluoroacetic acid) and an inorganic acid (e.g. hydrochloric acid, hydrobromic acid, sulfuric acid).

The reaction is usually carried out in a solvent such as water, an alcohol (e.g. methanol, ethanol,), a mixture thereof or any other solvent which does not adversely influence to the reaction. A liquid base or acid can be also used as the solvent. The reaction temperature is not critical and the reaction is usually carried out under cooling to warming.

Reduction can be applied preferably for elimination of the protective group such as 4-nitrobenzyl, 2-iodoethyl or 2,2,2-trichloroethyl. The reduction method applicable for the elimination reaction may include, for example, reduction by using a combination of a metal (e.g. zinc, zinc amalgam) or a salt of chrome compound (e.g. chromous chloride, chromous acetate) and an organic or inorganic acid (e.g. acetic acid, propionic acid, hydrochloric acid); and conventional catalytic reduction in the presence of a conventional metallic catalyst (e.g. palladium-carbon).

Process 5

The compound (I) or a salt thereof can be prepared by subjecting the compound (VI) to an elimination reaction of a carboxy-protective group.

The present elimination reaction can be carried out according to a similar manner to that of Process 4.

The Preparations of the starting compound (VI) are explained in detail in the following.

Preparation 1

The compound (VIII) can be prepared by reacting the compound (V) or its reactive derivative at the carboxy group or a salt thereof with the compound (VII) or its reactive derivative at the amino group or a salt thereof.

The present reaction can be carried out in a similar manner to that of Process 3.

Preparation 2

The compound (IX) or a salt thereof can be prepared by oxidizing the compound (VIII) or a salt thereof.

Suitable oxidizing agents to be used in this reaction may include all oxidizing agent which can oxidize the —S— group in cephalosporin compounds to the

$$-\overset{\displaystyle O}{\overset{\uparrow}{S}}-$$

group, for example, peroxide (e.g., hydrogen peroxide, 3-chloroperbenzoic acid).

The reaction is usually carried out in solvent such as methylene chloride or any other solvent which does not adversely affect the reaction. The reaction temperature is not critical but is preferably effected under cooling or at ambient temperature.

Preparation 3

The compound (X) can be prepared by reacting the compound (IX) or a salt thereof with the compound $R^{3a}$.

The reaction can be carried out according to a similar manner to that of Process 1.

Preparation 4

The compound (VI) can be prepared by reducing the compound (X).

The present reduction can be carried out in the presence of conventional reducing agent which can reduce

$$-\overset{\displaystyle O}{\overset{\uparrow}{S}}-$$

group in cephalosporin compound to —S— group, for example, phosphorus halide such as phosphorus trihalide (e.g., phosphorus trichloride).

The object compound (I) of the present invention exhibits high antimicrobial activity and inhibits the growth of a number of microorganisms including pathogenic Gram-positive and Gram-negative bacteria.

For therapeutic administration, the cephalosporin compounds according to the present invention are used in the form of pharmaceutical preparations which contain said compounds in admixture with a pharmaceutically acceptable carriers such as an organic or inorganic solid or liquid excipient suitable for oral, parenteral or external administration. The pharmaceutical preparations may be in solid form such as a capsule, tablet, dragee, ointment or suppository, or in liquid form such as a solution, suspension, or emulsion. If desired, there may be included in the above preparations auxiliary substances, stabilizing agents, wetting or emulsifying agents, buffers and other commonly used additives.

While the dosage of the compounds may vary from and also depend upon the age and condition of the patient, an average single dose of about 50 mg., 100 mg., 250 mg., and 500 mg. of the compounds according to the present invention has proved to be effective for treating of infectious diseases caused by a number of pathogenic bacteria. In general amounts, daily dose between 1 mg/body and about 1000 mg/body or even more may be administered.

Now in order to show the utility of the object compounds (I), test data on anti-microbial activity of representative compounds of the present invention are shown below.

Test method

One loopful of an overnight culture of each test strain in Trypticase-soy broth ($10^8$ viable cells per ml.) was streaked on heart infusion agar (HI-agar) containing graded concentrations of antibiotics, and the minimal inhibitory concentration (MIC) was expressed in terms of µg/ml after incubation at 37°C for 20 hours.

Test compound

(1) 7-[2-Ethoxyimino-2-(5-amino-1,2,4-thiadiazol-3-yl)-acetamido]-3-(3-hydroxymethyl-1-pyridiniomethyl)-3-cephem-4-carboxylate (syn isomer)

(2) 7-[2-Ethoxyimino-2-(5-amino-1,2,4-thiadiazol-3-yl)-acetamido]-3-(-4-hydroxymethyl-1-pyridiniomethyl)-3-cephem-4-carboxylate (syn isomer)

(3) 7-[2-Ethoxyimino-2-(5-amino-1,2,4-thiadiazol-3-yl)-acetamido]-3-(3-chloro-1-pyridiniomethyl)-3-cephem-4-carboxylate (syn isomer)

(4) 7-[2-Ethoxyimino-2-(5-amino-1,2,4-thiadiazol-3-yl)-acetamido]-3-(3,5-dimethyl-1-pyridiniomethyl)-3-cephem-4-carboxylate (syn isomer)

(5) 7-[2-Ethoxyimino-2-(5-amino-1,2,4-thiadiazol-3-yl)-acetamido]-3-[4-(3-hydroxypropyl)-1-pyridiniomethyl)-3-cephem-4-carboxylate (syn isomer)

Test Results

| Test Bacteria | Test Compound (1) MIC (µg/ml) | (2) | (3) | (4) | (5) |
|---|---|---|---|---|---|
| B. subtilis ATCC 6633 | 0.78 | 0.78 | 0.78 | 1.56 | 0.78 |
| Ps. aeruginosa 2 | 6.25 | 12.50 | 6.25 | 6.25 | 12.50 |
| S. marcescens 35 | 3.13 | 1.56 | 3.13 | 3.13 | 1.56 |

The following Preparations and Examples are given for the purpose of illustrating the present invention. The expressions "Nujol" and "Diaion" used in the Preparations and Examples are Trade Marks.

Preparation 1

To a suspension of isonicotinoyl chloride hydrochloride (8.94 g) in methylene chloride (50 ml) was dropped a solution of t-butylamine (11.0 g) in methylene chloride (30 ml) over 70 minutes below 40°C under stirring. The mixture was stirred for 4 hours at ambient temperature and poured into water. After the mixture was adjusted to pH 7.5 with aqueous sodium bicarbonate, the organic layer was separated out, washed with water, dried over magnesium sulfate and evaporated to dryness to give N-t-butylisonicotinamide (6.8 g), mp. 115°C to 117°C.

IR (Nujol): 3270, 1638, 1595, 1544, 1317, 1210 $cm^{-1}$.

NMR (DMSO-$d_6$, δ): 1.40 (9H, s), 7.7 (2H, m), 8.05 (1H, bs), 8.7 (2H, m).

Preparation 2

The following compounds wre obtained according to a similar manner to that of Preparation 1.

(1) N-Octylisonicotinamide, mp 61 to 63°C.

IR (Nujol): 3290, 1630, 1595, 1538, 1310, 1290 $cm^{-1}$.

(2) N-Dodecylisonicotinamide, mp 71 to 74°C.

IR (Nujol): 3400, 3320, 1630, 1598, 1535 $cm^{-1}$.

Preparation 3

A mixture of isonicotinoyl chloride hydrochloride (17.8 g), t-butylalcohol (14.8 g) and 4-(N,N-dimethylamino)pyridine (122 mg) in methylene chloride (200 ml) was refluxed for 6 hours. The mixture was washed with aqueous sodium bicarbonate and water, dried over magnesium sulfate and evaporated to give oily t-butyl isonicotinate (8.3 g).

IR (film): 2950, 2830, 1720, 1580, 1560, 1410, 1365, 1320, 1290, 1145, 1120 $cm^{-1}$.

NMR (DMSO-$d_6$, δ): 1.42 (9H, s), 7.7 (2H, m), 8.7 (2H, m).

Preparation 4

A mixture of N-hydroxyphthalimide (58.2 g), 1-chloro-2-cyclopentene (36.9 g), triethylamine (53.9 g) in acetonitrile (370 ml) was stirred to give N-(2-cyclopenten-1-yloxy)phthalimide (56.5 g)

IR (Nujol): 1780, 1730, 1610 $cm^{-1}$.

NMR (DMSO-$d_6$, δ): 7.92 (4H, s), 6.28 (1H, m), 6.00 (1H, m), 5.42 (1H, m), 2.9—1.98 (4H, m).

Preparation 5

(1) A mixture of N-(2-cyclopenten-1-yloxy)phthalimide (22.9 g) and hydrazine hydrate (4.75 g) in ethanol (115 ml) was refluxed for 5 minutes. The reaction mixture was filtered. The filtrate containing (2-cyclopenten-1-yl)oxyamine was added to a solution of sodium 2-(5-formamido-1,2,4-thiadiazol-3-yl)glyoxylate (22.4 g) in water. The mixture was adjusted to pH 2 with 10% hydrochloric acid, stirred for 2 hours and then concentrated. The concentrate was adjusted to pH 1 with 10% hydrochloric acid. The precipitates were collected by filtration and dried to give 2-(2-cyclopenten-1-yl)oxyimino-2-(5-formamido-1,2,4-thiadiazol-3-yl)acetic acid (syn isomer) (20.0 g), mp 150°C (dec.).

IR (Nujol): 3400, 3100, 1720, 1690, 1540 $cm^{-1}$.

NMR (DMSO-$d_6$, δ): 1.80—2.50 (4H, m), 5.30—5.50 (1H, m), 5.83—6.30 (2H, m), 8.90 (1H, s)

(2) To a solution of sodium hydroxide (11.2 g) in water (140 ml) was added S-methyl 2-(5-formamido-1,2,4-thiadiazol-3-yl)thioglyoxylate (27 g) at 10°C and the mixture was stirred for 30 minutes at 20°C. The reaction mixture containing sodium 2-(5-formamido-1,2,4-thiadiazol-3-yl)glyoxylate was cooled, adjusted to pH 7 with 10% hydrochloric acid and thereto was added a solution of cyclopentyloxyamine (15.3 g) in ethanol (150 ml). The mixture was adjusted to pH 3 with 10% hydrochoric acid, and stirred for 1.5 hours. The reaction mixture was adjusted to pH 7 with an aqueous solution of sodium bicarbonate and then evaporated to remove ethanol. The residue was washed with ethyl acetate. To the aqueous layer was

added ethyl acetate and the mixture was adjusted to pH 1 with 10% hydrochoric acid. The precipitates were collected by filtration to give 2-cyclopentyloxyimino-2-(5-formamido-1,2,4-thiadiazol-3-yl)acetic acid (syn isomer) (3.99 g). The filtrate was extracted with ethyl acetate and the extract was dried over magnesium sulfate and then concentrated. The precipitates were collected by filtration and washed with diethyl ether to give the same object compound (8.1 g). Total yield: 12.09 g, mp 180 to 185°C (dec.).

IR (Nujol): 3130, 3040, 2680, 2610, 2520, 1720, 1690, 1660, 1600, 1550 $cm^{-1}$.

NMR ($DMSO-d_6$, δ): 1.33—2.10 (8H, m), 4.67—5.0 (1H, m), 8.88 (1H, s), 13.50 (1H, s).

Preparation 6

A mixture of 2-(2-cyclopenten-1-yl)oxyimino-2-(5-formamido-1,2,4-thiadiazol-3-yl)acetic acid (syn isomer) (20.0 g) and 1N aqueous solution of sodium hydroxide (200 ml) was stirred for an hour at 50 to 55°C. The reaction mixture was cooled, adjusted to pH 7 with 10% hydrochloric acid and thereto was added ethyl acetate. The mixture was adjusted to pH 1 with 10% hydrochloric acid and extracted with ethyl acetate. The extract was washed with a saturated aqueous solution of sodium chloride, dried over magnesium sulfate and evaporated. The residue was pulverized with di-isopropyl ether to give 2-(2-cyclopenten-1-yl)oxyimino-2-(5-amino-1,2,4-thiadiazol-3-yl)acetic acid (syn isomer), mp 150°C (dec.).

IR (Nujol): 3300, 3150, 1710, 1620, 1520 $cm^{-1}$.

NMR ($DMSO-d_6$, δ): 1.80—2.50 (4H, m), 5.30—5.50 (1H, m), 5.83—6.30 (2H, m), 8.20 (2H, s)

Preparation 7

The following compound was obtained according to a similar manner to that of Preparation 6.

2-Cyclopentyloxyimino-2-(5-amino-1,2,4-thiadiazol-3-yl)acetic acid (syn isomer), mp 160 to 165°C (dec.).

IR (Nujol): 3470, 3290, 3200, 2400, 1715, 1615, 1600, 1520 $cm^{-1}$.

NMR ($DMSO-d_6$, δ): 1.17—2.10 (8H, m), 4.60—4.97 (1H, m), 8.22 (2H, s)

Preparation 8

A mixture of S-methyl(5-formamido-1,2,4-thiadiazol-3-yl)thioglyoxylate (6 g) and an aqueous solution (50 ml) of sodium hydroxide (4.2 g) was stirred for an hour at 50 to 55°C. The mixture was cooled to ambient temperature and adjusted to pH 7 with 10% hydrochloric acid. On the other hand, a mixture of N-(ethoxycarbonylmethoxy)phthalimide (12.9 g) and hydrazine hydrate (2.08 g) in ethanol (60 ml) was refluxed for 5 minutes and cooled in an ice bath. A resulting precipitate was filtered off and washed with ethanol. The filtrate and the washings were combined and the combined solution containing O-(ethoxycarbonylmethyl)hydroxylamine was added to the above aqueous solution. The mixture was adjusted to pH 3 to 4 with 10% hydrochloric acid and stirred for 1.5 hours at ambient temperature. The solution was neutralized with an aqueous solution of sodium bicarbonate, concentrated to half volume in vacuo and washed with ethyl acetate. The aqueous solution was acidified with 10% hydrochloric acid and extracted with ethyl acetate. The extract was dried over magnesium sulfate, evaporated to dryness and the residue was triturated with diisopropyl ether to give 2-ethoxycarbonyl-methoxyimino-2-(5-amino-1,2,4-thiadiazol-3-yl)acetic acid (syn isomer) (1.8 g), mp 135 to 140°C (dec.).

IR (Nujol): 3500, 3330, 3210, 2670, 2550, 1740, 1610, 1540 $cm^{-1}$.

NMR ($DMSO-d_6$, δ): 1.24 (3H, t, J=7Hz), 4.14 (2H, q, J=7Hz), 4.80 (2H, s), 8.15 (2H, broad s)

Preparation 9

The following compounds were obtained according to a similar manner to that of Preparation 8.

(1) 2-(t-Butoxycarbonylmethoxyimino)-2-(5-amino-1,2,4-thiadiazol-3-yl)acetic acid (syn isomer), mp 150 to 155°C (dec.).

IR (Nujol): 3420, 3230, 3100, 1725, 1610, 1530 $cm^{-1}$.

NMR ($DMSO-d_6$, δ): 1.45 (9H, s), 4.70 (2H, s), 8.12 (2H, broad s)

(2) 2-(1-Ethoxycarbonyl-1-methylethoxyimino)-2-(5-amino-1,2,4-thiadiazol-3-yl)acetic acid (syn isomer), mp 165 to 168°C (dec.).

IR (Nujol): 3450, 3350, 3240, 1750, 1730, 1630, 1530 $cm^{-1}$.

NMR ($DMSO-d_6$, δ): 1.18 (3H, t, J=7Hz), 1.50 (6H, s), 4.15 (2H, q, J=7Hz), 8.23 (2H, broad s)

Preparation 10

To a cold solution of phosphorus pentachloride (16.6 g) in methylene chloride (150 ml) was added 2-ethoxyimino-2-(5-amino-1,2,4-thiadiazol-3-yl)acetic acid (syn isomer)(17.3 g) at −18°C and the mixture was stirred for 15 minutes at −13 to −10°C. On the other hand, a mixture of 7-amino-3-acetoacetoxy-methyl-3-cephem-4-carboxylic acid (25.1 g) and trimethylsilylacetamide (80 g) in methylene chloride (400 ml) was warmed to make a clear solution and cooled to −10°C. The cold solution was added to the above activated mixture and the mixture was stirred for 1 hour at −10°C. The reaction mixture was poured into an aqueous solution (450 ml) of sodium bicarbonate (42 g) and stirred for 1 hour at room temperature. The aqueous layer was separated out, adjusted to pH 2 with 6N hydrochloric acid and extracted with ethyl acetate. The extract was washed with water, dried over anhydrous magnesium sulfate and evaporated to dryness. The residue was triturated with diethyl ether to give a powder of 7-[2-ethoxyimino-2-(5-amino-1,2,4-thiadiazol-

3-yl)acetamido]-3-acetoacetoxymethyl-3-cephem-4-carboxylic acid (syn isomer)(24.5 g). This compound was dissolved in 1N methanol solution of sodium acetate (48 ml) and stood on to give precipitates. Thereto was added acetone (100 ml). The precipitates were collected by filtration, washed with acetone to give sodium 7-[2-ethoxyimino-2-(5-amino-1,2,4-thiadiazol-3-yl)acetamido]-3-acetoacetoxymethyl-3-cephem-4-carboxylate (syn isomer) (19 g).

Physical constants of 7-[2-ethoxyimino-2-(5-amino-1,2,4-thiadiazol-3-yl)acetamido]-3-acetoacetoxymethyl-3-cephem-4-carboxylic acid (syn isomer): mp 101 to 106°C (dec.).

IR (Nujol): 3400, 3300, 3170, 1770, 1710, 1660, 1620, 1525, 1145, 1035 $cm^{-1}$.

Physical constants of sodium 7-[2-ethoxyimino-2-(5-amino-1,2,4-thiadiazol-3-yl)acetamido]-3-acetoacetoxymethyl-3-cephem-4-carboxylate (syn isomer): mp 175 to 180°C (dec.).

IR (Nujol): 3450, 3300, 3100, 1790, 1720, 1670, 1640, 1610, 1550 $cm^{-1}$.

NMR ($D_2O$, δ): 1.38 (3H, t, J=6Hz), 2.34 (3H, s), 3.44, 3.66 (2H, ABq, J=18Hz), 4.40 (2H, q, J=6Hz), 5.05, 5.86 (2H, ABq, J=12Hz), 5.26 (1H, d, J=4Hz), 5.90 (1H, d, J=4Hz).

Preparation 11

The following compounds were obtained according to a similar manner to that of Preparation 10.

(1) 7-[2-Cyclopentyloxyimino-2-(5-amino-1,2,4-thiadiazol-3-yl)acetamido]-3-acetoacetoxymethyl-3-cephem-4-carboxylic acid (syn isomer), powder, mp. 130 to 135°C (dec.).

IR (Nujol): 3300, 1780, 1720, 1680, 1620, 1525 $cm^{-1}$.

NMR (DMSO-$d_6$, δ): 1.3—2.0 (8H, m), 2.15 (3H, s), 3.52 (2H, bs), 3.60 (2H, s), 4.5—4.7 (1H, m), 4.77, 5.00 (2H, ABq, J=14Hz), 5.13 (1H, d, J=4Hz), 5.80 (1H, 2d, J=4 and 8Hz), 8.10 (2H, s), 9.50 (1H, d, J=8Hz)

(2) 7-[2-(2-Cyclopenten-1-yloxyimino)-2-(5-amino-1,2,4-thiadiazol-3-yl)acetamido]-3-acetoacetoxymethyl-3-cephem-4-carboxylic acid (syn isomer), mp. 135 to 140°C (dec.).

IR (Nujol): 3400, 3300, 3200, 1775, 1735, 1710, 1675, 1620, 1525 $cm^{-1}$.

NMR (DMSO-$d_6$, δ): 1.93—2.47 (4H, m), 2.18 (3H, s), 3.55 (3H, bs), 3.65 (2H, s), 4.80, 5.07 (2H, ABq, J=13Hz), 5.13 (1H, d, J=5Hz), 5.23—5.53 (1H, m), 5.70—6.23 (3H, m), 8.12 (2H, bs), 9.50 (1H, d, J=8Hz)

(3) 7-[2-Carboxymethoxyimino-2-(5-amino-1,2,4-thiadiazol-3-yl)acetamido]-3-acetoacetoxymethyl-3-cephem-4-carboxylic acid (syn isomer), mp. 95 to 100°C (dec.).

IR (Nujol): 3400, 3290, 3190, 1770, 1720, 1615, 1520 $cm^{-1}$.

NMR (DMSO-$d_6$, δ): 2.17 (3H, s), 3.53 (2H, bs), 3.63 (2H, s), 4.67 (2H, s), 4.80, 5.07 (2H, ABq, J=13Hz), 5.15 (1H, d, J=5Hz), 5.87 (1H, 2d, J=5 and 8Hz) 8.15 (2H, bs), 9.53 (1H, d, J=8Hz).

(4) 7-[2-t-Butoxycarbonylmethoxyimino-2-(5-amino-1,2,4-thiadiazol-3-yl)acetamido]-3-acetoacetoxymethyl-3-cephem-4-carboxylic acid (syn isomer), mp. 105 to 110°C (dec.).

IR (Nujol): 3350, 3250, 1780, 1720, 1620, 1525 $cm^{-1}$.

NMR (DMSO-$d_6$, δ): 1.43 (9H, s), 2.17 (3H, s), 3.53 (2H, bs), 3.63 (2H, s), 4.63 (2H, s), 4.82, 5.05 (2H, ABq, J=13Hz), 5.15 (1H, d, J=5Hz), 5.85 (1H, 2d, J=5 and 8Hz), 8.15 (2H, bs), 9.53 (1H, d, J=8Hz)

(5) 7-[2-Cyclopentyloxyimino-2-(5-amino-1,2,4-thiadiazol-3-yl)acetamido]cephalosporanic acid (syn isomer). mp 140 to 145°C (dec.).

IR (Nujol): 3480, 3370, 3250, 1785, 1730, 1680, 1630, 1530 $cm^{-1}$.

NMR (DMSO-$d_6$, δ): 1.33—2.17 (8H, m), 2.03 (3H, s), 3.57 (2H, broad s), 4.60—4.90 (1H, m), 4.73 and 4.97 (2H, ABq, J=13Hz), 5.15 (1H, d, J=5Hz), 5.80 (1H, dd, J=5 and 8Hz), 8.10 (2H, broad s), 9.47 (1H, d, J=8Hz).

(6) 7-[2-(2-Cyclopenten-1-yl)oxyimino-2-(5-amino-1,2,4-thiadiazol-3-yl)acetamido]cephalosporanic acid (syn isomer). mp 154 to 159°C (dec.).

IR (Nujol): 3300, 1770, 1720, 1670, 1620, 1520 $cm^{-1}$.

NMR (DMSO-$d_6$, δ): 2.0 (3H, s), 2.0—2.40 (4H, m), 3.52 (2H, broad s), 4.70 and 4.97 (2H, ABq, J=14Hz), 5.12 (1H, d, J=4Hz), 5.27—5.40 (1H, m), 5.82 (1H, dd, J=4 and 8Hz), 5.83—6.17 (2H, m), 8.13 (2H, s), 9.50 (1H, d, J=8Hz)

(7) 7-[2-(t-Butoxycarbonylmethoxyimino)-2-(5-amino-1,2,4-thiadiazol-3-yl)acetamido]cephalosporanic acid (syn isomer), mp 125 to 130°C (dec.).

IR (Nujol): 3300, 3200, 1770, 1720, 1680, 1620, 1520 $cm^{-1}$.

NMR (DMSO-$d_6$, δ): 1.50 (9H, s), 2.10 (3H, s), 3.62 (2H, broad s), 468 (2H, s), 4.77 and 5.03 (2H, ABq, J=13Hz), 5.20 (1H, d, J=4Hz), 5.88 (1H, dd, J=4 and 8Hz), 8.18 (2H, s), 9.55 (1H, d, J=8Hz)

(8) 7-[2-Carboxymethoxyimino-2-(5-amino-1,2,4-thiadiazol-3-yl)acetamido]cephalosporanic acid (syn isomer), mp 160 to 165°C (dec.).

IR (Nujol): 3300, 3200, 1770, 1720, 1680, 1520 $cm^{-1}$.

NMR (DMSO-$d_6$, δ): 2.10 (3H, s), 3.60 (2H, broad s), 4.70 (2H, s), 4.77 and 5.03 (2H, ABq, J=14Hz), 5.20 (1H, d, J=4Hz), 5.88 (1H, dd, J=4 and 8Hz), 8.18 (2H, s), 9.57 (1H, d, J=8Hz)

Preparation 12

To a suspension of 7-(2-thienyl-acetamido-3-(3-hydroxymethyl-1-pyridiniomethyl)-3-cephem-4-carboxylate (27.0 g) and N, N-dimethylaniline (50.8 g) in methylene chloride (240 ml) was dropped trimethylsilyl chloride (26.0 g) under stirring and the mixture was stirred for 1.5 hours at room temperature. To the resulting solution was added phosphorous pentachloride (31.2 g) at −30°C and the mixture was stirred for one hour at −30°C to −25°C. The mixture was added to a solution of n-butanol (120 g) in methylene chloride (240 ml) at −25°C under stirring, and the stirring was continued for 1.5 hours at room

temperature. A resulting precipitate was collected by filtration, washed with methylene chloride and dried on phosphorous pentoxide to give 7-amino-3-(3-hydroxymethyl-1-pyridiniomethyl)-3-cephem-4-carboxylate dihydrochloride (25.5 g), mp 120 to 125°C (dec.).

IR (Nujol): 1780, 1700, 1630 $cm^{-1}$.

NMR ($DCl+D_2O$, δ): 3.60, 3.83 (2H, ABq, J=18Hz), 4.97 (2H, s), 5.33 (1H, d, J=5Hz), 5.47 (1H, d, J=5Hz), 5.60, 5.93 (2H, ABq, J=14Hz), 8.0—8.33 (1H, m), 8.50—8.76 (1H, m), 8.83—9.16 (2H, m).

Example 1

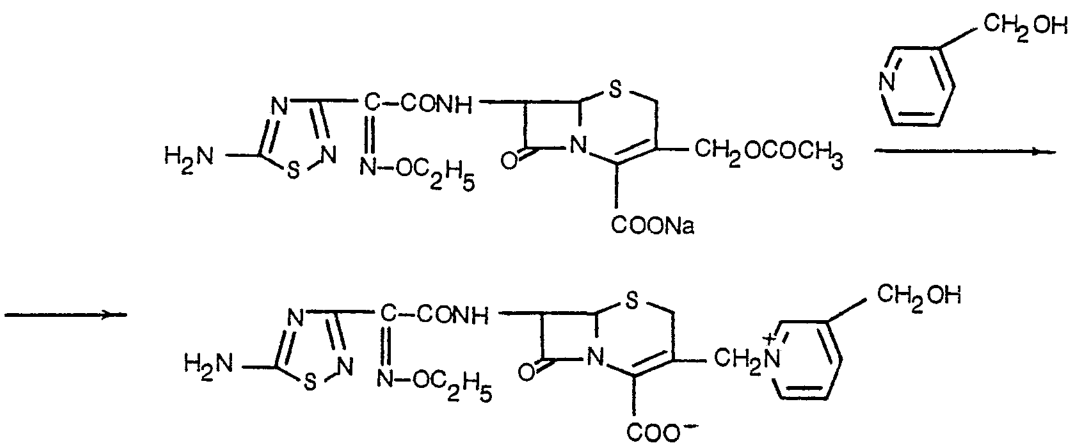

To a mixture of 3-hydroxymethylpyridine (26.6 g) and sodium iodide (150 g) in formamide (240 ml) was added sodium 7-(2-ethoxyimino-2-(5-amino-1,2,4-thiadiazol-3-yl)acetamido]-cephalosporanate (syn isomer) (60 g) by portions at 75°C under stirring, which was continued for 3 hrs. at 80 to 85°C. The mixture was cooled to 20°C and diluted with isopropyl alcohol (3 l) to give an oily substance, which was separated by decantation. The separated oily substance was triturated in isopropyl alcohol (1 l) to give a crude object compound as a powder. The crude product was dissolved in water (500 ml) and subjected to column chromatography on a non ionic adsorption resin "Diaion HP—20" (Trademark, prepared by Mitsubishi Chemical Industries) (1.8 l). After the Column was washed with water (5.5 l), the elution was carried out with 30% aqueous methanol. The eluates (2.5 l) containing an object compound were collected, concentrated to 550 ml under reduced pressure and passed through a column packed with acidic alumina (150 g). The eluate (700 ml) was lyophilized to give white powder of 7 - [2 - ethoxyimino - 2 - (5 - amino - 1,2,4 - thiadiazol - 3 - yl) - acetamido] - (3 - hydroxymethyl - 1 - pyridiniomethyl) - 3 - cephem - 4 - carboxylate (syn isomer) (12.5 g), mp 160 to 165°C (dec.).

IR (Nujol): 3300, 1770, 1660, 1610, 1520 $cm^{-1}$.

NMR ($D_2O$, δ): 1.23 (3H, t, J=7Hz), 3.23 and 3.57 (2H, ABq, J=17Hz), 4.30 (2H, q, J=7Hz), 4.83 (2H, s), 5.23 (1H, d, J=4Hz), 5.33 and 5.53 (2H, ABq, J=14Hz), 5.85 (1H, d, J=4Hz), 8.03 (1H, dd, J=6 and 8Hz), 8.50 (1H, d, J=8Hz), 8.85 (1H, d, J=6Hz), 8.90 (1H, s).

Example 2

To a mixture of 3-hydroxypyridine (2.32 g) and sodium iodide (15 g) in formamide (15 ml) was added sodium 7-[2-ethoxyimino-2-(5-amino-1,2,4-thiadiazol-3-yl)acetamido]cephalosporanate (syn isomer) (6.0 g) at 75°C under stirring, which was continued for 2.5 hours at 80 to 82°C. The mixture was cooled to room temperature and poured into isopropyl alcohol (150 ml). The resulting precipitates were collected by filtration, washed with isopropyl alcohol and diisopropyl ether and then dissolved in water (80 ml). The aqueous solution was adjusted to pH 3 with 6N-hydrochloric acid, washed with ethyl acetate, evaporated to remove ethyl acetate and subjected to column chromatography on a non ionic adsorption resin "Diaion HP—20" (160 ml). After the column was washed with water, the elution was carried out with 30% aqueous methanol. The eluates containing an object compound were collected, evaporated to remove methanol under reduced pressure and lyophilized to give 7 - [2 - ethoxyimino - 2 - (5 - amino - 1,2,4 - thiadiazol - 3 - yl)acetamido] - 3 - (3 - hydroxy - 1 - pyridiniomethyl) - 3 - cephem - 4 - carboxylate (syn isomer) (0.93 g), mp 175 to 180°C (dec.).

IR (Nujol): 3390, 3290, 3180, 3050, 1770, 1660, 1625, 1610, 1525 $cm^{-1}$.

NMR ($DMSO-d_6+D_2O$, δ): 1.20 (3H, t, J=7Hz), 3.17 and 3.47 (2H, ABq, J=18Hz), 4.13 (2H, q, J=7Hz), 5.07 (1H, d, J=5Hz), 5.22 and 5.52 (2H, ABq, J=14Hz), 5.70 (1H, d, J=5Hz), 7.67—8.17 (2H, m), 8.40—8.90 (2H, m).

Example 3

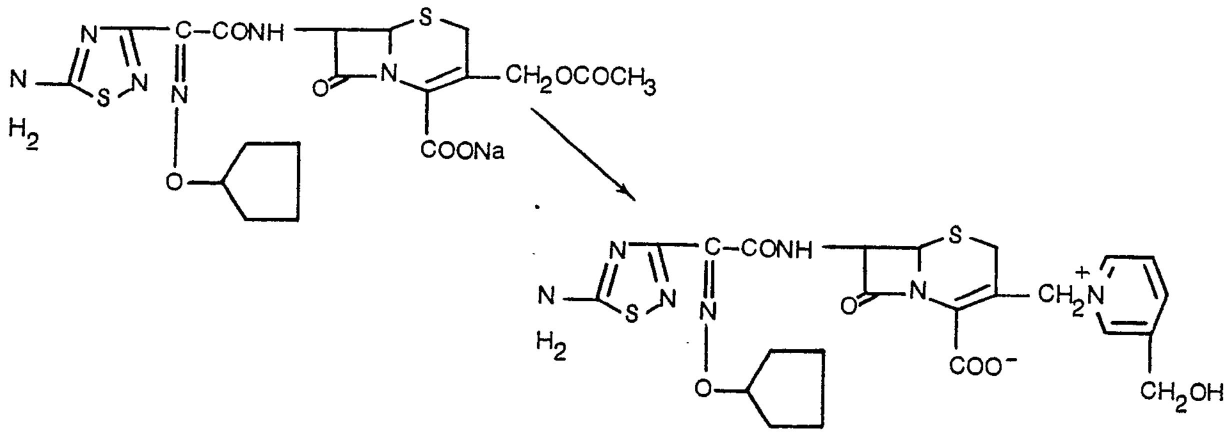

A mixture of 3-hydroxymethylpyridine (1.3 g) and potassium iodide (7.95 g) in water (5.3 ml) was warmed to 60°C under stirring and sodium 7-[2-cyclopentyloxyimino-2-(5-amino-1,2,4-thiadiazol-3-yl)acetamido]cephalosporanate (syn isomer) (5.32 g) was added thereto. The mixture was stirred for 5 hours at 60°C and diluted with a mixture of water (100 ml), ethyl acetate (50 ml) and acetone (10 ml). The mixture was adjusted to pH 1 with 6N hydrochloric acid and the aqueous layer was separated out. The aqueous solution was evaporated to remove acetone and ethyl acetate and subjected to column chromatography on a non ionic adsorption resin "Diaion HP—20" (160 ml). After the column was washed with water (500 ml), the elution was carried out with 40% aqueous methanol. The eluates containing an object compound were collected, evaporated to remove methanol under reduced pressure and lyophilized to give 7 - [2 - cyclopentyloxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer) (1.2 g), white powder, mp 160 to 165°C (dec.).

IR (Nujol): 3260, 3170, 1770, 1655, 1610, 1520 $cm^{-1}$.

NMR (DMSO-$d_6$, δ): 1.33—2.00 (8H, m), 3.17, 3.50 (2H, ABq, J=18Hz), 4.57—4.90 (3H, m), 5.08 (1H, d, J=5Hz), 5.30, 5.65 ( 2H, ABq, J=14Hz), 5.70 (1H, 2d, J=5 and 8Hz), 8.00—8.33 (3H, m), 8.33—8.67 (1H, m), 9.17—9.43 (2H, m), 9.42 (1H, d, J=8Hz).

Example 4

To a mixture of thiazole (2.5 ml), sodium iodide (18.0 g) and water 3 ml was added sodium 7-[2-ethoxyimino-2-(5-amino-1,2,4-thiadiazol-3-yl)acetamido]cephalosporanate (syn isomer) (5.41 g) at 60°C under stirring, which was continued for one hour at 75°C. The reaction mixture was diluted with water (50 ml), adjusted to pH 3.6 with 1N hydrochloric acid and filtered. The filtrate was subjected to column chromatography on a non ionic adsorption resin "Diaion HP—20" (150 ml). After the column was washed with water, the elution was carried out with 20% aqueous methanol. The eluants containing an object compound were collected, evaporated to remove methanol under reduced pressure and lyophilized to give 7 - [2 - ethoxyimino - 2 - (5 - amino - 1,2,4 - thiadiazol - 3 - yl)acetamido] - 3 - (3 - thiazoliomethyl) - 3 - cephem - 4 - carboxylate (syn isomer) (2.3 g), mp 155 to 160°C (dec.).

IR (Nujol): 3400—3200, 1770, 1660, 1600, 1520 $cm^{-1}$.

NMR ($D_2O$, δ): 1.30 (3H, t, J=7Hz), 3.20 and 3.72 (2H, ABq, J=18Hz), 4.32 (2H, q, J=7Hz), 5.25 (1H, d, J=5Hz), 5.29 and 5.50 (2H, ABq, J=13Hz), 5.83 (1H, d, J=5Hz), 8.19 (1H, d, J=4Hz), 8.42 (1H, d, J=4Hz).

Example 5

A mixture of sodium iodide (13 g), dodecyl isonicotinate (5.8 g), 7-[2-ethoxyimino-2-(5-amino-1,2,4-thiadiazol-3-yl)acetamido]-3-acetoacetoxymethyl-3-cephem-4-carboxylic acid (syn isomer) (5.12 g), water (2.6 ml) and acetonitrile (7.8 ml) was stirred at 50° to 60°C for 2 hours. To the reaction mixture was added a mixture of ethyl acetate (60 ml) and water (60 ml). A resulting precipitate was filtered and dissolved in a mixed solvent (1:1) of chloroform and ethanol. The solution was washed with water, dried over magnesium sulfate and concentrated to 30 ml. The resulting precipitates were filtered, washed with ethyl acetate and dried to give 7 - [2 - ethoxyimino - 2 - (5 - amino - 1,2,4 - thiadiazol - 3 - yl)acetamido] - 3 - (4 - dodecyloxycarbonyl - 1 - pyridiniomethyl) - 3 - cephem - 4 - carboxylate (syn isomer) (2.2 g), mp 152 to 157°C (dec.).

IR (Nujol): 3280, 3160, 1775, 1730, 1675, 1610, 1520 $cm^{-1}$.

NMR (DMSO-$d_6$+$D_2O$, δ): 0.86 (3H, t, J=6Hz), 1.0—1.9 (23H, m), 3.22 and 3.58 (2H, ABq, J=18Hz), 3.9—4.5 (4H, m), 5.12 (1H, d, J=5Hz), 5.10 and 5.76 (2H, ABq, J=14Hz), 5.72 (1H, d, J=5Hz), 8.52 (2H, d, J=6Hz), 9.56 (2H, d, J=6Hz).

Example 6

The following compound was obtained by reacting sodium 7-[2-ethoxyimino-2-(5-amino-1,2,4-thiadiazol-3-yl)acetamido]-3-acetoacetoxymethyl-3-cephem-4-carboxylate (syn isomer) with 4-cyanopyridine according to similar manners to those of Examples 1 to 5.

7 - [2 - Ethoxyimino - 2 - (5 - amino - 1,2,4 - thiadiazol - 3 - yl)acetamido] - 3 - (4 - cyano - 1 - pyridiniomethyl) - 3 - cephem - 4 - carboxylate (syn isomer), mp 147 to 151°C (dec.).

IR (Nujol): 3400—3100, 1780, 1670, 1610, 1530, 1040 $cm^{-1}$.

NMR ($D_2O$, δ): 1.31 (3H, t, J=7Hz), 3.20 and 3.70 (2H, ABq, J=18Hz), 4.31 (2H, q, J=7Hz), 5.25 (1H, d, J=5Hz), 5.40 and 5.71 (2H, ABq, J=14Hz), 5.82 (1H, d, J=5Hz), 8.45 (2H, d, J=6Hz), 9.26 (2H, d, J=6Hz).

Example 7

The following compounds were obtained according to similar manners to those of Examples 1 to 6.

(1) 7 - [2 - Ethoxyimino - 2 - (5 - amino - 1,2,4 - thiadiazol - 3 - yl)acetamido] - 3 - (4 - hydroxymethyl - 1 - pyridiniomethyl) - 3 - cephem - 4 - carboxylate (syn isomer), mp 150 to 155°C (dec.).

IR (Nujol): 3250, 1770, 1660, 1630, 1605, 1570, 1520 $cm^{-1}$.

NMR (DMSO-$d_6$+$D_2O$, δ): 1.23 (3H, t, J=7Hz), 3.22 and 3.58 (2H, ABq, J=18Hz), 4.25 (2H, q, J=7Hz), 4.90 (2H, s), 5.17 (1H, d, J=5Hz), 5.28 and 5.65 (2H, ABq, J=14Hz), 5.80 (1H, d, J=5Hz), 8.12 (2H, d, J=6Hz), 9.28 (2H, d, J=6Hz).

(2) 7 - [2 - Ethoxyimino - 2 - (5 - amino - 1,2,4 - thiadiazol - 3 - yl)acetamido] - 3 - [4 - (3 - hydroxypropyl) - 1 - pyridiniomethyl] - 3 - cephem - 4 - carboxylate (syn isomer), mp 159 to 167°C (dec.).

IR (Nujol): 3400—3100, 1770, 1670—1610, 1540 $cm^{-1}$.

NMR (DMSO-$d_6$+$D_2O$, δ): 1.23 (3H, t, J=7Hz), 1.7—2.1 (2H, m), 2.8—3.3 (2H, m), 3.2—4.0 (4H, m), 4.20 (2H, q, J=7Hz), 5.05 (1H, d, J=5Hz), 5.13 and 5.63 (2H, ABq, J=14Hz), 5.75 (1H, d, J=5Hz), 8.02 (2H, d, J=7Hz), 9.17 (2H, d, J=7Hz).

(3) 7 - [2 - Ethoxyimino - 2 - (5 - amino - 1,2,4 - thiadiazol - 3 - yl)acetamido] - 3 - (3 - chloro - 1 - pyridiniomethyl) - 3 - cephem - 4 - carboxylate (syn isomer), mp 210 to 220°C (dec.).

IR (Nujol): 3400—3100, 1770, 1660, 1610, 1520 $cm^{-1}$.

NMR (DMSO-$d_6$+$D_2O$, δ): 1.23 (3H, t, J=7Hz), 3.17 and 3.50 (2H, ABq, J=18Hz), 4.15 (2H, q, J=7Hz), 5.08 (1H, d, J=5Hz), 5.0—5.8 (2H, m), 5.73 (1H, d, J=5Hz), 7.8—8.8 (2H, m), 9.4 (1H, m), 9.7 (1H, m).

(4) 7 - [2 - Ethoxyimino - 2 - (5 - amino - 1,2,4 - thiadiazol - 3 - yl)acetamido] - 3 - (3 - methyl - 1 - pyridiniomethyl) - 3 - cephem - 4 - carboxylate (syn isomer), mp 175 to 180°C (dec.).

IR (Nujol): 3280, 1770, 1660, 1610, 1530, 1035 $cm^{-1}$.

NMR ($D_2O$, δ): 1.26 (3H, t, J=7Hz), 2.50 (3H, s), 3.12 and 3.75 (2H, ABq, J=18Hz), 4.29 (2H, q, J=7Hz), 5.22 (1H, d, J=5Hz), 5.20 and 5.53 (2H, ABq, J=13Hz), 5.82 (1H, d, J=5Hz), 7.88 (1H, m), 8.35 (1H, m), 8.73 (2H, m).

(5) 7 - [2 - Ethoxyimino - 2 - (5 - amino - 1,2,4 - thiadiazol - 3 - yl)acetamido] - 3 - (3,5 - dimethyl - 1 - pyridiniomethyl) - 3 - cephem - 4 - carboxylate (syn isomer), mp 165 to 175°C (dec.).

IR (Nujol): 3400—3150, 1770, 1660, 1610, 1530 $cm^{-1}$.

NMR ($D_2O$, δ): 1.32 (3H, t, J=7Hz), 2.50 (6H, s), 3.21 and 3.62 (2H, ABq, J=18Hz), 4.32 (2H, q, J=7Hz), 4.87 (1H, d, J=5Hz), 5.0—5.6 (3H, m), 8.16 (1H, bs), 8.58 (2H, s).

(6) 7 - [2 - Ethoxyimino - 2 - (5 - amino - 1,2,4 - thiadiazol - 3 - yl)acetamido] - 3 - (4 - t - butoxycarbonylaminomethyl - 1 - pyridiniomethyl) - 3 - cephem - 4 - carboxylate (syn isomer), mp 154 to 158°C (dec.).

IR (Nujol): 3600, 1775, 1670, 1640, 1610, 1520, 1280, 1035 $cm^{-1}$.

NMR ($D_2O$, δ): 1.21 (3H, t, J=7Hz), 1.40 (9H, s), 3.0—3.7 (2H, m); 4.13 (2H, q, J=7Hz), 4.40 (2H, broad s), 5.03 (1H, d, J=5Hz), 4.9—5.8 (2H, m), 5.67 (1H, d, J=5Hz), 7.90 (2H, d, J=6Hz), 9.25 (2H, d, J=6Hz).

(7) 7 - [2 - Ethoxyimino - 2 - (5 - amino - 1,2,4 - thiadiazol - 3 - yl)acetamido] - 3 - (4 - aminomethyl - 1 - pyridiniomethyl) - 3 - cephem - 4 - carboxylate diformate (syn isomer), mp 149 to 159°C (dec.).

IR (Nujol): 3250, 3150, 1770, 1660, 1640, 1580, 1520, 1040 $cm^{-1}$.

(8) 7 - [2 - Ethoxyimino - 2 - (5 - amino - 1,2,4 - thiadiazol - 3 - yl)acetamido] - 3 - (4 - t - butyl - 1 - pyridiniomethyl) - 3 - cephem - 4 - carboxylate (syn isomer), mp 169.5 to 175.0°C (dec.).

IR (Nujol): 3280, 1770, 1670, 1630, 1610, 1530 $cm^{-1}$.

NMR ($D_2O$, δ): 1.40 (9H, s), 1.88 (3H, t, J=7Hz), 3.13 and 3.70 (2H, ABq, J=19Hz), 4.21 (2H, q, J=7Hz), 5.25 and 5.56 (2H, ABq, J=14Hz), 5.28 (1H, d, J=5Hz), 5.87 (1H, d, J=5Hz), 8.05 (2H, d, J=7Hz), 8.82 (2H, d, J=7Hz).

(9) 7 - [2 - Cyclopentyloxyimino - 2 - (5 - amino - 1,2,4 - thiadiazol - 3 - yl)acetamido] - 3 - (4 - hydroxymethyl - 1 - pyridiniomethyl) - 3 - cephem - 4 - carboxylate (syn isomer), mp 155 to 160°C (dec.).

IR (Nujol): 3350, 3200, 1775, 1670, 1615, 1525 $cm^{-1}$.

NMR (DMSO-$d_6$, δ): 1.20—2.00 (8H, m), 3.13, 3.50 (2H, ABq, J=18Hz), 4.57—4.98 (3H, m), 5.08 (1H, d, J=5Hz), 5.25, 5.60 (2H, ABq, J=14Hz), 5.67 (1H, 2d, J=5 and 8Hz), 8.05 (2H, d, J=6Hz), 8.10 (2H, bs), 9.27 (2H, d, J=6Hz), 9.40 (1H, d, J=8Hz).

(10) 7 - [2 - Cyclopentyloxyimino - 2 - (5 - amino - 1,2,4 - thiadiazol - 3 - yl)acetamido] - 3 - (4 - acetyl - 1 - pyridiniomethyl) - 3 - cephem - 4 - carboxylate (syn isomer), mp 145 to 150°C (dec.).

IR (Nujol): 3270, 1770, 1700, 1670, 1610, 1520 $cm^{-1}$.

NMR (DMSO-$d_6$+$D_2O$, δ): 1.33—2.00 (8H, m), 2.67 (3H, s), 3.07, 3.45 (2H, ABq, J=18Hz), 4.50—4.77 (1H,

m), 5.02 (1H, d, J=5Hz), 5.25, 5.58 (2H, ABq, J=14Hz), 5.63 (1H, d, J=5Hz (1H, d, J=5Hz), 8.40 (2H, d, J=6Hz), 9.52 (2H, d, J=6Hz).

(11) 7 - [2 - Cyclopentyloxyimino - 2 - (5 - amino - 1,2,4 - thiadiazol - 3 - yl)acetamido] - 3 - (3 - carboxymethyl - 1 - pyridiniomethyl) - 3 - cephem - 4 - carboxylate (syn isomer), mp 165 to 170°C (dec.).

IR (Nujol): 3350, 3200, 1775, 1520, 1670, 1620, 1525 $cm^{-1}$.

NMR ($DMSO-d_6+D_2O$, δ): 1.30—2.00 (8H, m), 3.17, 3.52 (2H, ABq, J=18Hz), 3.93 (2H, s), 4.57—4.87 (1H, m), 5.07 (1H, d, J=5Hz), 5.27, 5.63 (2H, ABq, J=14Hz), 5.70 (1H, d, J=5Hz), 7.93—8.23 (1H, m), 8.37—8.63 (1H, m), 9.10—9.37 (2H, m).

(12) 7 - [2 - Cyclopentyloxyimino - 2 - (5 - amino - 1,2,4 - thiadiazol - 3 - yl)acetamido] - 3 - (4 - hydroxyiminomethyl - 1 - pyridiniomethyl) - 3 - cephem - 4 - carboxylate (syn isomer), mp 140 to 145°C (dec.).

IR (Nujol): 3280, 3160, 1770, 1680, 1630, 1610, 1520 $cm^{-1}$.

NMR ($DMSO-d_6$, δ): 1.33—2.00 (8H, m), 3.23, 3.57 (2H, ABq, J=18Hz), 4.57—4.90 (1H, m), 5.12 (1H, d, J=5Hz), 5.28, 5.65 (2H, ABq, J=14Hz), 5.73 (1H, 2d, J=5 and 8Hz), 8.18 (2H, bs), 8.25 (2H, d, J=6Hz), 8.42 (1H, s), 9.35 (2H, d, J=6Hz), 9.43 (1H, d, J=8Hz).

(13) 7 - [2 - Cyclopentyloxyimino - 2 - (5 - amino - 1,2,4 - thiadiazol - 3 - yl)acetamido] - 3 - [4 - methyl - 5 - (2 - hydroxyethyl) - 3 - thiazoliomethyl] - 3 - cephem - 4 - carboxylate (syn isomer), mp 150 to 155°C (dec.).

IR (Nujol): 3330, 1780, 1670, 1610, 1530 $cm^{-1}$.

NMR ($DMSO-d_6+D_2O$, δ): 1.33—2.00 (8H, m), 2.57 (3H, s), 2.90—3.17 (2H, m), 3.17—3.43 (2H, m), 3.57—3.80 (2H, m), 4.60—4.90 (1H, m), 5.07 (1H, d, J=5Hz), 5.37 (2H, bs), 5.70 (1H, 2d, J=5 and 8Hz), 8.17 (2H, bs), 9.43 (1H, d, J=8Hz), 10.23 (1H, s).

(14) 7 - [2 - (2 - Cyclopenten - 1 - yloxyimino) - 2 - (5 - amino - 1,2,4 - thiadiazol - 3 - yl)acetamido] - 3 - (4 - hydroxymethyl - 1 - pyridiniomethyl) - 3 - cephem - 4 - carboxylate (syn isomer), mp 140 to 145°C (dec.).

IR (Nujol): 3300, 3200, 1770, 1660, 1620, 1520 $cm^{-1}$.

NMR ($DMSO-d_6$, δ): 1.8—2.5 (4H, m), 3.13, 3.50 (2H, ABq, J=18Hz), 4.83 (2H, s), 5.07 (1H, d, J=4Hz), 5.20—5.40 (1H, m), 5.27, 5.67 (2H, ABq, J=14Hz), 5.80 (1H, 2d, J=4 and 8Hz), 5.87—6.20 (2H, m), 8.07 (2H, d, J=7Hz), 8.17 (2H, s), 9.33 (2H, d, J=7Hz), 9.47 (1H, d, J=8Hz).

(15) 7 - [2 - (2 - Cyclopenten - 1 - yloxyimino) - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp 143 to 148°C (dec.).

IR (Nujol): 3300, 3200, 1770, 1660, 1610, 1520 $cm^{-1}$.

NMR ($DMSO-d_6$, δ): 1.8—2.5 (4H, m), 3.13, 3.47 (2H, ABq, J=17Hz), 4.73 (2H, s), 5.35 (1H, d, J=4Hz), 5.17—5.40 (1H, m), 5.40—6.20 (5H, m), 8.13 (2H, s), 8.0—8.30 (1H, m), 8.37—8.60 (1H, m), 9.27—9.43 (2H, m), 9.47 (1H, d, J=8Hz).

(16) 7 - [2 - Carboxymethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp 170 to 175°C (dec.).

IR (Nujol): 3300, 1770, 1670, 1620, 1525 $cm^{-1}$.

NMR ($DMSO-d_6+D_2O$, δ): 3.20, 3.50 (2H, ABq, J=18Hz), 4.63 (2H, s), 4.73 (2H, s), 5.07 (1H, d, J=4Hz), 5.27, 5.60 (2H, ABq, J=14Hz), 5.87 (1H, d, J=4Hz), 7.90—8.17 (1H, m), 8.37—8.60 (1H, m), 9.0—9.3 (2H, m).

(17) 7 - [2 - Ethoxycarbonylmethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp 155 to 160°C (dec.).

IR (Nujol): 3400—3150, 1770, 1670, 1610 $cm^{-1}$.

NMR ($DMSO-d_6+D_2O$, δ): 1.23 (3H, t, J=7Hz), 3.11, 3.67 (2H, ABq, J=18Hz), 4.23 (2H, q, J=7Hz), 4.82 (4H, bs), 5.17 (1H, d, J=5Hz), 5.30, 5.73 (2H, ABq, J=13Hz), 5.83 (1H, d, J=5Hz), 8.17 (1H, m), 8.60 (1H, m), 9.23 (2H, m).

(18) 7 - [2 - t - Butoxycarbonylmethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp 145 to 150°C (dec.).

IR (Nujol): 3300, 1775, 1740, 1670, 1610, 1525 $cm^{-1}$.

NMR ($DMSO-d_6+D_2O$, δ): 1.50 (9H, s), 3.23, 3.60 (2H, ABq, J=17Hz), 4.65 (2H, s), 4.80 (2H, s), 5.13 (1H, d, J=4Hz), 5.37, 5.67 (2H, ABq, J=14Hz), 5.80 (1H, d, J=4Hz), 8.23 (2H, s), 8.0—8.27 (1H, m), 8.43—8.67 (1H, m), 9.30—9.50 (2H, m).

(19) 7 - [2 - (1 - Methyl - 1 - ethoxycarbonylethoxyimino) - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp 155 to 160°C (dec.).

IR (Nujol): 3400—3200, 1780, 1740, 1680 $cm^{-1}$.

NMR ($DMSO-d_6+D_2O$, δ): 1.16 (3H, t, J=7Hz), 1.48 (6H, s), 3.16, 3.62 (2H, ABq, J=18Hz), 4.16 (2H, q, J=7Hz), 4.80 (2H, s), 5.14 (1H, d, J=5Hz), 5.2—5.8 (2H, m), 5.80 (1H, d, J=5Hz), 8.20 (1H, m), 8.56 (1H, m), 9.32 (2H, m).

(20) 7 - [2 - Ethoxyimino - 2 - (5 - amino - 1,2,4 - thiadiazol - 3 - yl)acetamido] - 3 - [4 - (N - hydroxycarbamoyl) - 1 - pyridiniomethyl] - 3 - cephem - 4 - carboxylate (syn isomer), mp 178 to 185°C (dec.).

IR (Nujol): 3250, 3180, 1770, 1670, 1640, 1605, 1530 $cm^{-1}$.

NMR ($D_2O$, δ): 1.29 (3H, t, J=7Hz), 3.20 and 3.72 (2H, ABq, J=19Hz), 4.30 (2H, q, J=7Hz), 5.28 (1H, d, J=5Hz), 5.37 and 5.70 (2H, ABq, J=14Hz), 5.88 (1H, d, J=5Hz), 8.31 (2H, d, J=7Hz), 9.13 (2H, d, J=7Hz).

(21) 7 - [2 - Ethoxyimino - 2 - (5 - amino - 1,2,4 - thiadiazol - 3 - yl)acetamido] - 3 - (4 - butoxycarbonyl - 1 - pyridiniomethyl) - 3 - cephem - 4 - carboxylate (syn isomer), mp 154 to 159°C (dec.).

IR (Nujol): 3300, 3160, 1775, 1728, 1670, 1605, 1525 $cm^{-1}$.

NMR (DMSO-$d_6$+$D_2O$, δ): 0.8—2.0 (10H, m), 3.13 and 3.53 (2H, ABq, J=18Hz), 4.0—4.6 (4H, m), 5.08 (1H, d, J=5Hz), 5.33 and 5.76 (2H, ABq, J=14Hz), 5.73 (1H, d, J=5Hz), 8.55 (2H, d, J=6Hz), 9.72 (2H, d, J=6Hz).

(22) 7 - [2 - Ethoxyimino - 2 - (5 - amino - 1,2,4 - thiadiazol - 3 - yl)acetamido] - 3 - (4 - t - butoxycarbonyl - 1 - pyridiniomethyl) - 3 - cephem - 4 - carboxylate (syn isomer), mp 154 to 157°C (dec.).

IR (Nujol): 3300, 3150, 1775, 1725, 1670, 1610, 1525 $cm^{-1}$.

NMR (DMSO-$d_6$+$D_2O$, δ): 1.23 (3H, t, J=6.5Hz), 1.60 (9H, s), 3.25 and 3.63 (2H, ABq, J=18Hz), 4.17 (2H, q, J=6.5Hz), 5.10 (1H, d, J=5Hz), 5.30 and 5.83 (2H, ABq, J=14Hz), 5.72 (1H, d, J=5Hz), 8.52 (2H, d, J=6Hz), 9.68 (2H, d, J=6Hz).

(23) 7 - [2 - Ethoxyimino - 2 - (5 - amino - 1,2,4 - thiadiazol - 3 - yl)acetamido] - 3 - [4 - (N - butylcarbamoyl) - 1 - pyridiniomethyl] - 3 - cephem - 4 - carboxylate (syn isomer), mp 161 to 162°C (dec.).

IR (Nujol): 3270, 1775, 1655, 1610, 1560, 1530 $cm^{-1}$.

NMR (DMSO-$d_6$, δ): 0.90 (3H, t, J=7Hz), 1.20 (3H, t, J=7Hz), 1.1—1.8 (4H, m), 2.9—3.9 (4H, m), 4.15 (2H, q, J=7Hz), 5.08 (1H, d, J=5Hz), 5.28 and 5.73 (2H, ABq, J=14Hz), 5.72 (1H, 2d, J=5 and 8Hz), 8.15 (2H, bs), 8.53 (2H, d, J=6Hz), 9.48 (1H, d, J=8Hz), 9.60 (2H, d, J=6Hz).

(24) 7 - [2 - Ethoxyimino - 2 - (5 - amino - 1,2,4 - thiadiazol - 3 - yl)acetamido] - 3 - [4 - (N - t - butylcarbamoyl) - 1 - pyridiniomethyl] - 3 - cephem - 4 - carboxylate (syn isomer), mp 165 to 167°C (dec.).

IR (Nujol): 3270, 1775, 1660, 1610, 1530 $cm^{-1}$.

NMR (DMSO-$d_6$+$D_2O$, δ): 1.22 (3H, t, J=7Hz), 1.42 (9H, s), 3.16 and 3.50 (2H, ABq, J=18Hz), 4.17 (2H, q, J=7Hz), 5.08 (1H, d, J=5Hz), 5.25 and 5.68 (2H, ABq, J=14Hz), 5.72 (1H, d, J=5Hz), 8.47 (2H, d, J=7Hz), 9.62 (2H, d, J=7Hz).

(25) 7 - [2 - Ethoxyimino - 2 - (5 - amino - 1,2,4 - thiadiazol - 3 - yl)acetamido] - 3 - [4 - (N - dodecylcarbamoyl) - 1 - pyridiniomethyl] - 3 - cephem - 4 - carboxylate (syn isomer), mp 176 to 177°C (dec.).

IR (Nujol): 3270, 1770, 1655, 1610, 1540 $cm^{-1}$.

NMR (DMSO-$d_6$+$D_2O$, δ): 0.85 (3H, t, J=5Hz), 1.0—1.8 (23H, m), 3.18 and 3.57 (2H, ABq, J=18Hz), 3.1—3.9 (2H, m), 4.15 (2H, q, J=7Hz), 5.0—6.0 (4H, m), 8.6 (2H, bs), 9.6 (2H, bs).

(26) 7 - [2 - Ethoxyimino - 2 - (5 - amino - 1,2,4 - thiadiazol - 3 - yl)acetamido] - 3 - (4 - octylcarbonyl - 1 - pyridiniomethyl] - 3 - cephem - 4 - carboxylate (syn isomer), mp 155 to 158°C (dec.).

IR (Nujol): 3270, 1775, 1735, 1680, 1615, 1520 $cm^{-1}$.

NMR (DMSO-$d_6$+$D_2O$, δ): 0.86 (3H, t, J=6Hz), 1.0—1.7 (15H, m), 3.25 and 3.53 (2H, ABq, J=18Hz), 3.9—4.7 (4H, m), 5.15 (1H, d, J=5Hz), 5.50 and 5.72 (2H, ABq, J=14Hz), 5.83 (1H, d, J=5Hz), 8.57 (2H, d, J=6Hz), 9.62 (2H, d, J=6Hz).

(27) 7 - [2 - Ethoxyimino - 2 - (5 - amino - 1,2,4 - thiadiazol - 3 - yl)acetamido] - 3 - [4 - (N - octylcarbamoyl) - 1 - pyridiniomethyl] - 3 - cephem - 4 - carboxylate (syn isomer), mp 181 to 183°C (dec.).

IR (Nujol): 3250, 1770, 1655, 1610, 1565, 1550, 1530 $cm^{-1}$.

NMR (DMSO-$d_6$+$D_2O$, δ): 0.86 (3H, t, J=6Hz), 0.9—1.7 (15H, m), 2.9—3.6 (4H, m), 4.15 (2H, q, J=6Hz), 5.15 (1H, d, J=5Hz), 5.15 and 5.77 (2H, ABq, J=14Hz), 5.77 (1H, d, J=5Hz), 8.62 (2H, d, J=6Hz), 9.51 (2H, d, J=6Hz).

(28) 7 - [2 - Ethoxyimino - 2 - (5 - amino - 1,2,4 - thiadiazol - 3 - yl)acetamido] - 3 - (3 - mesylamino - 1 - pyridiniomethyl)- 3 - cephem - 4 - carboxylate (syn isomer), mp 170 to 175°C (dec.).

IR (Nujol): 3400, 1760, 1670, 1610, 1520, 1150 $cm^{-1}$.

NMR (DMSO-$d_6$, δ): 1.17 (3H, t, J=7Hz), 2.87 (3H, s), 3.20 and 3.47 (2H, ABq, J=16Hz), 4.10 (2H, q, J=7Hz), 5.10 (1H, d, J=4Hz), 5.17 and 5.47 (2H, ABq, J=14Hz), 5.80 (1H, 2d, J=4 and 8Hz), 7.6—8.5 (4H, m), 9.47 (1H, d, J=8Hz).

(29) 7 - [2 - Ethoxyimino - 2 - (5 - amino - 1,2,4 - thiadiazol - 3 - yl)acetamido] - 3 - (4 - ethoxycarbonyl - 1 - pyridiniomethyl) - 3 - cephem - 4 - carboxylate (syn isomer), mp 150 to 155°C (dec.).

IR (Nujol): 3320, 3160, 1775, 1730, 1670, 1610, 1530, 1290 $cm^{-1}$.

NMR (DMSO-$d_6$+$D_2O$, δ): 1.21 (3H, t, J=7Hz), 1.36 (3H, t, J=7Hz), 3.12, 3.52 (2H, ABq, J=18Hz), 4.12 (2H, q, J=7Hz), 4.43 (2H, q, J=7Hz), 5.06 (1H, d, J=5Hz), 5.33, 5.68 (2H, ABq, J=14Hz), 5.71 (1H, d, J=5Hz), 8.47 (2H, d, J=6Hz), 9.60 (2H, d, J=6Hz).

Example 8

A solution of 7-[2-ethoxyimino-2-(5-amino-1,2,4-thiadiazol-3-yl)acetamido]-3-(4-t-butoxycarbonylaminomethyl-1-pyridiniomethyl)-3-cephem-4-carboxylate (syn isomer) (1.23 g) in formic acid (20 ml) was stirred for 6.5 hours at ambient temperature. The solution was evaporated to dryness under reduced pressure and the residue was dissolved in water. An insoluble material was filtered off and the filtrate was subjected to column chromatography on a non-ionic adsorption resin "Diaion HP—20" (50 ml). The elution

was carried out with water. The eluates containing an object compound were collected and lyophilized to give 7 - [2 - ethoxyimino - 2 - (5 - amino - 1,2,4 - thiadiazol - 3 - yl)acetamido] - 3 - (4 - aminomethyl - 1 - pyridiniomethyl) - 3 - cephem - 4 - carboxylate diformate (syn isomer) (0.37 g), mp 149 to 159°C (dec.).

IR (Nujol): 3250, 3150, 1770, 1660, 1640, 1580, 1520, 1040 $cm^{-1}$.

NMR ($D_2O$, δ): 1.22 (3H, t, J=7Hz), 3.3—3.9 (2H, m), 4.05 (2H, broad s), 4.17 (2H, q, J=7Hz), 4.9—5.6 (2H, m) 5.06 (1H, d, J=4Hz), 5.70 (1H, d, J=4Hz), 7.45 (2H, d, J=5Hz), 8.26 (2H, s), 8.55 (2H, d, J=5Hz).

Example 9

To a cold solution of phosphorus pentachloride (3.2 g) in methylene chloride (45 ml) was added 2 - t - butoxycarbonylmethoxyimino - 2 - (5 - amino - 1,2,4 - thiadiazol - 3 - yl)acetic acid (syn isomer) (4.2 g) at −18°C and the mixture was stirred for 45 minutes at −12 to −10°C. To the reaction mixture was added diisopropyl ether (150 ml) at −10°C and the mixture was stirred for several minutes and the resulting precipitate was collected by filtration. On the other hand, a mixture of 7-amino-3-(3-hydroxymethyl-1-pyridiniomethyl)-3-cephem-4-carboxylate dihydrochloride (4.0 g) and trimethylsilylacetamide (20 g) in methylene chloride (40 ml) was stirred for 30 minutes at room temperature and cooled to −20°C. To the cold solution was added the precipitate obtained above and the mixture was stirred for 30 minutes at −10 to −5°C. The reaction mixture was poured into an aqueous solution (100 ml) of sodium bicarbonate (5.9 g) and stirred for 30 minutes at room temperature. The aqueous layer was separated out. To the aqueous solution was added ethyl acetate and the mixture was adjusted to pH 1.5 with 6N hydrochloric acid. The aqueous layer was separated out, washed with ethyl acetate and subjected to column chromatography on a non ionic adsorption resin "Diaion HP—20" (100 ml). After the column was washed with water. The elution was carried out with 40% aqueous methanol. The eluates containing an object compound were collected, evaporated to remove methanol under reduced pressure and lyophilized to give 7 - [2-t - butoxycarbonylmethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer) (2.2 g), mp 145 to 150°C (dec.).

IR (Nujol): 3300, 1775, 1740, 1670, 1610, 1525 $cm^{-1}$.

NMR (DMSO-$d_6$+$D_2O$, δ): 1.50 (9H, s), 3.23, 3.60 (2H, ABq, J=17Hz), 4.65 (2H, s), 4.80 (2H, s), 5.13 (1H, d, J=4Hz), 5.37, 5.67 (2H, ABq, J=14Hz), 5.80 (1H, d, J=4Hz), 8.23 (2H, s), 8.0—8.27 (1H, m), 8.43—8.67 (1H, m), 9.30—9.50 (2H, m).

Example 10

The following compounds were obtained according to a similar manner to that of Example 9.

(1) 7 - [2-Ethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp 160 to 165°C (dec.).

IR (Nujol): 3300, 1770, 1660, 1610, 1520 $cm^{-1}$.

(2) 7 - [2-Ethoxyimino - 2 - (5 - amino - 1,2,4 - thiadiazol - 3 - yl)acetamido] - 3 - (3 - hydroxy - 1 - pyridiniomethyl) - 3 - cephem - 4 - carboxylate (syn isomer), mp 175 to 180°C (dec.).

IR (Nujol): 3390, 3290, 3180, 3050, 1770, 1660, 1625, 1610, 1525 $cm^{-1}$.

(3) 7 - [2-Ethoxyimino - 2 - (5 - amino - 1,2,4 - thiadiazol - 3 - yl)acetamido] - 3 - (4 - cyano - 1 - pyridiniomethyl) - 3 - cephem - 4 - carboxylate (syn isomer), mp 147 to 151°C (dec.).

IR (Nujol): 3400—3100, 1780, 1670, 1610, 1530, 1040 $cm^{-1}$.

(4) 7 - [2-Ethoxyimino - 2 - (5 - amino - 1,2,4 - thiadiazol - 3 - yl)acetamido] - 3 - (4 - hydroxymethyl - 1 - pyridiniomethyl) - 3 - cephem - 4 - carboxylate (syn isomer), mp 150 to 155°C (dec.).

IR (Nujol): 3250, 1770, 1660, 1630, 1605, 1570, 1520 $cm^{-1}$.

(5) 7 - [2-Ethoxyimino - 2 - (5 - amino - 1,2,4 - thiadiazol - 3 - yl)acetamido] - 3 - [4 - (3 - hydroxypropyl) - 1 - pyridiniomethyl] - 3 - cephem - 4 - carboxylate (syn isomer), mp 159 to 167°C (dec.).

IR (Nujol): 3400—3100, 1770, 1670—1610, 1540 $cm^{-1}$.

(6) 7 - [2-Ethoxyimino - 2 - (5 - amino - 1,2,4 - thiadiazol - 3 - yl)acetamido] - 3 - (3 - chloro - 1 - pyridiniomethyl) - 3 - cephem - 4 - carboxylate (syn isomer), mp 210 to 220°C (dec.).

IR (Nujol): 3400—3100, 1770, 1660, 1610, 1520 $cm^{-1}$.

(7) 7 - [2-Ethoxyimino - 2 - (5 - amino - 1,2,4 - thiadiazol - 3 - yl)acetamido] - 3 - (3 - methyl - 1 - pyridiniomethyl) - 3 - cephem - 4 - carboxylate (syn isomer), mp 175 to 180°C (dec.).

IR (Nujol): 3280, 1770, 1660, 1610, 1530, 1035 $cm^{-1}$.

(8) 7 - [2-Ethoxyimino - 2 - (5 - amino - 1,2,4 - thiadiazol - 3 - yl)acetamido] - 3 - (3,5 - dimethyl - 1 - pyridiniomethyl) - 3 - cephem - 4 - carboxylate (syn isomer), mp 165 to 175°C (dec.).

IR (Nujol): 3400—3150, 1770, 1660, 1610, 1530 $cm^{-1}$.

(9) 7 - [2-Ethoxyimino - 2 - (5 - amino - 1,2,4 - thiadiazol - 3 - yl)acetamido] - 3 - (4 - t - butoxycarbonylaminomethyl - 1 - pyridiniomethyl) - 3 - cephem - 4 - carboxylate (syn isomer), mp 154 to 158°C (dec.).

IR (Nujol): 3600, 1775, 1670, 1640, 1610, 1520, 1280, 1035 $cm^{-1}$.

(10) 7 - [2-Ethoxyimino - 2 - (5 - amino - 1,2,4 - thiadiazol - 3 - yl)acetamido] - 3 - (4 - amino-methylmethyl - 1 - pyridiniomethyl) - 3 - cephem - 4 - carboxylate diformate (syn isomer), mp 149 to 159°C (dec.).

IR (Nujol): 3250, 3150, 1770, 1660, 1640, 1580, 1520, 1040 $cm^{-1}$.

(11) 7 - [2-Ethoxyimino - 2 - (5 - amino - 1,2,4 - thiadiazol - 3 - yl)acetamido] - 3 - (4 - t - butyl - 1 - pyridiniomethyl) - 3 - cephem - 4 - carboxylate (syn isomer), mp 169.5 to 175.0°C (dec.).

IR (Nujol): 3280, 1770, 1670, 1630, 1610, 1530 $cm^{-1}$.

(12) 7 - [2-Cyclopentyloxyimino - 2 - (5 - amino - 1,2,4 - thiadiazol - 3 - yl)acetamido] - 3 - (4 - hydroxymethyl - 1 - pyridiniomethyl) - 3 - cephem - 4 - carboxylate (syn isomer), mp 155 to 160°C (dec.).

IR (Nujol): 3350, 3200, 1775, 1670, 1615, 1525 $cm^{-1}$.

(13) 7 - [2-Cyclopentyloxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp 160 to 165°C (dec.).

IR (Nujol): 3260, 3170, 1770, 1655, 1610, 1520 $cm^{-1}$.

(14) 7 - [2-Cyclopentyloxyimino - 2 - (5 - amino - 1,2,4 - thiadiazol - 3 - yl)acetamido] - 3 - (4 - acetyl - 1 - pyridiniomethyl) - 3 - cephem - 4 - carboxylate (syn isomer), mp 145 to 150°C (dec.).

IR (Nujol): 3270, 1770, 1700, 1670, 1610, 1520 $cm^{-1}$.

(15) 7 - [2-Cyclopentyloxyimino - 2 - (5 - amino - 1,2,4 - thiadiazol - 3 - yl)acetamido] - 3 - (3 - carboxymethyl - 1 - pyridiniomethyl) - 3 - cephem - 4 - carboxylate (syn isomer), mp 165 to 170°C (dec.).

IR (Nujol): 3350, 3200, 1775, 1720, 1670, 1620, 1525 $cm^{-1}$.

(16) 7 - [2-Cyclopentyloxyimino - 2 - (5 - amino - 1,2,4 - thiadiazol - 3 - yl)acetamido] - 3 - (4 - hydroxyiminomethyl - 1 - pyridiniomethyl) - 3 - cephem - 4 - carboxylate (syn isomer), mp 140 to 145°C (dec.).

IR (Nujol): 3280, 3160, 1770, 1680, 1630, 1610, 1520 $cm^{-1}$.

(17) 7 - [2-Cyclopentyloxyimino - 2 - (5 - amino - 1,2,4 - thiadiazol - 3 - yl)acetamido] - 3 - [4 - methyl - 5 - (2 - hydroxyethyl) - 3 - thiazoliomethyl] - 3 - cephem - 4 - carboxylate (syn isomer), mp 150 to 155°C (dec.).

IR (Nujol): 3330, 1780, 1670, 1610, 1530 $cm^{-1}$.

(18) 7 - [2 - (2 - Cyclopenten - 1 - yloxyimino) - 2 - (5 - amino - 1,2,4 - thiadiazol - 3 - yl)acetamido] - 3 - (4 - hydroxymethyl - 1 - pyridiniomethyl) - 3 - cephem - 4 - carboxylate (syn isomer), mp 140 to 145°C (dec.).

IR (Nujol): 3300, 3200, 1770, 1660, 1620, 1520 $cm^{-1}$.

(19) 7 - [2 - (2 - Cyclopenten - 1 - yloxyimino) - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp 143 to 148°C (dec.).

IR (Nujol): 3300, 3200, 1770, 1660, 1610, 1520 $cm^{-1}$.

(20) 7 - [2 - Carboxymethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp 170 to 175°C (dec.).

IR (Nujol): 3300, 1770, 1670, 1620, 1525 $cm^{-1}$.

(21) 7 - [2 - Ethoxycarbonylmethoxyimino - 2 - (5 - amino - 1,2,4 - thiadiazol - 3 - yl)acetamido] - 3 - (3-hydroxymethyl - 1 - pyridiniomethyl) - 3 - cephem - 4 - carboxylate (syn isomer), mp 155 to 160°C (dec.).

IR (Nujol): 3400—3150, 1770, 1670, 1610 $cm^{-1}$.

NMR ($DMSO-d_6+D_2O$, δ): 1.23 (3H, t, J=7Hz), 3.11, 3.67 (2H, ABq, J=18Hz), 4.23 (2H, q, J=7Hz), 4.82 (4H, bs), 5.17 (1H, d, J=5Hz), 5.30, 5.73 (2H, ABq, J=13Hz), 5.83 (1H, d, J=5Hz), 8.17 (1H, m), 8.60 (1H, m), 9.23 (2H, m).

(22) 7 - [2 - Ethoxyimino - 2 - (5 - amino - 1,2,4 - thiadiazol - 3 - yl)acetamido] - 3 - (3 - mesylamino - 1 - pyridiniomethyl) - 3 - cephem - 4 - carboxylate (syn isomer), mp 170 to 175°C (dec.).

IR (Nujol): 3400, 1760, 1670, 1610, 1520, 1150 $cm^{-1}$.

(23) 7 - [2 - (1 - Methyl - 1 - ethoxycarbonylethoxyimino) - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp 155 to 160°C (dec.).

IR (Nujol): 3400—3200, 1780, 1740, 1680 $cm^{-1}$.

NMR ($DMSO-d_6+D_2O$, δ): 1.16 (3H, t, J=7Hz), 1.48 (6H, s), 3.16, 3.62 (2H, ABq, J=18Hz), 4.16 (2H, q, J=7Hz), 4.80 (2H, s), 5.14 (1H, d, J=5Hz), 5.2—5.8 (2H, m), 5.80 (1H, d, J=5Hz), 8.20 (1H, m), 8.56 (1H, m), 9.32 (2H, m).

(24) 7 - [2 - Ethoxyimino - 2 - (5 - amino - 1,2,4 - thiadiazol - 3 - yl)acetamido] - 3 - (3 - thiazoliomethyl) - 3 - cephem - 4 - carboxylate (syn isomer), mp 155 to 160°C (dec.).

IR (Nujol): 3400—3200, 1770, 1660, 1600, 1520 $cm^{-1}$.

(25) 7 - [2 - Ethoxyimino - 2 - (5 - amino - 1,2,4 - thiadiazol - 3 - yl)acetamido] - 3 - [4 - (N - hydroxycarbamoyl) - 1 - pyridiniomethyl] - 3 - cephem - 4 - carboxylate (syn isomer), mp 178 to 185°C (dec.).

IR (Nujol): 3250, 3180, 1770, 1670, 1640, 1605, 1530 $cm^{-1}$.

(26) 7 - [2 - Ethoxyimino - 2 - (5 - amino - 1,2,4 - thiadiazol - 3 - yl)acetamido] - 3 - (4 - butoxycarbonyl - 1 - pyridiniomethyl) - 3 - cephem - 4 - carboxylate (syn isomer), mp 154 to 159°C (dec.).

IR (Nujol): 3300, 3160, 1775, 1728, 1670, 1605, 1525 $cm^{-1}$.

(27) 7 - [2 - Ethoxyimino - 2 - (5 - amino - 1,2,4 - thiadiazol - 3 - yl)acetamido] - 3 - (4 - t - butoxycarbonyl - 1 - pyridiniomethyl) - 3 - cephem - 4 - carboxylate (syn isomer), mp 154 to 157°C (dec.).

IR (Nujol): 3300, 3150, 1775, 1725, 1670, 1610, 1525 $cm^{-1}$.

(28) 7 - [2 - Ethoxyimino - 2 - (5 - amino - 1,2,4 - thiadiazol - 3 - yl)acetamido] - 3 - [4 - (N - butylcarbamoyl) - 1 - pyridiniomethyl] - 3 - cephem - 4 - carboxylate (syn isomer), mp 161 to 162°C (dec.).

IR (Nujol): 3270, 1775, 1655, 1610, 1560, 1530 $cm^{-1}$.

(29) 7 - [2 - Ethoxyimino - 2 - (5 - amino - 1,2,4 - thiadiazol - 3 - yl)acetamido] - 3 - [4 - (N - t - butylcarbamoyl) - 1 - pyridiniomethyl] - 3 - cephem - 4 - carboxylate (syn isomer), mp 165 to 167°C (dec.).

IR (Nujol): 3270, 1775, 1660, 1610, 1530 $cm^{-1}$.

(30) 7 - [2 - Ethoxyimino - 2 - (5 - amino - 1,2,4 - thiadiazol - 3 - yl)acetamido] - 3 - (4 - dodecyloxycarbonyl - 1 - pyridiniomethyl) - 3 - cephem - 4 - carboxylate (syn isomer), mp 152 to 157°C (dec.).

IR (Nujol): 3280, 3160, 1775, 1730, 1675, 1610, 1520 $cm^{-1}$.

(31) 7 - [2 - Ethoxyimino - 2 - (5 - amino - 1,2,4 - thiadiazol - 3 - yl)acetamido] - 3 - [4 - (N - dodecylcarbamoyl) - 1 - pyridiniomethyl] - 3 - cephem - 4 - carboxylate (syn isomer), mp 176 to 177°C (dec.).

IR (Nujol): 3270, 1770, 1655, 1610, 1540 $cm^{-1}$.

(32) 7 - [2 - Ethoxyimino - 2 - (5 - amino - 1,2,4 - thiadiazol - 3 - yl)acetamido] - 3 - [4 - octyloxycarbonyl - 1 - pyridiniomethyl) - 3 - cephem - 4 - carboxylate (syn isomer), mp 155 to 158°C (dec.).

IR (Nujol): 3270, 1775, 1735, 1680, 1615, 1520 $cm^{-1}$.

(33) 7 - [2 - Ethoxyimino - 2 - (5 - amino - 1,2,4 - thiadiazol - 3 - yl)acetamido] - 3 - [4 - (N - octylcarbamoyl) - 1 - pyridiniomethyl] - 3 - cephem - 4 - carboxylate (syn isomer), mp 181 to 183°C (dec.).

IR (Nujol): 3250, 1770, 1655, 1610, 1565, 1550, 1530 $cm^{-1}$.

(34) 7 - [2 - Ethoxyimino - 2 - (5 - amino - 1,2,4 - thiadiazol - 3 - yl)acetamido] - 3 - (4 - ethoxycarbonyl - 1 - pyridiniomethyl) - 3 - cephem - 4 - carboxylate (syn isomer), mp 150 to 155°C (dec.).

IR (Nujol): 3320, 3160, 1775, 1730, 1670, 1610, 1530, 1290 $cm^{-1}$.

NMR ($DMSO-d_6+D_2O$, δ): 1.21 (3H, t, J=7Hz), 1.36 (3H, t, J=7Hz), 3.12, 3.52 (2H, ABq, J=18Hz), 4.12 (2H, q, J=7Hz), 4.43 (2H, q, J=7Hz), 5.06 (1H, d, J=5Hz), 5.33, 5.68 (2H, ABq, J=14Hz), 5.71 (1H, d, J=5Hz), 8.47 (2H, d, J=6Hz), 9.60 (2H, d, J=6Hz).

## Example 11

To a cold mixture of trifluoroacetic acid (14 ml) and anisole (2.5 ml) was added 7 - [2 - t - butoxycarbonylmethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer) (2.0 g) under stirring and the mixture was stirred for 80 minutes at room temperature. The mixture was evaporated to remove trifluoroacetic acid and the residue was triturated with isopropyl alcohol to give a powder. The powder was dissolved in water. The aqueous solution was subjected to a column chromatography on a non ionic adsorption resin "Diaion HP—20" (100 ml). After the column was washed with water, the elution was carried out with 15% aqueous methanol. The eluates containing an object compound were collected, evaporated to remove methanol under reduced pressure and lyophilized to give 7 - [2 - carboxymethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer) (1.05 g), mp 170 to 175°C (dec.).

IR (Nujol): 3300, 1770, 1670, 1620, 1525 $cm^{-1}$.

NMR ($DMSO-d_6+D_2O$, δ): 3.20, 3.50 (2H, ABq, J=18Hz), 4.63 (2H, s), 4.73 (2H, s), 5.07 (1H, d, J=4Hz), 5.27, 5.60 (2H, ABq, J=14Hz), 5.87 (1H, d, J=4Hz), 7.90—8.17 (1H, m), 8.37—8.60 (1H, m), 9.0—9.3 (2H, m).

## Preparation 13

To a solution of phosphorus pentachloride (54.6 g) in methylene chloride (500 ml) was added 2 - ethoxyimino - 2 - (5 - amino - 1,2,4 - thiadiazol - 3 - yl)acetic acid (syn isomer) (54.0 g) under stirring and cooling at −20°C. The mixture was stirred for 30 minutes at −15 to −12°C and for 2 hours at −5°C. To the mixture containing precipitates of an object compound was added diisopropyl ether (500 ml) at −5°C and the mixture was stirred for 30 minutes at −5 to 10°C. The resulting precipitates were collected by filtration, washed with diisopropyl ether and dried to give 2 - ethoxyimino - 2 - (5 - amino - 1,2,4 - thiadiazol - 3 - yl)acetyl chloride hydrochloride (syn isomer) (60.17 g), mp. 125 to 127°C (dec.).

IR (Nujol): 1785, 1625, 1055 $cm^{-1}$.

Analysis for $C_6H_8O_2N_4SCl_2$

| | C | H | N | S | Cl |
|---|---|---|---|---|---|
| calc'd: | 26.57 | 2.95 | 20.66 | 11.81 | 26.20 |
| found: | 26.13 | 2.99 | 20.49 | 11.77 | 26.41 |

## Preparation 14

To a suspension of diphenylmethyl 7-amino-3-chloromethyl-3-cephem-4-carboxylate hydrochloride (27 g) in methylene chloride (300 ml) was added N,N-dimethylaniline (36.2 g) under cooling in an ice bath at 5°C. To the solution was added portionwise 2-ethoxyimino-2-(5-amino-1,2,4-thiadiazol-3-yl)acetyl chloride hydrochloride (syn isomer) (16.2 g) below 11°C and the mixture was stirred for 45 minutes at 5°C. The reaction mixture was diluted with a mixed solvent of methylene chloride (100 ml) and water (200 ml) and adjusted to pH 2 with 1N hydrochloric acid. The organic layer was separated out, washed with water, dried over magnesium sulfate and evaporated to dryness. The residue was triturated in diethyl ether to give diphenylmethyl 7 - [2 - ethoxyimino - 2 - (5 - amino - 1,2,4 - thiadiazol - 3 - yl)acetamido] - 3 -

chloromethyl - 3 - cephem - 4 - carboxylate (syn isomer) (32.4 g), mp. 120 to 125°C (dec.).

IR (Nujol): 3300, 3150, 1780, 1725, 1675, 1625, 1530, 1495 $cm^{-1}$.

NMR (DMSO-$d_6$, δ): 1.28 (3H, t, J=7Hz), 3.68 (2H, broad s), 4.23 (2H, q, J=7Hz), 4.47 (2H, s), 5.27 (1H, d, J=5Hz), 5.97 (1H, 2d, J=5 and 8Hz), 7.0 (1H, s), 7.2—7.7 (10H, m), 8.17 (2H, broad s), 9.62 (1H, d, J=8Hz).

Preparation 15

To a cold solution of diphenylmethyl 7 - [2 - ethoxyimino - 2 - (5 - amino - 1,2,4 - thiadiazol - 3 - yl)-acetamido] - 3 - chloromethyl - 3 - cephem - 4 - carboxylate (syn isomer) (10 g) in a mixed solution of methylene chloride (100 ml) and acetic acid (10 ml) were added 30% hydrogen peroxide (1.84 ml) and sodium tungstate (0.3 g).

The mixture was stirred for 45 minutes in an ice bath and poured into diethyl ether (300 ml). The precipitates were collected by filtration and washed with diethyl ether to give diphenylmethyl 7 - [2 - ethoxyimino - 2 - (5 - amino - 1,2,4 - thiadiazol - 3 - yl)acetamido] - 3 - chloromethyl - 3 - cephem - 4 - carboxylate - 1 - oxide (syn isomer) (8.9 g), mp 150 to 155°C (dec.).

IR (Nujol): 3280, 3170, 1785, 1723, 1667, 1628, 1530 $cm^{-1}$.

NMR (DMSO-$d_6$, δ): 1.30 (3H, t, J=7Hz), 3.90 (2H, broad s), 4.23 (2H, q, J=7Hz), 4.58 (2H, broad s), 5.12 (1H, d, J=5Hz), 6.10 (1H, 2d, J=5 and 8Hz), 7.02 (1H, s), 7.20—7.73 (10H, m), 8.15 (2H, broad s), 9.00 (1H, d, J=8Hz).

Preparation 16

To a cold solution of diphenylmethyl 7 - [2 - ethoxyimino - 2 - (5 - amino - 1,2,4 - thiadiazol - 3 - yl)-acetamido] - 3 - chloromethyl - 3 - cephem - 4 - carboxylate - 1 - oxide (syn isomer) (9.85 g) in acetone (222 ml) was added sodium iodide (8.22 g) and the mixture was stirred for 2 hours under cooling in an ice bath. The solvent was evaporated under reduced pressure and the residue was dissolved in a mixture of methylene chloride (200 ml) and water (100 ml). The organic layer was separated out, washed with an aqueous solution of sodium thiosulfate and water successively, dried over magnesium sulfate and evaporated to dryness. The residue was triturated in diethyl ether to give diphenylmethyl 7 - [2 - ethoxyimino - 2 - (5 - amino - 1,2,4 - thiadiazol - 3 - yl)acetamido] - 3 - iodomethyl - 3 - cephem - 4 - carboxylate - 1 - oxide (syn isomer) (10.39 g), mp. 159 to 163°C (dec.).

IR (Nujol): 3250, 3150, 1780, 1720, 1670, 1620, 1520 $cm^{-1}$.

NMR (DMSO-$d_6$, δ): 1.29 (3H, t, J=7Hz), 3.95 (2H, m), 4.25 (2H, q, J=7Hz), 4.50 (2H, m), 5.12 (1H, d, J=5Hz), 6.05 (1H, 2d, J=5 and 9Hz), 7.00 (1H, s), 7.4 (10H, m), 8.13 (2H, broad s), 8.96 (1H, d, J=9Hz).

Preparation 17

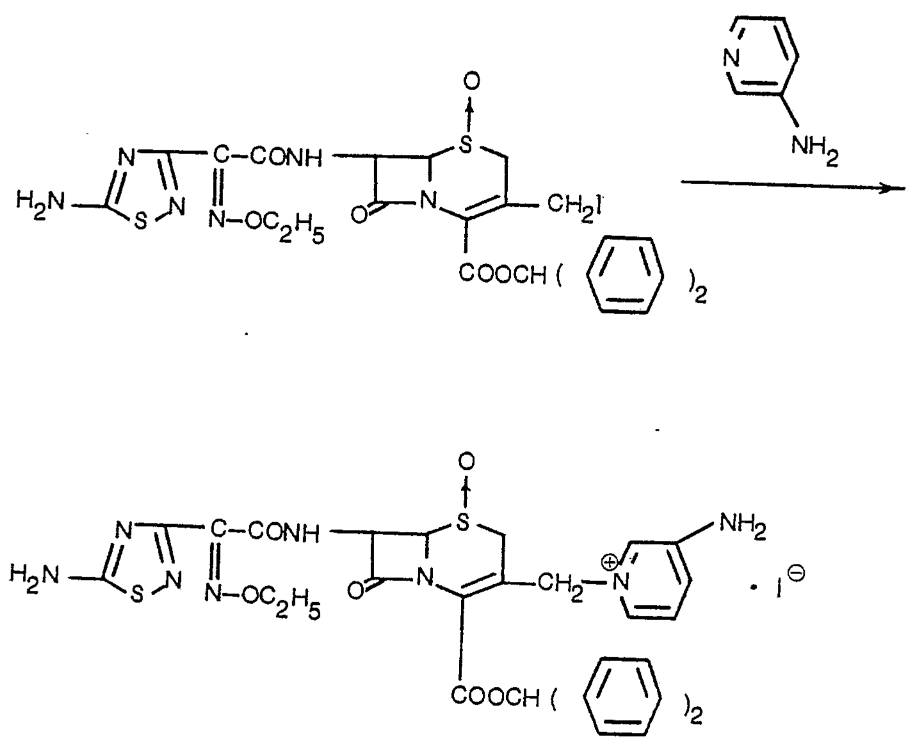

A mixture of diphenylmethyl 7 - [2 - ethoxyimino - 2 - (5 - amino - 1,2,4 - thiadiazol - 3 - yl)acetamido] - 3 - iodomethyl - 3 - cephem - 4 - carboxylate - 1 - oxide (syn isomer) (5.0 g) and 3-aminopyridine (0.717 g) in tetrahydrofuran (35 ml) was stirred for one hour at room temperature. The resulting

precipitates were collected by filtration, washed with tetrahydrofuran and diethyl ether and dried to give diphenylmethyl 7 - [2 - ethoxyimino - 2 - (5 - amino - 1,2,4 - thiadiazol - 3 - yl) - acetamido] - 3 - (3 - amino - 1 - pyridiniomethyl) - 3 - cephem - 4 - carboxylate - 1 - oxide iodide (syn isomer) (5.41 g), mp. 157 to 162°C (dec.).

IR (Nujol): 3400—3200, 1795, 1725, 1680, 1610, 1530, 1510, 1035, 705 $cm^{-1}$.

NMR ($DMSO-d_6$, δ): 1.26 (3H, t, J=7Hz), 3.3—4.0 (2H, m), 4.19 (2H, q, J=7Hz), 5.08 (1H, d, J=5Hz), 5.35 (2H, broad s), 6.10 (1H, 2d, J=5 and 8Hz), 6.6 (2H, m), 6.92 (1H, s), 7.1—7.7 (12H, m), 7.9 (2H, m), 8.03 (2H, broad s), 8.93 (1H, d, J=8Hz).

Preparation 18

To a mixture of diphenylmethyl 7 - [2 - ethoxyimino - 2 - (5 - amino - 1,2,4 - thiadiazol - 3 - yl)acetamido] - 3 - (3 - amino - 1 - pyridiniomethyl) - 3 - cephem - 4 - carboxylate - 1 - oxide iodide (syn isomer) (4.50 g) and N,N-dimethylaniline (1.34 g) in acetonitrile (45 ml) was dropped phosphorus trichloride (1.52 g) below 8°C under stirring and cooling in an ice bath. The mixture was stirred for 70 minutes at the same temperature and diethyl ether (50 ml) was added thereto. The resulting precipitates were collected by filtration, washed with diethyl ether and dried to give diphenylmethyl 7 - [2 - ethoxyimino - 2 - (5 - amino - 1,2,4 - thiadiazol - 3 - yl)acetamido] - 3 - (3 - amino - 1 - pyridiniomethyl) - 3 - cephem - 4 - carboxylate iodide (syn isomer) (5.05 g), mp. 85 to 95°C (dec.).

IR (Nujol): 3300, 3200, 1790, 1680, 1630, 1510, 1220, 1185 $cm^{-1}$.

NMR ($DMSO-d_6+D_2O$, δ): 1.23 (3H, t, J=7Hz), 3.2—3.8 (2H, m), 4.13 (2H, q, J=7Hz), 5.0—5.8 (4H, m), 6.87 (1H, s), 7.1—7.8 (12H, m), 7.8—8.1 (2H, m).

Preparation 19

A solution of diphenylmethyl 7 - [2 - ethoxyimino - 2 - (5 - amino - 1,2,4 - thiadiazol - 3 - yl)acetamido] - 3 - (3 - amino - 1 - pyridiniomethyl) - 3 - cephem - 4 - carboxylate iodide (syn isomer) (4.9 g) in a mixed solvent (12 ml) of acetone and methanol (1:1) was subjected to a column packed with an ionexchange resin [Amberlite IRA—400 (Trademark: prepared by Rohm & Haas Co.) trifluoroacetate-form] (50 ml). The elution was carried out with the same solvent (100 ml) and the eluate was evaporated to dryness. The residue was triturated in diethyl ether to give diphenylmethyl 7 - [2 - ethoxyimino - 2 - (5 - amino - 1,2,4 - thiadiazol - 3 - yl)acetamido] - 3 - (3 - amino - 1 - pyridiniomethyl) - 3 - cephem - 4 - carboxylate trifluoroacetate (syn isomer) (3.72 g), mp. 125 to 130°C (dec.).

IR (Nujol): 3300, 3200, 1790, 1690—1620, 1510, 1200, 1180, 1035 $cm^{-1}$.

NMR ($DMSO-d_6$, δ): 1.25 (3H, t, J=7Hz), 3.2—3.7 (2H, m), 4.19 (2H, q, J=7Hz), 5.1—5.5 (2H, m), 5.29 (1H, d, J=5Hz), 6.00 (1H, 2d, J=5 and 8Hz), 6.91 (1H, s), 7.1—7.7 (12H, m), 7.8—8.3 (4H, m), 9.73 (1H, d, J=8Hz).

Example 12

A mixture of diphenylmethyl 7 - [2 - ethoxyimino - 2 - (5 - amino - 1,2,4 - thiadiazol - 3 - yl)acetamido] - 3 - (3 - amino - 1 - pyridiniomethyl) - 3 - cephem - 4 - carboxylate trifluoroacetate (syn isomer) (500 mg) and anisole (1.0 ml) in trifluoroacetic acid (3 ml) was stirred for 35 minutes under cooling in an ice-salt bath. The mixture was poured into cold diisopropyl ether (20 ml) under stirring and resulting precipitates were collected by filtration. The powder was suspended in water (18 ml), adjusted to pH 4 to 5 with aqueous solution of sodium bicarbonate and an insoluble material was filtered off. The filtrate was subjected to column chromatography on a non ionic adsorption resin "Diaion HP 20" (Trademark: Prepared by Mitsubishi Chemical Industries) (20 ml). After the column was washed with water, the elution was carried out with 20% aqueous methanol. The eluates containing an object compound were collected, evaporated to remove methanol under reduced pressure and lyophilized to give 7 - [2 - ethoxyimino - 2 - (5 - amino - 1,2,4 - thiadiazol - 3 - yl)acetamido] - 3 - (3 - amino - 1 - pyridiniomethyl) - 3 - cephem - 4 - carboxylate (syn isomer) (100 mg), mp. 175 to 180°C (dec.).

IR (Nujol): 3300, 3200, 1770, 1660—1590, 1510, 1150, 1035 $cm^{-1}$.

NMR ($DMSO-d_6+D_2O$, δ): 1.24 (3H, t, J=7Hz), 3.04 and 3.53 (2H, ABq, J=18Hz), 4.16 (2H, q, J=7Hz), 5.06 and 5.61 (2H, ABq, J=14Hz), 5.10 (1H, d, J=5Hz), 5.73 (1H, 2d, J=5 and 8Hz), 6.6—7.2 (2H, m), 7.7 (2H, m), 8.22 (2H, broad s), 8.5—8.6 (2H, m), 9.51 (1H, d, J=8Hz).

Example 13

The following compounds were obtained according to a similar manner to that of Example 12.

(1) 7 - [2 - Ethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp. 160 to 165°C (dec.).

IR (Nujol): 3300, 1770, 1660, 1610, 1520 $cm^{-1}$.

(2) 7 - [2 - Ethoxyimino - 2 - (5 - amino - 1,2,4 - thiadiazol - 3 - yl)acetamido] - 3 - (3 - hydroxy - 1 - pyridiniomethyl) - 3 - cephem - 4 - carboxylate (syn isomer), mp. 175 to 180°C (dec.).

IR (Nujol): 3390, 3290, 3180, 3050, 1770, 1660, 1625, 1610, 1525 $cm^{-1}$.

(3) 7 - [2 - Ethoxyimino - 2 - (5 - amino - 1,2,4 - thiadiazol - 3 - yl)acetamido] - 3 - (4 - cyano - 1 - pyridiniomethyl) - 3 - cephem - 4 - carboxylate (syn isomer), mp. 147 to 151°C (dec.).

IR (Nujol): 3400—3100, 1780, 1670, 1610, 1530, 1040 $cm^{-1}$.

(4) 7 - [2 - Ethoxyimino - 2 - (5 - amino - 1,2,4 - thiadiazol - 3 - yl)acetamido] - 3 - (4 - hydroxymethyl - 1 - pyridiniomethyl) - 3 - cephem - 4 - carboxylate (syn isomer), mp. 150 to 155°C (dec.).

IR (Nujol): 3250, 1770, 1660, 1630, 1605, 1570, 1520 $cm^{-1}$.

(5) 7 - [2 - Ethoxyimino - 2 - (5 - amino - 1,2,4 - thiadiazol - 3 - yl)acetamido] - 3 - [4 - (3 - hydroxypropyl) - 1 - pyridiniomethyl) - 3 - cephem - 4 - carboxylate (syn isomer), mp. 159 to 167°C (dec.).

IR (Nujol): 3400—3100, 1770, 1670—1610, 1540 $cm^{-1}$.

(6) 7 - [2 - Ethoxyimino - 2 - (5 - amino - 1,2,4 - thiadiazol - 3 - yl)acetamido] - 3 - (3 - chloro - 1 - pyridiniomethyl) - 3 - cephem - 4 - carboxylate (syn isomer), mp. 210 to 220°C (dec.).

IR (Nujol): 3400—3100, 1770, 1660, 1610, 1520 $cm^{-1}$.

(7) 7 - [2 - Ethoxyimino - 2 - (5 - amino - 1,2,4 - thiadiazol - 3 - yl)acetamido] - 3 - (3 - methyl - 1 - pyridiniomethyl) - 3 - cephem - 4 - carboxylate (syn isomer), mp. 175 to 180°C (dec.).

IR (Nujol): 3280, 1770, 1660, 1610, 1530, 1035 $cm^{-1}$.

(8) 7 - [2 - Ethoxyimino - 2 - (5 - amino - 1,2,4 - thiadiazol - 3 - yl)acetamido] - 3 - (3,5 - dimethyl - 1 - pyridiniomethyl) - 3 - cephem - 4 - carboxylate (syn isomer), mp. 165 to 175°C (dec.).

IR (Nujol): 3400—3150, 1770, 1660, 1610, 1530 $cm^{-1}$.

(9) 7 - [2 - Ethoxyimino - 2 - (5 - amino - 1,2,4 - thiadiazol - 3 - yl)acetamido] - 3 - (4 - aminomethyl - 1 - pyridiniomethyl) - 3 - cephem - 4 - carboxylate diformate (syn isomer), mp. 149 to 159°C (dec.).

IR (Nujol): 3250, 3150, 1770, 1660, 1640, 1580, 1520, 1040 $cm^{-1}$.

(10) 7 - [2 - Ethoxyimino - 2 - (5 - amino - 1,2,4 - thiadiazol - 3 - yl)acetamido] - 3 - (4 - t - butyl - 1 - pyridiniomethyl) - 3 - cephem - 4 - carboxylate (syn isomer), mp. 169.5 to 175.0°C (dec.).

IR (Nujol): 3280, 1770, 1670, 1630, 1610, 1530 $cm^{-1}$.

(11) 7 - [2 - Cyclopentyloxyimino - 2 - (5 - amino - 1,2,4 - thiadiazol - 3 - yl)acetamido] - 3 - (4 - hydroxymethyl - 1 - pyridiniomethyl) - 3 - cephem - 4 - carboxylate (syn isomer), mp. 155 to 160°C (dec.).

IR (Nujol): 3350, 3200, 1775, 1670, 1615, 1525 $cm^{-1}$.

(12) 7 - [2 - Cyclopentyloxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp. 160 to 165°C (dec.).

IR (Nujol): 3260, 3170, 1770, 1655, 1610, 1520 $cm^{-1}$.

(13) 7 - [2 - Cyclopentyloxyimino - 2 - (5 - amino - 1,2,4 - thiadiazol - 3 - yl)acetamido] - 3 - (4 - acetyl - 1 - pyridiniomethyl) - 3 - cephem - 4 - carboxylate (syn isomer), mp. 145 to 150°C (dec.).

IR (Nujol): 3270, 1770, 1700, 1670, 1610, 1520 $cm^{-1}$.

(14) 7 - [2 - Cyclopentyloxyimino - 2 - (5 - amino - 1,2,4 - thiadiazol - 3 - yl)acetamido] - 3 - (3 - carboxymethyl - 1 - pyridiniomethyl) - 3 - cephem - 4 - carboxylate (syn isomer), mp. 165 to 170°C (dec.).

IR (Nujol): 3350, 3200, 1775, 1720, 1670, 1620, 1525 $cm^{-1}$.

(15) 7 - [2 - Cyclopentyloxyimino - 2 - (5 - amino - 1,2,4 - thiadiazol - 3 - yl)acetamido] - 3 - (4 - hydroxyiminomethyl - 1 - pyridiniomethyl) - 3 - cephem - 4 - carboxylate (syn isomer), mp. 140 to 145°C (dec.).

IR (Nujol): 3280, 3160, 1770, 1680, 1630, 1610, 1520 $cm^{-1}$.

(16) 7 - [2 - Cyclopentyloxyimino - 2 - (5 - amino - 1,2,4 - thiadiazol - 3 - yl)acetamido] - 3 - [4 - methyl - 5 - (2 - hydroxyethyl) - 3 - thiazoliomethyl] - 3 - cephem - 4 - carboxylate (syn isomer), mp. 150 to 155°C (dec.).

IR (Nujol): 3330, 1780, 1670, 1610, 1530 $cm^{-1}$.

(17) 7 - [2 - (2 - Cyclopenten - 1 - yloxyimino) - 2 - (5 - amino - 1,2,4 - thiadiazol - 3 - yl)acetamido] - 3 - (4 - hydroxymethyl - 1 - pyridiniomethyl) - 3 - cephem - 4 - carboxylate (syn isomer), mp. 140 to 145°C (dec.).

IR (Nujol): 3300, 3200, 1770, 1660, 1620, 1520 $cm^{-1}$.

(18) 7 - [2 - (2 - Cyclopenten - 1 - yloxyimino) - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp. 143 to 148°C (dec.).

IR (Nujol): 3300, 3200, 1770, 1660, 1610, 1520 $cm^{-1}$.

(19) 7 - [2 - Carboxymethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp. 170 to 175°C (dec.).

IR (Nujol): 3300, 1770, 1670, 1620, 1525 $cm^{-1}$.

(20) 7 - [2 - Ethoxycarbonylmethoxyimino - 2 - (5 - amino - 1,2,4 - thiadiazol - 3 - yl)acetamido] - 3 - (3 - hydroxymethyl - 1 - pyridiniomethyl) - 3 - cephem - 4 - carboxylate (syn isomer), mp. 155 to 160°C (dec.).

IR (Nujol): 3400—3150, 1770, 1670, 1610 $cm^{-1}$.

(21) 7 - [2 - Ethoxyimino - 2 - (5 - amino - 1,2,4 - thiadiazol - 3 - yl)acetamido] - 3 - (3 - mesylamino - 1 - pyridiniomethyl) - 3 - cephem - 4 - carboxylate (syn isomer), mp. 170 to 175°C (dec.).

IR (Nujol): 3400, 1760, 1670, 1610, 1520, 1150 $cm^{-1}$.

(22) 7 - [2 - (1 - methyl - 1 - ethoxycarbonylethoxyimino) - 2 - (5 - amino - 1,2,4 - thiadiazol - 3 -

yl)acetamido] - 3 - (3 - hydroxymethyl - 1 - pyridiniomethyl) - 3 - cephem - 4 - carboxylate (syn isomer), mp. 155 to 160°C (dec.).

IR (Nujol): 3400—3200, 1780, 1740, 1680 $cm^{-1}$.

(23) 7 - [2 - Ethoxyimino - 2 - (5 - amino - 1,2,4 - thiadiazol - 3 - yl)acetamido] - 3 - (3 - thiazoliomethyl) - 3 - cephem - 4 - carboxylate (syn isomer), mp. 155 to 160°C (dec.).

IR (Nujol): 3400—3200, 1770, 1660, 1600, 1520 $cm^{-1}$.

(24) 7 - [2 - Ethoxyimino - 2 - (5 - amino - 1,2,4 - thiadiazol - 3 - yl)acetamido] - 3 - [4 - (N - hydroxycarbamoyl) - 1 - pyridiniomethyl] - 3 - cephem - 4 - carboxylate (syn isomer), mp. 178 to 185°C (dec.).

IR (Nujol): 3250, 3180, 1770, 1670, 1640, 1605, 1530 $cm^{-1}$.

(25) 7 - [2 - Ethoxyimino - 2 - (5 - amino - 1,2,4 - thiadiazol - 3 - yl)acetamido] - 3 - (4 - butoxycarbonyl - 1 - pyridiniomethyl) - 3 - cephem - 4 - carboxylate (syn isomer), mp. 154 to 159°C (dec.).

IR (Nujol): 3300, 3160, 1775, 1728, 1670, 1605, 1525 $cm^{-1}$.

(26) 7 - [2 - Ethoxyimino - 2 - (5 - amino - 1,2,4 - thiadiazol - 3 - yl)acetamido] - 3 - [4 - (N - butylcarbamoyl) - 1 - pyridiniomethyl] - 3 - cephem - 4 - carboxylate (syn isomer), mp. 161 to 162°C (dec.).

IR (Nujol): 3270, 1775, 1655, 1610, 1560, 1530 $cm^{-1}$.

(27) 7 - [2 - Ethoxyimino - 2 - (5 - amino - 1,2,4 - thiadiazol - 3 - yl)acetamido] - 3 - [4 - (N - t - butylcarbamoyl) - 1 - pyridiniomethyl] - 3 - cephem - 4 - carboxylate (syn isomer), mp. 165 to 167°C (dec.).

IR (Nujol): 3270, 1775, 1660, 1610, 1530 $cm^{-1}$.

(28) 7 - [2 - Ethoxyimino - 2 - (5 - amino - 1,2,4 - thiadiazol - 3 - yl)acetamido] - 3 - (4 - dodecyloxycarbonyl - 1 - pyridiniomethyl) - 3 - cephem - 4 - carboxylate (syn isomer), mp. 152 to 157°C (dec.).

IR (Nujol): 3280, 3160, 1775, 1730, 1675, 1610, 1520 $cm^{-1}$.

(29) 7 - [2 - Ethoxyimino - 2 - (5 - amino - 1,2,4 - thiadiazol - 3 - yl)acetamido] - 3 - [4 - (N - dodecylcarbamoyl) - 1 - pyridiniomethyl] - 3 - cephem - 4 - carboxylate (syn isomer), mp. 176 to 177°C (dec.).

IR (Nujol): 3270, 1770, 1655, 1610, 1540 $cm^{-1}$.

(30) 7 - [2 - Ethoxyimino - 2 - (5 - amino - 1,2,4 - thiadiazol - 3 - yl)acetamido] - 3 - (4 - octyloxycarbonyl - 1 - pyridiniomethyl) - 3 - cephem - 4 - carboxylate (syn isomer), mp. 155 to 158°C (dec.).

IR (Nujol): 3270, 1775, 1735, 1680, 1615, 1520 $cm^{-1}$.

(31) 7 - [2 - Ethoxyimino - 2 - (5 - amino - 1,2,4 - thiadiazol - 3 - yl)acetamido] - 3 - [4 - (N - octylcarbamoyl) - 1 - pyridiniomethyl] - 3 - cephem - 4 - carboxylate (syn isomer), mp. 181 to 183°C (dec.).

IR (Nujol): 3250, 1770, 1655, 1610, 1565, 1550, 1530 $cm^{-1}$.

(32) 7 - [2 - Ethoxyimino - 2 - (5 - amino - 1,2,4 - thiadiazol - 3 - yl)acetamido] - 3 - (4 - ethoxycarbonyl - 1 - pyridiniomethyl) - 3 - cephem - 4 - carboxylate (syn isomer), mp. 150 to 155°C (dec.).

IR (Nujol): 3320, 3160, 1775, 1730, 1670, 1610, 1530, 1290 $cm^{-1}$.

Example 14

The following compounds were obtained according to similar manners to those of aforesaid Examples.

(1) 7 - [2 - Ethoxyimino - 2 - (5 - amino - 1,2,4 - thiadiazol - 3 - yl)acetamido] - 3 - (3 - amino - 1 - pyridiniomethyl) - 3 - cephem - 4 - carboxylate (syn isomer), mp. 175 to 180°C (dec.).

IR (Nujol): 3300, 3200, 1770, 1660—1590, 1510, 1150, 1035 $cm^{-1}$.

(2) 7 - [2 - Ethoxyimino - 2 - (5 - amino - 1,2,4 - thiadiazol - 3 - yl)acetamido] - 3 - (4 - hexadecyloxycarbonyl - 1 - pyridiniomethyl) - 3 - cephem - 4 - carboxylate (syn isomer), mp. 161 to 166°C (dec.).

IR (Nujol): 3400, 3250, 1770, 1730, 1670, 1610, 1520, 1285, 1120, 1035 $cm^{-1}$.

NMR ($DMSO-d_6+D_2O$, δ): 0.83 (3H, t, J=7Hz), 1.0—1.7 (29H, m), 3.2—3.8 (2H, m), 4.0—4.5 (4H, m), 4.9—5.3 (2H, m), 5.5—6.0 (2H, m), 8.4 (2H, m), 9.4 (2H, m).

Preparation 20

The following compound was obtained according to a similar manner to that of Preparation 3. Hexadecyl isonicotinate, mp. 60 to 63°C.

IR (Nujol): 1722, 1560, 1480, 1410, 1290, 1275 $cm^{-1}$.

NMR ($DMSO-d_6$, δ): 0.7—1.8 (31H, m), 4.30 (2H, t, J=5Hz), 7.7 (2H, m), 8.7 (2H, m).

## Claims

1. New cephem compounds of the formula

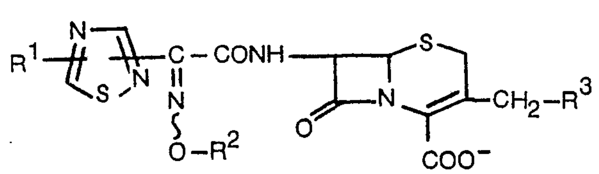

wherein $R^1$ is amino or a protected amino group,

$R^2$ is $(C_{1-6})$aliphatic hydrocarbon group which may be substituted with substituent(s) selected from the group consisting of carboxy and protected carboxy, cyclo$(C_{3-6})$alkyl or cyclo$(C_{1-6})$alkenyl, and

$R^3$ is a thiazolio group which may be substituted with substituents(s) selected from the group consisting of $(C_{1-6})$alkyl and hydroxy$(C_{1-6})$alkyl: a pyridinio group substituted with substituent(s) selected from the group consisting of halogen, cyano, hydroxy, amino protected in a usual manner by acyl, $(C_{1-6})$alkanoyl, hydroxycarbamoyl, $(C_{1-14})$alkylcarbmoyl, protected carboxy, $(C_{1-6})$alkyl, hydroxy$(C_{1-6})$alkyl, amino$(C_{1-6})$alkyl, protected amino$(C_{1-6})$alkyl, carboxy$(C_{1-6})$alkyl and hydroxyimino$(C_{1-6})$alkyl,

or a pyridinio group substituted with amino: with proviso that, when $R^2$ is $(C_{1-6})$alkyl, $R^3$ is not a pyridinio group substituted with carboxy$(C_{1-6})$alkyl and when $R^2$ is 2-carboxyprop-2-yl, $R^3$ is not 3-carboxymethylpyridinio; or pharmaceutically acceptable salts thereof.

2. Syn isomer of a compound of claim 1.

3. A compound of claims 1 or 2, wherein

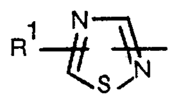 group is 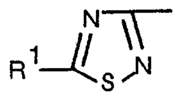 .

4. A compound of any of claims 1 to 3, wherein $R^1$ is amino; $R^2$ is $C_1$ to $C_6$ aliphatic hydrocarbon group which may be substituted with 1 to 3 substituent(s) selected from the group consisting of carboxy and protected carboxy, cyclo ($C_3$ to $C_6$)alkyl or cyclo ($C_3$ to $C_6$)alkenyl; and $R^3$ is a thiazolio group which may be substituted with 1 to 3 substituent(s) selected from the group consisting of $C_1$ to $C_6$ alkyl and hydroxy($C_1$ to $C_6$)alkyl; a pyridinio group substituted with 1 to 3 substituent(s) selected from the group consisting of halogen, cyano, hydroxy, acylamino, $C_1$ to $C_6$ alkanoyl, hydroxycarbamoyl, ($C_1$ to $C_{14}$)alkylcarbamoyl, protected carboxy, $C_1$ to $C_6$ alkyl, hydroxy ($C_1$ to $C_6$)alkyl, protected amino ($C_1$ to $C_6$)alkyl, amino $C_1$ to $C_6$)alkyl, carboxy($C_1$ to $C_6$)alkyl and hydroxyimino($C_1$ to $C_6$)alkyl or a pyridinio group.

5. A compound of any of claims 1 to 4, wherein $R^2$ is $C_1$ to $C_6$ alkyl which may be substituted with 1 substituent selected from the group consisting of carboxy and protected carboxy, cyclo ($C_1$ to $C_6$)alkyl or cyclo($C_3$ to $C_6$)alkenyl.

6. A compound of any of claims 1 to 5, wherein $R^2$ is $C_1$ to $C_6$ alkyl or cyclo($C_3$ to $C_6$)alkyl and $R^3$ is a thiazolio group which may be substituted with one $C_1$ to $C_6$ alkyl and one hydroxy($C_1$ to $C_6$)alkyl.

7. A compound of any of claims 1 to 6, wherein $R^2$ is ethyl or cyclopentyl and $R^3$ is a thiazolio group or a thazolio group substituted with one methyl and one hydroxyethyl.

8. A compound of claim 7, which is selected from the group consisting of:

7-[2-ethoxyimino-2-(5-amino-1,2,4-tiadiazol-3-yl)-acetamido]-3-(3-thiazoliomethyl)-3-cephem-4-carboxylate (syn isomer) and

7-[2-cyclopentyloxyimino-2-(5-amino-1,2,4-thiadiazol-3-yl)-acetamido]-3-[4-methyl-5-(2-hydroxyethyl)-3-thiazoliomethyl]-3-cephem-4-carboxylate (syn isomer).

9. A compound of any of claims 1 to 5, wherein $R^3$ is a pyridinio group substituted with 1 to 2 substituent(s) selected from the group consisting of halogen, cyano, hydroxy, $C_1$ to $C_6$ alkanesulfonylamino, $C_1$ to $C_6$ alkanoyl, hydroxycarbamoyl, ($C_1$ to $C_{14}$) alkylcarbamoyl, alkoxycarbonyl, $C_1$ to $C_6$ alkyl, hydroxy($C_1$ to $C_6$)alkyl, $C_1$ to $C_6$ alkoxycarbonylamino($C_1$ to $C_6$) alkyl, amino($C_1$ to $C_6$)alkyl, carboxy($C_1$ to $C_6$)alkyl and hydroxyimino($C_1$ to $C_6$)alkyl or a pyridinio group substituted with amino.

10. A compound of any of claims 1 to 5 and 9, wherein $R^2$ is ethyl, carboxymethyl, ethoxycarbonylmethyl, t-butoxycarbonylmethyl, 1-methyl-1-ethoxycarbonylethyl, cyclopentyl or 2-cyclopenten-1-yl and $R^3$ is a pyridinio group substituted with 1 to 2 substituent(s) selected from the group consisting of chloro, cyano, hydroxy, mesylamino, acetyl, hydroxycarbamoyl, butylcarbamoyl, t-butylcarbamoyl, octylcarbamoyl, dodecylcarbamoyl, ethoxycarbonyl, butoxycarbonyl, t-butoxycarbonyl octyloxycarbonyl, dodecyloxycarbonyl, hexadecyloxycarbonyl, methyl, t-butyl hydroxymethyl, 3-hydroxypropyl, t-butoxycarbonylaminomethyl, aminomethyl, carboxymethyl and hydroxyiminomethyl or a pyridinio group substituted with amino.

11. A compound of claim 10, which is selected from the group consisting of:

7-[2-ethoxyimino-2-(5-amino-1,2,4-thiadiazol-3-yl)-acetamido]-3-(3-hydroxymethyl-1-pyridiniomethyl)-3-cephem-4-carboxylate (syn isomer),

7-[2-ethoxyimino-2-(5-amino-1,2,4-thiadiazol-3-yl)-acetamido]-3-(4-hydroxymethyl-1-pyridiniomethyl)-3-cephem-4-carboxylate (syn isomer),

7-[2-ethoxyimino-2-(5-amino-1,2,4-thiadiazol-3-yl)-acetamido]-3-(4-cyano-1-pyridiniomethyl)-3-cephem-4-carboxylate (syn isomer),

7-[2-ethoxyimino-2-(5-amino-1,2,4-thiadiazol-3-yl)-acetamido]-3-(4-aminomethyl-1-pyridiniomethyl)-3-cephem-4-carboxylate (syn isomer),

7-[2-ethoxyimino-2-(5-amino-1,2,4-thiadiazol-3-yl)-acetamido]-3-(3-chloro-1-pyridiniomethyl)-3-cephem-4-carboxylate (syn isomer),

7-[2-ethoxyimino-2-(5-amino-1,2,4-thiadiazol-3-yl)-acetamido]-3-(3,5-dimethyl-1-pyridiniomethyl)-3-cephem-4-carboxylate (syn isomer),

7-[2-ethoxyimino-2-(5-amino-1,2,4-thiadiazol-3-yl)-acetamido]-3-(4-t-butyl-1-pyridiniomethyl)-3-cephem-4-carboxylate (syn isomer),

7-[2-ethoxyimino-2-(5-amino-1,2,4-thiadiazol-3-yl)-acetamido]-3-(3-methyl-1-pyridiniomethyl)-3-cephem-4-carboxylate (syn isomer),

7-[2-ethoxyimino-2-(5-amino-1,2,4-thiadiazol-3-yl)-acetamido]-3-(3-hydroxy-1-pyridiniomethyl)-3-cephem-4-carboxylate (syn isomer),

7-[2-ethoxyimino-2-(5-amino-1,2,4-thiadiazol-3-yl)-acetamido]-3-[4-(3-hydroxypropyl)-1-pyridiniomethyl)-3-cephem-4-carboxylate (syn isomer),

7-[2-ethoxyimino-2-(5-amino-1,2,4-thiadiazol-3-yl)-acetamido]-3-(4-dodecyloxycarbonyl-1-pyridiniomethyl)-3-cephem-4-carboxylate (syn isomer),

7-[2-ethoxyimino-2-(5-amino-1,2,4-thiadiazol-3-yl)-acetamido]-3-[4-(N-hydroxycarbamoyl)-1-pyridiniomethyl)-3-cephem-4-carboxylate (syn isomer),

7-[2-ethoxyimino-2-(5-amino-1,2,4-thiadiazol-3-yl)-acetamido]-3-(4-butoxycarbonyl-1-pyridiniomethyl)-3-cephem-4-carboxylate (syn isomer),

7-[2-ethoxyimino-2-(5-amino-1,2,4-thiadiazol-3-yl)-acetamido]-3-(4-t-butoxycarbonyl-1-pyridiniomethyl)-3-cephem-4-carboxylate (syn isomer),

7-[2-ethoxyimino-2-(5-amino-1,2,4-thiadiazol-3-yl)-acetamido]-3-[4-(N-butylcarbamoyl)-1-pyridiniomethyl)-3-cephem-4-carboxylate (syn isomer),

7-[2-ethoxyimino-2-(5-amino-1,2,4-thiadiazol-3-yl)-acetamido]-3-[4-(N-t-butylcarbamoyl)-1-pyridiniomethyl)-3-cephem-4-carboxylate (syn isomer),

7-[2-ethoxyimino-2-(5-amino-1,2,4-thiadiazol-3-yl)-acetamido]-3-[4-(N-dodecylcarbamoyl)-1-pyridiniomethyl)-3-cephem-4-carboxylate (syn isomer),

7-[2-ethoxyimino-2-(5-amino-1,2,4-thiadiazol-3-yl)-acetamido]-3-(3-amino-1-pyridiniomethyl)-3-cephem-4-carboxylate (syn isomer),

7-[2-ethoxyimino-2-(5-amino-1,2,4-thiadiazol-3-yl)-acetamido]-3-(4-octyloxycarbonyl-1-pyridiniomethyl)-3-cephem-4-carboxylate (syn isomer),

7-[2-ethoxyimino-2-(5-amino-1,2,4-thiadiazol-3-yl)-acetamido]-3-[4-(N-octylcarbamoyl)-1-pyridiniomethyl)-3-cephem-4-carboxylate (syn isomer),

7-[2-ethoxyimino-2-(5-amino-1,2,4-thiadiazol-3-yl)-acetamido]-3-(3-mesylamino-1-pyridiniomethyl)-3-cephem-4-carboxylate (syn isomer),

7-[2-ethoxyimino-2-(5-amino-1,2,4-thiadiazol-3-yl)-acetamido]-3-(4-ethoxycarbonyl-1-pyridiniomethyl)-3-cephem-4-carboxylate (syn isomer), and

7-[2-ethoxyimino-2-(5-amino-1,2,4-thiadiazol-3-yl)-acetamido]-3-(4-hexadecyloxycarbonyl-1-pyridiniomethyl)-3-cephem-4-carboxylate (syn isomer).

12. A compound of claim 10, which is selected from the group consisting of:

7-[2-cyclopentyloxyimino-2-(5-amino-1,2,4-thiadiazol-3-yl)-acetamido]-3-(3-hydroxymethyl-1-pyridiniomethyl)-3-cephem-4-carboxylate (syn isomer),

7-[2-cyclopentyloxyimino-2-(5-amino-1,2,4-thiadiazol-3-yl)-acetamido]-3-(4-hydroxymethyl-1-pyridiniomethyl)-3-cephem-4-carboxylate (syn isomer),

7-[2-cyclopentyloxyimino-2-(5-amino-1,2,4-thiadiazol-3-yl)-acetamido]-3-(4-acetyl-1-pyridiniomethyl)-3-cephem-4-carboxylate (syn isomer),

7-[2-cyclopentyloxyimino-2-(5-amino-1,2,4-thiadiazol-3-yl)-acetamido]-3-(3-carboxymethyl-1-pyridiniomethyl)-3-cephem-4-carboxylate (syn isomer),

7-[2-cyclopentyloxyimino-2-(5-amino-1,2,4-thiadiazol-3-yl)-acetamido]-3-(4-hydroxyiminomethyl-1-pyridiniomethyl)-3-cephem-4-carboxylate (syn isomer),

13. A compound of claim 10, which is selected from the group consisting of:

7-[2-(2-cyclopenten-1-yloxyimino)-2-(5-amino-1,2,4-thiadiazol-3-yl)-acetamido]-3-(4-hydroxymethyl-1-pyridiniomethyl)-3-cephem-4-carboxylate (syn isomer),

7-[2-(2-cyclopenten-1-yloxyimino)-2-(5-amino-1,2,4-thiadiazol-3-yl)-acetamido]-3-(3-hydroxymethyl-1-pyridiniomethyl)-3-cephem-4-carboxylate (syn isomer),

14. A compound of claim 10, which is

7-[2-carboxymethoxyimino-2-(5-amino-1,2,4-thiadiazol-3-yl)-acetamido]-3-(3-hydroxymethyl-1-pyridiniomethyl)-3-cephem-4-carboxylate (syn isomer),

15. A process for preparing new cephem compounds of the formula:

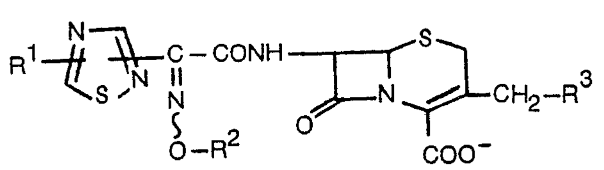

wherein $R^1$, $R^2$ and $R^3$ are each as defined in claim 1 or pharmaceutically acceptable salts thereof, which comprises reacting a compound of the formula:

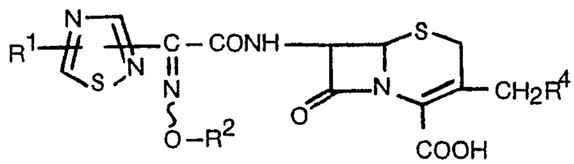

wherein $R^1$ and $R^2$ are each as defined above; and $R^4$ is a group which can be replaced by a group of the formula: $R^3$ wherein $R^3$ is as defined above; or a salt thereof with a compound of the formula: $R^{3a}$ wherein $R^{3a}$ is thiazole which may be substituted with substituent(s) selected from the group consisting of $C_1$ to $C_6$ alkyl and hydroxy ($C_1$ to $C_6$)alkyl; pyridine substituted with substituent(s) selected from the group consisting of halogen, cyano, hydroxy, amino protected in a usual manner by acyl, $C_1$ to $C_6$alkanoyl, hydroxycarbamoyl, ($C_1$ to $C_{14}$)alkylcarbamoyl, protected carboxy, $C_1$ to $C_6$alkyl, hydroxy($C_1$ to $C_6$)alkyl, protected amino ($C_1$ to $C_6$)alkyl, amino($C_1$ to $C_6$)alkyl, carboxy($C_1$ to $C_6$)alkyl and hydroxyimino($C_1$ to $C_6$)alkyl or pyridine substituted with amino; with proviso that, when $R^2$ is ($C_1$ to $C_6$)alkyl, $R^{3a}$ is not pyridine substituted with carboxy($C_1$ to $C_6$)alkyl and when $R^2$ is 2-carboxyprop-2-yl, $R^{3a}$ is not 3-carboxymethylpyridine.

16. A process for preparing new cephem compounds of the formula:

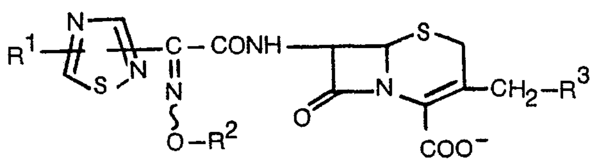

wherein $R^1$, $R^2$ and $R^3$ are each as defined in claim 1 or pharmaceutically acceptable salts thereof which comprises reacting a compound of the formula:

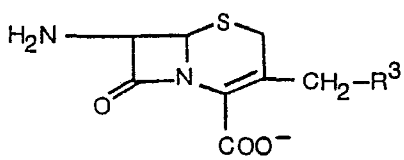

wherein $R^3$ is as defined above; or its reactive derivative at the amino group or a salt thereof with a compound of the formula:

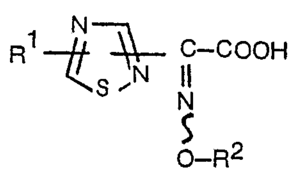

wherein $R^1$ and $R^2$ are each as defined above; or its reactive derivative at the carboxy group or a salt thereof.

17. A process for preparing new cephem compounds of the formula:

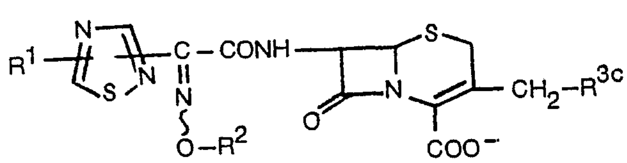

wherein $R^1$ and $R^2$ are each as defined in claim 1 and $R^{3c}$ is a pyridinio group substituted with amino($C_1$ to $C_6$)-alkyl; or pharmaceutically acceptable salts thereof, which comprises subjecting a compound of the formula:

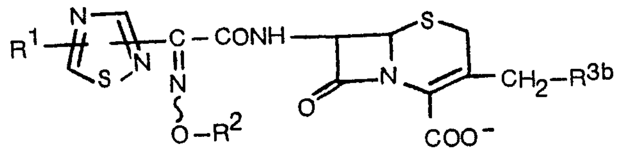

wherein $R^1$ and $R^2$ are each as defined above and $R^{3b}$ is a pyridinio group substituted with protected amino($C_1$ to $C_6$)alkyl, or a salt thereof, to elimination reaction of the amino-protective group.

18. A process for preparing new cephem compounds of the formula:

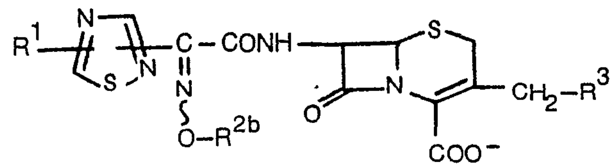

wherein $R^1$ and $R^3$ are each as defined in claim 1 and $R^{2b}$ is carboxy ($C_1$ to $C_6$)alkyl, or pharmaceutically acceptable salts thereof, which comprises subjecting a compound of the formula:

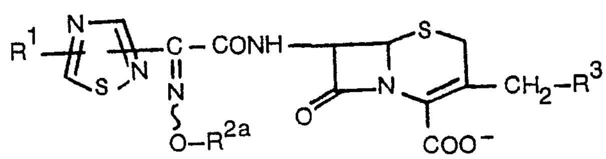

wherein $R^1$ and $R^3$ are each as defined above and $R^{2a}$ is protected carboxy($C_1$ to $C_6$)alkyl, or a salt thereof, to elimination reaction of the carboxy-protective group.

19. A process for preparing new cephem compounds of the formula:

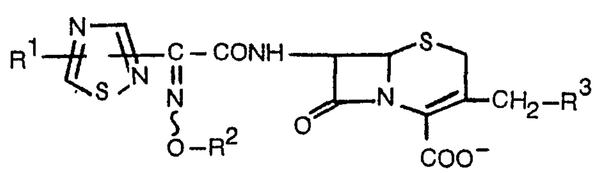

wherein $R^1$, $R^2$ and $R^3$ are each as defined in claim 1, or pharmaceutically acceptable salts thereof, which comprises subjecting a compound of the formula:

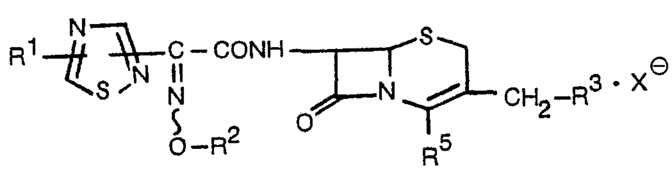

wherein $R^1$, $R^2$ and $R^3$ are each as defined above, $R^5$ is a protected carboxy group and X is an acid residue, to elimination reaction of the carboxy-protective group.

20. A pharmaceutically antibacterial composition comprising a compound of claim 1 in association with a pharmaceutically acceptable, substantially non-toxic carrier or excipient.

21. A method for producing a pharmaceutical antibacterial composition which comrpises mixing a compound of claim 1 as an active ingredient with an inert carrier.

22. A compound of the formula:

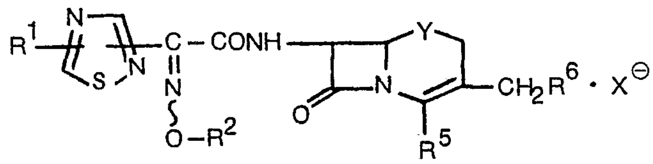

wherein $R^1$ and $R^2$ are each as defined in claim 1;

$R^5$ is a protected carboxy group;

$R^6$ is a thiazolio group which may be substituted with substituent(s) selected from the group consisting of $C_1$ to $C_6$alkyl and hydroxy ($C_1$ to $C_6$)alkyl; pyridinio group substituted with substituent(s) selected from the group consisting of halogen, cyano, hydroxy, amino protected in a usual manner by acyl, $C_1$ to $C_6$alkanoyl, hydroxycarbamoyl, ($C_1$ to $C_{14}$)alkylcarbamoyl, protected carboxy, $C_1$ to $C_6$alkyl, hydroxy($C_1$ to $C_6$)alkyl, protected amino($C_1$ to $C_6$)alkyl, amino($C_1$ to $C_6$)alkyl, carboxy($C_1$ to $C_6$)alkyl and hydroxyimino($C_1$ to $C_6$)alkyl or a pyridinio group substituted with amino; with proviso that, when $R^2$ is ($C_1$ to $C_6$)alkyl, $R^6$ is not a pyridinio group substituted with carboxy ($C_1$ to $C_6$)alkyl and when $R^3$ is 2-carboxyprop-2-yl, $R^6$ is not 3-carboxymethylpyridinio; X is an acid residue; and

$$Y \text{ is } —S— \text{ or } —\overset{\overset{O}{\uparrow}}{S}—\ ;$$

and salts thereof.

23. A process for preparing a compound of the formula:

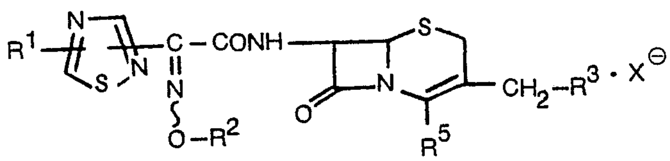

wherein $R^1$, $R^2$ and $R^3$ are each as defined in claim 1;

$R^5$ is a protected carboxy group; and

X is an acid residue; or salts thereof; which comprises reducing a compound of the formula:

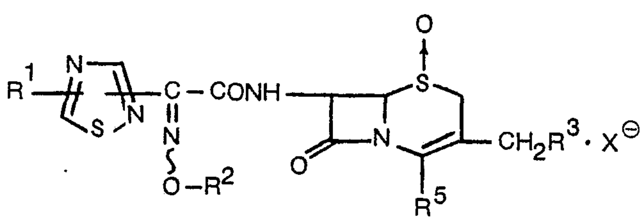

wherein $R^1$, $R^2$, $R^3$, $R^5$ and X are each as defined above, or a salt thereof.

24. A compound of the formula of claim 1 for use as a medicament.

25. A compound of the formula of claim 1 for use in treatment of infectious diseases.

**Patentansprüche**

1. Neue Cephem-Verbindungen der Formel:

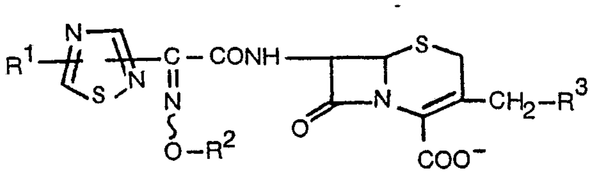

worin bedeuten: $R^1$ eine Amino oder geschützte Aminogruppe,

$R^2$ eine $(C_{1-6})$-aliphatische Kohlenwasserstoffgruppe, die durch einen oder mehr Substituenten substituiert sein kann, die ausgewählt werden aus der Gruppe, die besteht aus Carboxy und geschütztem Carboxy, Cyclo$(C_3$-$C_6)$alkyl oder Cyclo$(C_3$-$C_6)$alkenyl und

$R^3$ eine Thiazoliogruppe, die durch einen oder mehr Substituenten substituiert sein kann, die ausgewählt werden aus der Gruppe, die besteht aus $(C_{1-6})$Alkyl und Hydroxy$(C_{1-6})$alkyl; eine Pyridiniogruppe,'die substituiert ist durch einen oder mehr Substituenten, die ausgewählt werden aus der Gruppe, die besteht aus Halogen, cyano, Hydroxy, Amino, das geschützt ist auf übliche Weise durch Acyl, $(C_{1-6})$Alkanoyl, Hydroxycarbamoyl, $(C_{1-14})$Alkylcarbamoyl), geschütztes Carboxy, $(C_{1-6})$-Alkyl, Hydroxy$(C_{1-6})$alkyl, Amino$(C_{1-6})$alkyl, geschütztes Amino$(C_{1-6})$alkyl, Carboxy$(C_{1-6})$alkyl und Hydroxyimino$(C_{1-6})$alkyl oder eine Pyridiniogruppe, die durch Amino substituiert ist, mit der Maßgabe, daß dann, wenn $R^2$ für $(C_{1-6})$Alkyl steht, $R^3$ nicht eine durch Carboxy$(C_{1-6})$alkyl substituierte Pyridiniogruppe bedeutet, und dann, wenn $R^2$ für 2-Carboxyprop-2-yl steht, $R^3$ nicht 3-Carboxymethylpyridinio bedeutet; oder die pharmazeutisch akzeptablen Salze davon.

2. Syn-Isomeres einer Verbindung nach Anspruch 1.

3.Verbindung nach Anspruch 1 oder 2, worin die

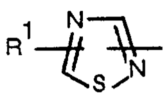 -Gruppe bedeutet 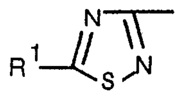 .

4. Verbindung nach einem der Ansprüche 1 bis 3, worin bedeuten:

$R^1$ Amino

$R^2$ eine $C_1$—$C_6$-aliphatische Kohlenwasserstoffgruppe, die substituiert sein kann durch 1 bis 3 Substituenten, ausgewählt aus der Gruppe, die besteht aus Carboxy und geschütztem Carboxy, Cyclo$(C_3$—$C_6)$alkyl oder Cyclo$(C_3$—$C_6)$alkenyl; und

$R^3$ eine Thiazoliogruppe, die substituiert sein kann durch 1 bis 3 Substituenten, ausgewählt aus der Gruppe, die besteht aus $C_1$—$C_6$-Alkyl und Hydroxy$(C_1$—$C_6)$alkyl; eine Pyridiniogruppe, die substituiert ist durch 1 bis 3 Substituenten, ausgewählt aus der Gruppe, die besteht aus Halogen, Cyano, Hydroxy, Acylamino, $C_1$—$C_6$-Alkanoyl, Hydroxycarbamoyl, $(C_1$—$C_{14})$Alkylcarbamoyl, geschütztes Carboxy, $C_1$—$C_6)$-Alkyl, Hydroxy$(C_1$—$C_6)$alkyl, geschütztes Amino$(C_1$—C.512)-alkyl, Amino$(C_1$—$C_6)$-alkyl, Carboxy$(C_1$—$C_6)$-alkyl und Hydroxyimino$(C_1$—$C_6)$alkyl oder eine durch Amino substituierte Pyridiniogruppe

5. Verbindung nach einem der—rüche 1 bis 4, worin $R^2$ bedeutet $C_1$—$C_6$-Alkyl, das substituiert sein kann durch einen Substituenten, ausgewählt aus der Gruppe, die besteht aus Carboxy und geschütztem Carboxy, Cyclo$(C_3$—$C_6)$alkyl oder Cyclo$(C_3$—$C_6)$alkenyl.

6. Verbindung nach einem der Ansprüche 1 bis 5,—n bedeuten:

$R^2$ $C_1$—$C_6)$alkyl oder Cyclo$(C_3$—$C_6)$alkyl und

$R^3$ eine Thiazoliogruppe, die substituiert sein kann durch ein $C_1$—$C_6$-Alkyl und ein Hydroxy$(C_1$—$C_6)$alkyl.

7. Verbindung nach einem der Ansprüche 1 bis 6, worin bedeuten:

$R^2$ Ethyl oder Cyclopentyl und

$R^3$ eine Thiazoliogruppe oder eine durch ein Methyl und ein Hydroxyethyl substituierte Thiazoliogruppe.

8. Verbindung nach Anspruch 7, die ausgewählt wird aus der Gruppe, die besteht aus:

7-[2-Ethoxyimino-2-(5-amino-1,2,4-thiadiazol-3-yl)-acetamido]-3-(3-thiazoliomethyl)-3-cephem-4-carboxylat (syn-Isomeres) und

7-[2-Cyclopentyloxyimino-2-(5-amino-1,2,4-thiadiozol-3-yl)acetamido]-3-[4-methyl-5-(2-hydroxyethyl)-3-thiazoliomethyl]-3-cephem-4-carboxylat (syn-Isomeres).

9. Verbindung nach einem der Ansprüche 1 bis 5, worin $R^3$ bedeutet eine Pyridiniogruppe, die substituiert ist durch 1 bis 2 Substituenten, ausgewählt aus der Gruppe, die besteht aus Halogen, Cyano, Hydroxy, $C_1$—$C_6$-Alkansulfonylamino, $C_1$—$C_6$-Alkanoyl, Hydroxycarbamoyl, $(C_1$—$C_{14})$-Alkylcarbamoyl, Alkoxycarbonyl, $C_1$—$C_6$-Alkyl, Hydroxy$(C_1$—$C_6)$alkyl, $(C_1$—$C_6)$-Alkoxycarbonylamino$(C_1$—$C_6)$alkyl, Amino$(C_1$—$C_6$alkyl, Carboxy$(C_1$—$C_6$alkyl und Hydroxyimino$(C_1$—$C_6)$alkyl oder eine durch Amino substituierte Pyridiniogruppe.

10. Verbindung nach einem der Ansprüche 1 bis 5 und 9, worin bedeuten:

$R^2$ Ethyl, Carboxymethyl, Ethoxycarbonylmethyl, t-Butoxycarbonylmethyl, 1-Methyl-1-ethoxycarbonylethyl, Cyclopentyl oder 2-Cyclopenten-1-yl und

$R^3$ eine Pyridiniogruppe, die substituiert ist durch 1 bis 2 Substituenten, ausgewählt aus der Gruppe, die besteht aus Chlor, Cyano, Hydroxy, Mesylamino, Acetyl, Hydroxycarbamoyl, Buitylcarbamoyl, t-Butylcarbamoyl, Octylcarbamoyl, Dodecylcarbamoyl, Ethoxycarbonyl, Butoxycarbonyl, t-Butoxycarbonyl, Octyloxycarbonyl, Dodecyloxycarbonyl, Hexadecyloxycarbonyl, Methyl, t-Butyl Hydroxymethyl, 3-Hydroxypropyl, T-Butoxycarbonylaminoethyl, Aminoethyl, Carboxymethyl und Hydroxyiminomethyl oder eine durch Amino substituiert Pyridiniogruppe.

11. Verbindung nach Anspruch 10, die ausgewählt wird aus der Gruppe, die besteht aus:

7-[2-Ethoxyimino-2-(5-amino-1,2,4-thiadiazol-3-yl)-acetamido]-3-(3-hydroxymethyl-1-pyridiniomethyl)-3-cephem-4-carboxylat (syn-Isomeres),

7-[2-Ethoxyimino-2-(5-amino-1,2,4-thiadiazol-3-yl)-acetamido]-3-(4-hydroxymethyl-1-pyridiniomethyl)-3-cephem-4-carboxylat (syn-Isomeres),

7-[2-Ethoxyimino-2-(5-amino-1,2,4-thiadiazol-3-yl)-acetamido]-3-(4-cyano-1-pyridiniomethyl)-3-cephem-4-carboxylat (syn-Isomeres),

7-[2-Ethoxyimino-2-(5-amino-1,2,4-thiadiazol-3-yl)-acetamido]-3-(4-aminomethyl-1-pyridiniomethyl)-3-cephem-4-carboxylatdiformiat (syn-Isomeres),

7-[2-Ethoxyimino-2-(5-amino-1,2,4-thiadiazol-3-yl)-acetamido]-3-(3-chloro-1-pyridiniomethyl)-3-cephem-4-carboxylat (syn-Isomeres),

7-[2-Ethoxyimino-2-(5-amino-1,2,4-thiadiazol-3-yl)-acetamido]-3-(3,5-dimethyl-1-pyridiniomethyl)-3-cephem-4-carboxylat (syn-Isomeres),

7-[2-Ethoxyimino-2-(5-amino-1,2,4-thiadiazol-3-yl)-acetamido]-3-(4-t-butyl-1-pyridiniomethyl)-3-cephem-4-carboxylat (syn-Isomeres),

7-[2-Ethoxyimino-2-(5-amino-1,2,4-thiadiazol-3-yl)-acetamido]-3-(3-methyl-1-pyridiniomethyl)-3-cephem-4-carboxylat (syn-Isomeres),

7-[2-Ethoxyimino-2-(5-amino-1,2,4-thiadiazol-3-yl)-acetamido]-3-(3-hydroxy-1-pyridiniomethyl)-3-cephem-4-carboxylat (syn-Isomeres),

7-[2-Ethoxyimino-2-(5-amino-1,2,4-thiadiazol-3-yl)-acetamido]-3-[4-(3-hydroxypropyl)-1-pyridiniomethyl)-3-cephem-4-carboxylat (syn-Isomeres),

7-[2-Ethoxyimino-2-(5-amino-1,2,4-thiadiazol-3-yl)-acetamido]-3-(4-dodecyloxycarbonyl-1-pyridiniomethyl)-3-cephem-4-carboxylat (syn-Isomeres),

7-[2-Ethoxyimino-2-(5-amino-1,2,4-thiadiazol-3-yl)-acetamido]-3-[4-(N-hydroxycarbamoyl)-1-pyridiniomethyl)-3-cephem-4-carboxylat (syn-Isomeres),

7-[2-Ethoxyimino-2-(5-amino-1,2,4-thiadiazol-3-yl)-acetamido]-3-(4-butoxycarbonyl-1-pyridiniomethyl)-3-cephem-4-carboxylat (syn-Isomeres),

7-[2-Ethoxyimino-2-(5-amino-1,2,4-thiadiazol-3-yl)-acetamido]-3-(4-t-butoxycarbonyl-1-pyridiniomethyl)-3-cephem-4-carboxylat (syn-Isomeres),

7-[2-Ethoxyimino-2-(5-amino-1,2,4-thiadiazol-3-yl)-acetamido]-3-[4-(N-butylcarbamoyl)-1-pyridiniomethyl)-3-cephem-4-carboxylat (syn-Isomeres),

7-[2-Ethoxyimino-2-(5-amino-1,2,4-thiadiazol-3-yl)-acetamido]-3-[4-(N-t-butylcarbamoyl)-1-pyridiniomethyl)-3-cephem-4-carboxylat (syn-Isomeres),

7-[2-Ethoxyimino-2-(5-amino-1,2,4-thiadiazol-3-yl)-acetamido]-3-[4-(N-dodecylcarbamoyl)-1-pyridiniomethyl)-3-cephem-4-carboxylat (syn-Isomeres),

7-[2-Ethoxyimino-2-(5-amino-1,2,4-thiadiazol-3-yl)-acetamido]-3-(3-amino-1-pyridiniomethyl)-3-cephem-4-carboxylat (syn-Isomeres),

7-[2-Ethoxyimino-2-(5-amino-1,2,4-thiadiazol-3-yl)-acetamido]-3-(4-octyloxycarbonyl-1-pyridiniomethyl)-3-cephem-4-carboxylat (syn-Isomeres),

7-[2-Ethoxyimino-2-(5-amino-1,2,4-thiadiazol-3-yl)-acetamido]-3-[4-(N-octylcarbamoyl)-1-pyridiniomethyl)-3-cephem-4-carboxylat (syn-Isomeres),

7-[2-Ethoxyimino-2-(5-amino-1,2,4-thiadiazol-3-yl)-acetamido]-3-(3-mesylamino-1-pyridiniomethyl)-3-cephem-4-carboxylat (syn-Isomeres),

7-[2-Ethoxyimino-2-(5-amino-1,2,4-thiadiazol-3-yl)-acetamido]-3-(4-ethoxycarbonyl-1-pyridiniomethyl)-3-cephem-4-carboxylat (syn-Isomeres), und

7-[2-Ethoxyimino-2-(5-amino-1,2,4-thiadiazol-3-yl)-acetamido]-3-(4-hexadecyloxycarbonyl-1-pyridiniomethyl)-3-cephem-4-carboxylat (syn-Isomeres),

12. Verbindung nach Anspruch 10, die ausgewählt wird aus der Gruppe, die besteht aus:

7-[2-Cyclopentyloxyimino-2-(5-amino-1,2,4-thiadiazol-3-yl)-acetamido]-3-(3-hydroxymethyl-1-pyridiniomethyl)-3-cephem-4-carboxylat (syn-Isomeres),

7-[2-Cyclopentyloxyimino-2-(5-amino-1,2,4-thiadiazol-3-yl)-acetamido]-3-(4-hydroxymethyl-1-pyridiniomethyl)-3-cephem-4-carboxylat (syn-Isomeres),

7-[2-Cyclopentyloxyimino-2-(5-amino-1,2,4-thiadiazol-3-yl)-acetamido]-3-(4-acetyl-1-pyridiniomethyl)-3-cephem-4-carboxylat (syn-Isomeres),

7-[2-Cyclopentyloxyimino-2-(5-amino-1,2,4-thiadiazol-3-yl)-acetamido]-3-(3-carboxymethyl-1-pyridiniomethyl)-3-cephem-4-carboxylat (syn-Isomeres),

7-[2-Cyclopentyloxyimino-2-(5-amino-1,2,4-thiadiazol-3-yl)-acetamido]-3-(4-hydroxyiminomethyl-1-pyridiniomethyl)-3-cephem-4-carboxylat (syn-Isomeres).

13. Verbindung nach Anspruch 10, die ausgewählt wird aus der Gruppe, die besteht aus:

7-[2-(2-Cyclopenten-1-yloxyimino)-2-(5-amino-1,2,4-thiadiazol-3-yl)-acetamido]-3-(4-hydroxymethyl-1-pyridiniomethyl)-3-cephem-4-carboxylat (syn-Isomeres),

7-[2-(2-Cyclopenten-1-yloxyimino)-2-(5-amino-1,2,4-thiadiazol-3-yl)-acetamido]-3-(3-hydroxymethyl-1-pyridiniomethyl)-3-cephem-4-carboxylat (syn-Isomeres).

14. Verbindung nach Anspurch 10, bei der es sich handelt um

7-[2-Carboxymethoxyimino)-2-(5-amino-1,2,4-thiadiazol-3-yl)-acetamido]-3-(3-hydroxymethyl-1-pyridiniomethyl)-3-cephem-4-carboxylat (syn-Isomeres).

15. Verfahren zur Herstellung neuer Cephem-Verbindungen der Formel:

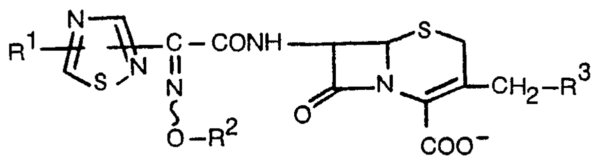

worin $R^1$, $R^2$ und $R^3$ jeweils wie in Anspruch 1 definiert sind, oder der pharmazeutisch akzeptablen Salze davon, das umfaßt die Umsetzung einer Verbindung der Formel:

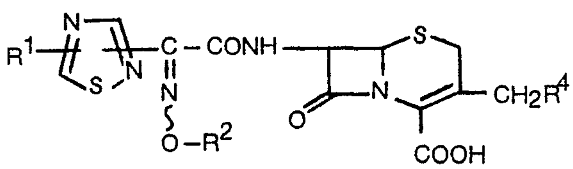

worin $R^1$ und $R^2$ jeweils wie oben definiert sind und $R^4$ eine Gruppe darstellt, die durch eine Gruppe der Formel $R^3$ ersetzt sein kann, worin $R^3$ wie oben definiert ist, oder eines Salzes davon mit einer Verbindung der Formel: $R^{3a}$ worin $R^{3a}$ bedeutet Thiazol, das substituiert sein kann durch einen oder mehr Substituenten, ausgewählt aus der Gruppe, die besteht aus ($C_1$—$C_6$)-Alkyl und Hydroxy($C_1$—$C_6$)-alkyl; Pyridin, das substituiert ist durch einen oder mehr Substituenten, ausgewählt aus der Gruppe, die besteht aus Halogen, Cyano, Hydroxy, Amino, das auf übliche Weise geschützt ist durch Acyl, ($C_1$—$C_6$)-Alkanoyl, Hydroxycarbamoyl, ($C_1$—$C_{14}$)Alkylcarbamoyl, geschütztes Carboxy, ($C_1$—$C_6$)-Alkyl, Hydroxy($C_1$—$C_6$)alkyl, geschütztes Amino-($C_1$—$C_6$)alkyl, Amino($C_1$—$C_6$)alkyl, Carboxy($C_1$—$C_6$)alkyl und Hydroxyimino($C_1$—$C_6$)alkyl oder durch Amino substituiertes Pyridin; mit der Maßgabe, daß dann, wenn $R^2$ für ($C_1$—$C_6$)-Alkyl steht, $R^{3a}$ nicht durch Carboxy($C_1$—$C_6$)alkyl substituiertes Pyridin bedeutet, und dann, wenn $R^2$ für 2-Carboxyprop-2-yl steht, $R^{3a}$ nicht 3-Carboxymethylpyridin bedeutet.

16. Verfahren zur Herstellung neuer Cephem-Verbindungen der Formel:

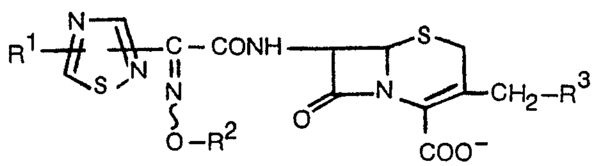

worin $R^1$, $R^2$ und $R^3$ jeweils wie in Anspruch 1 definiert sind, oder pharmazeutisch akzeptabler Salze davon, das umfaßt die Umsetzung einer Verbindung der Formel:

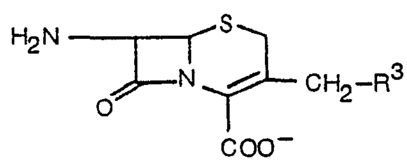

worin $R^3$ wie oben definiert ist, oder ihres reaktionsfähigen Derivats an der Aminogruppe oder eines Salzes davon mit einer Verbindung der Formel:

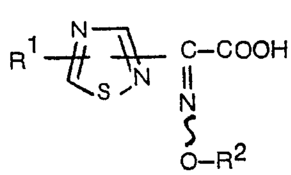

worin $R^1$ und $R^2$ jeweils wie oben definiert sind, oder ihrem reaktionsfähigen Derivat an der Carboxygruppe oder einem Saz davon.

17. Verfahren zur Herstellung neuer Cepham-Verbindungen der formel

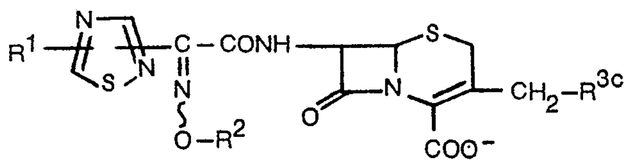

worin $R^1$ und $R^2$ jeweils wie in Anspruch 1 definiert sind und $R^{3c}$ eine durch Amino($C_1$—$C_6$)alkyl substituierte Pyridiniogruppe bedeutet, oder der pharmazeutisch akzeptablen Salze Davon, ds umfaßt die Umsetzung einer Verbindung der Formel:

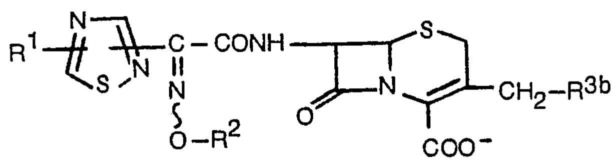

worin $R^1$ und $R^2$ jeweils wie oben definiert sind und $R^{3b}$ eine Pyridiniogruppe bedeutet, die durch geschütztes Amino($C_1$—$C_6$)alkyl substituiert ist, oder eines Salzes davon zur Eliminierung der Aminoschutzgruppe.

18. Verfahren zur Herstellung neuer Cephem-Verbindungen der Formel:

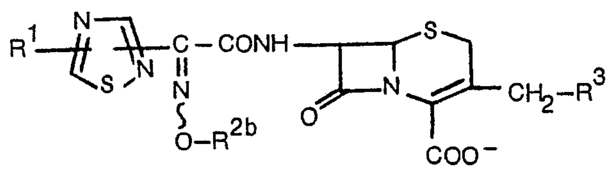

worin $R^1$ und $R^3$ jeweils wie in Anspruch 1 definiert sind und $R^{2b}$ Carboxy($C_1$—$C_6$)alkyl bedeutet, oder der pharmazeutisch akzeptablen Salze davon, das umfaßt die Eliminierung der Carboxyschutzgruppe aus einer Verbindung der Formel:

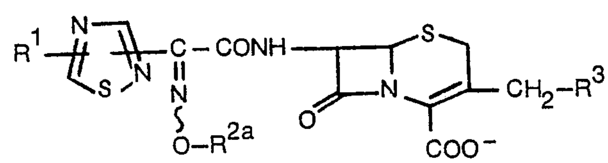

worin $R^1$ und $R^3$ jeweils wie oben definiert sind und $R^{2a}$ geschütztes Carboxy($C_1$—$C_6$)alkyl bedeutet, oder einem Salz davon.

19. Verfahren zur Herstellung neuer Cephem-Verbindungen der Formel:

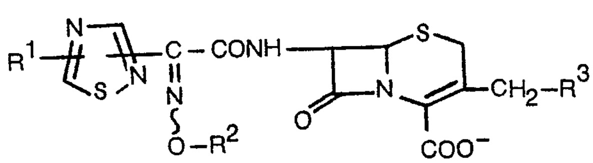

worin $R^1$, $R^2$ und $R^3$ wie in Anspruch 1 definiert sind, oder der pharmazeutisch akseptablen Salze davon, das umfaßt die Eliminierung der Carboxyschutzgruppe aus einer Verbindung der Formel:

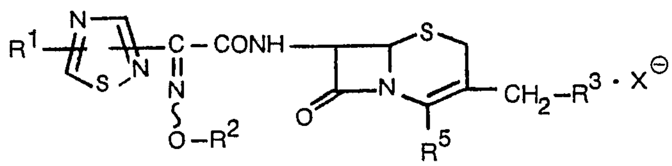

worin $R^1$, $R^2$ und $R^3$ jeweils wie oben definiert sind, $R^5$ eine geschützte Carboxygruppe und X einen Säurerest bedeuten.

20. Pharmazeutische antibakterielle Zusammensetzung, die enthält eine Verbindung nach Anspruch 1 in Assoziation mit einem pharmazeutisch akseptablen, im wesentlichen nicht-toxischen Träger oder Hilfsstoff.

21. Verfahren zur Herstellung einer pharmazeutischen antibakteriellen Zusammensetzung, das umfaßt das Mischen einer Verbindung nach Anspruch 1 als aktiven Bestandteil (Wirkstoff) mit einem inerten Träger.

22. Verbindung der Formel:

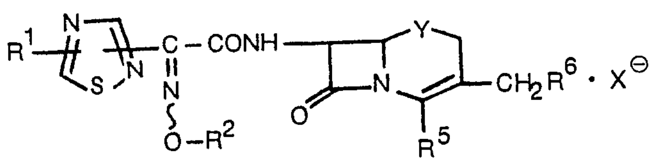

worin $R^1$ und $R^2$ jeweils wie in Anspruch 1 definiert sind;

$R^5$ eine geschützte Carboxygruppe bedeutet;

$R^6$ bedeutet eine Thiazoliogruppe, die substituiert sein kann durch einen oder mehr Substituienten, Ausgewählt aus der Gruppe, die besteht aus ($C_1$—$C_6$)Alkyl und Hydroxy($C_1$—$C_6$)alkyl; eine Pyridiniogruppe, die substituiert ist durch einen oder mehr Substituienten, ausgewählt aus der Gruppe, die besteht aus Halogen, Cyano, Hydroxy, Amino, das auf übliche Weise geschützt ist durch acyl, ($C_1$—$C_6$)-Alkanoyl, Hydroxycarbamoyl, ($C_1$—$C_{14}$)-Alkylcarbamoyl, geschütztes Carboxy, ($C_1$—$C_6$)alkyl, Hydroxy($C_1$—$C_6$)alkyl, geschütztes Amino($C_1$—$C_6$)alkyl, Amino($C_1$—$C_6$)alkyl, Carboxy($C_1$—$C_6$)alkyl und Hydroxyimino($C_1$—$C_6$)alkyl oder eine Pyridiniogruppe, die durch Amino substituiert ist; mit der Maßgabe, daß dann, wenn $R^2$ für ($C_1$—$C_6$)-Alkyl steht, $R^6$ nicht eine durch Carboxy($C_1$—$C_6$)alkyl substituierte Pyridiniogruppe bedeutet, und dann, wenn $R^3$ für 2-Carboxyprop-2-yl steht, $R^6$ nicht 3-Carboxymethylpyridinio bedeutet; X einen Säurerest darstellt, und

$$\text{Y für —S— oder —}\overset{\overset{O}{\uparrow}}{S}\text{— steht,}$$

sowie Salze davon.

23. Verfahren zur Herstellung einer Verbindung der Formel:

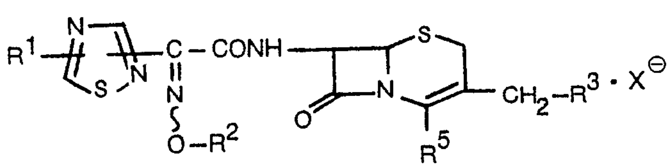

worin $R^1$, $R^2$ und $R^3$ jeweils wie in Anspruch 1 definiert sind,

$R^5$ eine geschützte Carboxygruppe bedeutet, und

X einen Säurerest darstellt, oder von Salzen davon, das umfaßt die Reduktion einer Verbindung der Formel:

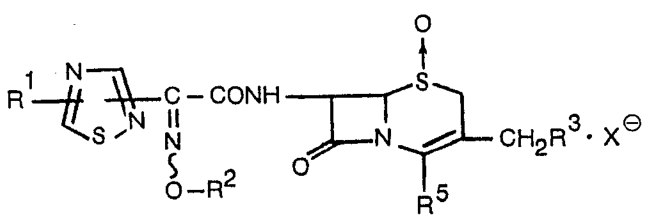

worin $R^1$, $R^2$, $R^3$, $R^5$, X jeweils wie oben definiert sind, oder eines Salzes davon.

24. Verbindung der Formel nach Anspruch 1 für die Verwendung als Arzneimittel.

25. Verbindung der Formel nach Anspruch 1 für die Verwendung zur Behandlung von Infektionserkrankungen.

**Revendications**

1. Nouveaux déphèmes répondant à la formule:

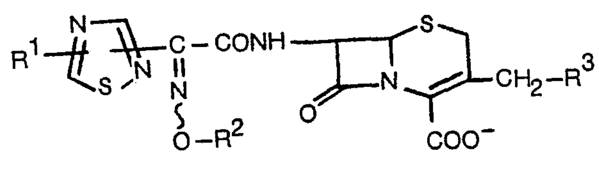

dans laquelle $R^1$ est un groupe amino ou un groupe amino protégé,

$R^2$ est un groupe hydrocarboné aliphatique en $C_1$ à $C_6$ qui peut être substitué avec un ou plusieurs substituants choisis parmi les groupes carboxy et carboxy protégé, cycloalkyle en $C_3$ à $C_6$, cycloalcényle en $C_3$ à $C_6$, et

$R^3$ est un groupe thiazolo qui peut être substitué avec un ou plusieurs substituants choisis parmi les groupes alkyle en $C_1$ à $C_6$ et hydroxyalkyle en $C_1$ à $C_6$: un groupe pyridino substitué par un ou plusieurs substituants choisis parmi les halogènes, les groupes cyano, hydroxy, amino protégé de la manière habituelle par un groupe acyle, alcanoyle en $C_1$ à $C_6$, hydroxycarbamoyle, (alkyle en $C_1$ à $C_{14}$)carbamoyle), carboxy protégé, alkyle en $C_1$ à $C_6$, hydroxy(alkyle en $C_1$ à $C_6$), amino(alkyle en $C_1$ à $C_6$), (amino protégé) (alkyle en $C_1$ à $C_6$), carboxy(alkyle en $C_1$ à $C_6$) et hydroxyimino(alkyle en $C_1$ à $C_6$), ou un groupe pyridino substitué par un groupe amino, sous réserve que lorsque $R^2$ est un groupe alkyle en $C_1$ à $C_6$, $R^3$ n'est pas un groupe pyridino substitué par un groupe carboxy(alkyle en $C_1$ à $C_6$) et que lorsque $R^2$ est un groupe 2-carboxyprop-2-yle, $R^3$ n'est pas un groupe 3-carboxyméthylpyridino; ou ses sels pharmaceutiquement acceptables.

2. Isomère syn d'un composé selon la revendication 1.

3. Composé selon les revendications 1 ou 2, dans lequel

le groupe 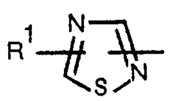 est 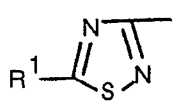 .

4. Composé selon l'une quelconque des revendications 1 à 3 dans lequel;

$R^1$ est un groupe amino;

$R^2$ est un groupe hydrocarboné aliphatique en $C_1$ à $C_6$ qui peut être substitué par 1 à 3 substituants choisis parmi les groupes carboxy ou carboxy protégé, cycloalkyle en $C_3$ à $C_6$ ou cycloalcényle en $C_3$ à $C_6$; et

$R^3$ est un groupe thiazolo qui peut être substituè par 1 à 3 substituants choisis parmi les groupes alkyle en $C_1$ à $C_6$ et hydroxyalkyle en $C_1$ à $C_6$, un groupe pyridino substitué par 1 à 3 substituants choisis parmi les halogènes, les groupes cyano, hydroxy, acylamino, alcanoyle en $C_1$ à $C_6$, hydroxycarbamoyle, (alkyle en $C_1$ à $C_{14}$)carbamoyle, carboxy protégé, alkyle en $C_1$ à $C_6$, hydroxyalkyle en $C_1$ à $C_6$, (amino protégé) (alkyle en $C_1$ à $C_6$) aminoalkyle en $C_1$ à $C_6$, carboxy(alkyle en $C_1$ à $C_6$) et hydroxyimino(alkyle en $C_1$ à $C_6$) ou un groupe pyridino.

5. Composé selon l'une quelconque des revendications 1 à 4, dans lequel: $R^2$ est un groupe alkyle en $C_1$ à $C_6$ qui peut être substitué par un substituants choisi parmi les groupes carboxy et carboxy protégé, cycloalkyle en $C_3$ à $C_6$ ou cycloalcényle en $C_3$ à $C_6$.

6. Composé selon l'une quelconque des revendications 1 à 5, dans lequel: $R^2$ est un groupe alkyle en $C_1$ à $C_6$ ou cycloalkyle en $C_3$ à $C_6$; et $R^3$ est un groupe thiazolo qui peut être substitué par un groupe alkyle en $C_1$ à $C_6$ et un groupe hydroxy(alkyle en $C_1$ à $C_6$.

7. Composé selon l'une quelconque des revendications 1 à 6, dans lequel: $R^2$ est un groupe éthyle ou cyclopentyle et $R^3$ est un groupe thiazolo ou un groupe thiazolo substitué par un groupe méthyle et un groupe hydroxyéthyle.

8. Composé selon la revendication 7, choisi parmi:

le 7-[2-éthoxyimino-2-(5-amino-1,2,4-thiadiazol-3-yl)-acétamido]-3-(3-thiazoliométhyl)-3-céphème-4-carboxylate (isomère syn) et
le 7-[2-cyclopentyloxyimino-2-(5-amino-1,2,4-thiadiazol-3-yl)-acétamido]-3-[4-méthyl-5-(2-hydroxyéthyl)-3-thiazoliométhyl]-3-céphème-4-carboxylate (isomère syn).

9. Composé selon l'une quelconque des revendications 1 à 5, dans lequel: $R^3$ est un groupe pyridino substitué avec 1 ou 2 substituants choisis parmi les halogènes, les groupes cyano, hydroxy(alcane en $C_1$ à $C_6$)sulfonylamino, alcanoyle en $C_1$ à $C_6$, hydroxycarbamoyl (alkyle en $C_1$ à $C_{14}$)carbamoyle, alcoxy-carbonyle, alkyle en $C_1$ à $C_6$, hydroxy(alkyle en $C_1$ à $C_6$), (alcoxy en $C_1$ à $C_6$) carbonylamino (alkyle en $C_1$ à $C_6$), amino(alkyle en $C_1$ à $C_6$), carboxy(alkyle en $C_1$ à $C_6$) et hydroxyimino(alkyle en $C_1$ à $C_6$) ou un groupe pyridino substitué par un groupe amino.

10. Composé selon l'une quelconque des revendications 1 à 5 et 9, dans lequel: $R^2$ est un groupe éthyle, carboxyméthyle, éthoxycarbonylméthyle, t-butoxycarbonylméthyle, 1-méthyl-1-éthoxycarbonyl-éthyle, cyclopentyle ou 2-cyclopentèn-1-yle et $R^3$ est un groupe pyridino substitué par 1 à 2 substituants choisis parmi les groupes chloro, cyano, hydroxy, mésylamino, acétyle, hydroxycarbamoyle, butylcarbamoyle, t-butylcarbamoyle, octylcarbamoyle, dodécylcarbamoyle, éthoxycarbonyle, butoxycarbonyle, t-butoxycarbonyle, octyloxycarbonyle, dodécyloxycarbonyle, hexadécyloxycarbonyle, méthyle, t-butyle, hydroxyméthyle, 3-hydroxypropyle, t-butoxycarbonylaminométhyle, aminométhyle, carboxyméthyle et hydroxyiminométhyle ou un groupe pyridino substiuté par un groupe amino.

11. Composé selon la revendication 10, choisi dans le groupe comprenant:
le 7-[2-éthoxyimino-2-(5-amino-1,2,4-thiadiazol-3-yl)-acétamido]-3-(3-hydroxyméthyl-1-pyridinométhyl)-3-céphème-4-carboxylate (isomère syn),
le 7-[2-éthoxyimino-2-(5-amino-1,2,4-thiadiazol-3-yl)-acétamido]-3-(4-hydroxyméthyl-1-pyridinométhyl)-3-céphème-4-carboxylate (isomère syn),
le 7-[2-éthoxyimino-2-(5-amino-1,2,4-thiadiazol-3-yl)-acétamido]-3-(4-cyano-1-pyridinométhyl)-3-céphème-4-carboxylate (isomère syn),
le diformiate du 7-[2-éthoxyimino-2-(5-amino-1,2,4-thiadiazol-3-yl)-acétamido]-3-(4-aminométhyl-1-pyridinométhyl)-3-céphème-4-carboxylate (isomère syn),
le 7-[2-éthoxyimino-2-(5-amino-1,2,4-thiadiazol-3-yl)-acétamido]-3-(3-chloro-1-pyridinométhyl)-3-céphème-4-carboxylate (isomère syn),
le 7-[2-éthoxyimino-2-(5-amino-1,2,4-thiadiazol-3-yl)-acétamido]-3-(3,5-diméthyle-1-pyridinométhyl)-3-céphème-4-carboxylate (isomère syn),
le 7-[2-éthoxyimino-2-(5-amino-1,2,4-thiadiazol-3-yl)-acétamido]-3-(4-t-butyl-1-pyridinométhyl)-3-céphème-4-carboxylate (isomère syn),
le 7-[2-éthoxyimino-2-(5-amino-1,2,4-thiadiazol-3-yl)-acétamido]-3-(3-méthyl-1-pyridinométhyl)-3-céphème-4-carboxylate (isomère syn),
le 7-[2-éthoxyimino-2-(5-amino-1,2,4-thiadiazol-3-yl)-acétamido]-3-(3-hydroxy-1-pyridinométhyl)-3-céphème-4-carboxylate (isomère syn),
le 7-[2-éthoxyimino-2-(5-amino-1,2,4-thiadiazol-3-yl)-acétamido]-3-[4-(3-hydroxypropyl)-1-pyridinométhyl)-3-céphème-4-carboxylate (isomère syn),
le 7-[2-éthoxyimino-2-(5-amino-1,2,4-thiadiazol-3-yl)-acétamido]-3-(4-dodécyloxycarbonyl-1-pyridinométhyl)-3-céphème-4-carboxylate (isomère syn),
le 7-[2-éthoxyimino-2-(5-amino-1,2,4-thiadiazol-3-yl)-acétamido]-3-[4-(N-hydroxycarbamoyl)-1-pyridinométhyl)-3-céphème-4-carboxylate (isomère syn),
le 7-[2-éthoxyimino-2-(5-amino-1,2,4-thiadiazol-3-yl)-acétamido]-3-(4-butoxycarbonyl-1-pyridinométhyl)-3-céphème-4-carboxylate (isomère syn),
le 7-[2-éthoxyimino-2-(5-amino-1,2,4-thiadiazol-3-yl)-acétamido]-3-(4-t-butoxycarbonyl-1-pyridinométhyl)-3-céphème-4-carboxylate (isomère syn),
le 7-[2-éthoxyimino-2-(5-amino-1,2,4-thiadiazol-3-yl)-acétamido]-3-[4-(N-butoxycarbamoyl)-1-pyridinométhyl)-3-céphème-4-carboxylate (isomère syn),
le 7-[2-éthoxyimino-2-(5-amino-1,2,4-thiadiazol-3-yl)-acétamido]-3-[4-(N-t-butylcarbamoyl)-1-pyridinométhyl)-3-céphème-4-carboxylate (isomère syn),
le 7-[2-éthoxyimino-2-(5-amino-1,2,4-thiadiazol-3-yl)-acétamido]-3-[4-(N-dodécylcarbamoyl)-1-pyridinométhyl)-3-céphème-4-carboxylate (isomère syn),
le 7-[2-éthoxyimino-2-(5-amino-1,2,4-thiadiazol-3-yl)-acétamido]-3-(3-amino-1-pyridinométhyl)-3-céphème-4-carboxylate (isomère syn),
le 7-[2-éthoxyimino-2-(5-amino-1,2,4-thiadiazol-3-yl)-acétamido]-3-(4-octyloxycarbonyl-1-pyridinométhyl)-3-céphème-4-carboxylate (isomère syn),
le 7-[2-éthoxyimino-2-(5-amino-1,2,4-thiadiazol-3-yl)-acétamido]-3-[4-(N-octylcarbamoyl)-1-pyridinométhyl)-3-céphème-4-carboxylate (isomère syn),
le 7-[2-éthoxyimino-2-(5-amino-1,2,4-thiadiazol-3-yl)-acétamido]-3-(3-mésylamino-1-pyridinométhyl)-3-céphème-4-carboxylate (isomère syn),
le 7-[2-éthoxyimino-2-(5-amino-1,2,4-thiadiazol-3-yl)-acétamido]-3-(4-éthoxycarbonyl-1-pyridinométhyl)-3-céphème-4-carboxylate (isomère syn), et
le 7-[2-éthoxyimino-2-(5-amino-1,2,4-thiadiazol-3-yl)-acétamido]-3-(4-hexadécyloxycarbonyl-1-pyridinométhyl)-3-céphème-4-carboxylate (isomère syn).

12. Composé selon la revendication 10, choisi parmi
le 7-[2-cyclopentyloxyimino-2-(5-amino-1,2,4-thiadiazol-3-yl)-acétamido]-3-(3-hydroxyméthyl-1-pyridinométhyl)-3-céphème-4-carboxylate (isomère syn),
le 7-[2-cyclopentyloxyimino-2-(5-amino-1,2,4-thiadiazol-3-yl)-acétamido]-3-(4-hydroxyméthyl-1-pyridinométhyl)-3-céphème-4-carboxylate (isomère syn),
le 7-[2-cyclopentyloxyimino-2-(5-amino-1,2,4-thiadiazol-3-yl)-acétamido]-3-(4-acétyl-1-pyridinométhyl)-3-céphème-4-carboxylate (isomère syn),
le 7-[2-cyclopentyloxyimino-2-(5-amino-1,2,4-thiadiazol-3-yl)-acétamido]-3-(3-carboxyméthyl-1-pyridinométhyl)-3-céphème-4-carboxylate (isomère syn),
le 7-[2-cyclopentyloxyimino-2-(5-amino-1,2,4-thiadiazol-3-yl)-acétamido]-3-(4-hydroxyiminométhyl-1-pyridinométhyl)-3-céphème-4-carboxylate (isomère syn),

13. Composé selon la revendication 10, choisi parmi
le 7-[2-(2-cyclopentèn-1-yloxyimino)-2-(5-amino-1,2,4-thiadiazol-3-yl)-acétamido]-3-(4-hydroxyméthyl-1-pyridinométhyl)-3-céphème-4-carboxylate (isomère syn),
le 7-[2-(2-cyclopentèn-1-yloxyimino)-2-(5-amino-1,2,4-thiadiazol-3-yl)-acétamido]-3-(3-hydroxyméthyl-1-pyridinométhyl)-3-céphème-4-carboxylate (isomère syn).

14. Composé selon la revendication 10, qui est le 7-[2-carboxyméthoxyimino)-2-(5-amino-1,2,4-thiadiazol-3-yl)-acétamido]-3-(3-hydroxyméthyl-1-pyridinométhyl)-3-céphème-4-carboxylate (isomère syn).

15. Procédé de préparation de nouveaux céphèmes répondant à la formule:

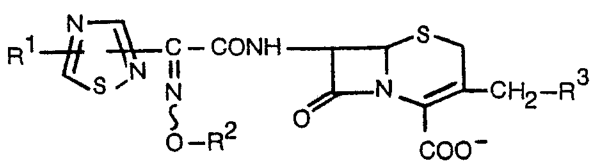

dans laquelle $R^1$, $R^2$ et $R^3$ sont chacun tels que définis dans le revendication 1, ou de leurs sels pharmaceutiquement acceptables, qui consiste à faire réagir un composé répondant à la formule:

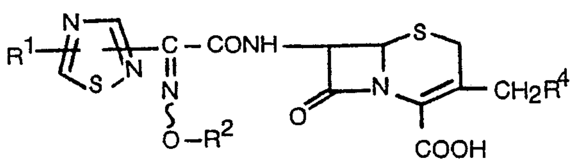

dans laquelle $R^1$ et $R^2$ sont chacun tels que définis ci-dessus; et $R^4$ est un groupe qui être remplacé par un groupe répondant à la formule: $R^3$
dans laquelle $R^3$ est tel que défini ci-dessus; ou un de ses sels, avec un composé répondant à la formule: $R^{3a}$ dans laquelle $R^{3a}$ est un thiazole qui peut être substitué avec des substituants choisis parmi des groupes alkyle en $C_1$ à $C_6$ et hydroxyalkyle en $C_1$ à $C_6$; pyridine substituée par des substituants choisis parmi de halogènes, des groupes cyano, hydroxy, amino protégé de la manière habituelle par un groupe acyle, alcanoyle en $C_1$ à $C_6$, hydroxycarbamoyle (alkyle en $C_1$ à $C_{14}$)carbamoyle, carboxy protégé, alkyle en $C_1$ à $C_6$, hydroxy(alkyle en $C_1$ à $C_6$), (amino protégé)(alkyle en $C_1$ à $C_6$), amino(alkyle en $C_1$ à $C_6$), carboxy(alkyle en $C_1$ à $C_6$) et hydroxyimino(alkyle en $C_1$ à $C_6$) ou pyridine substituée par un groupe amino, sous réserve que lorsque $R^2$ est un groupe alkyle en $C_1$ à $C_6$, $R^{3a}$ n'est pas une pyridine substituée par un groupe carboxy(alkyle en $C_1$ à $C_6$), et lorsque $R^2$ est un groupe 2-carboxyprop-2-yle, $R^{3a}$ n'est pas une 3-carboxyméthylpyridine.

16. Procédé de préparation de nouveaux céphèmes répondant à la formule:

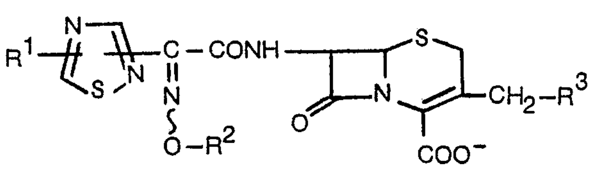

dans laquelle $R^1$, $R^2$ et $R^3$ sont chacun tels que définis dans la revendication 1, ou de leurs sels pharmaceutiquement acceptables, qui consiste à faire réagir un composé répondant à la fomule:

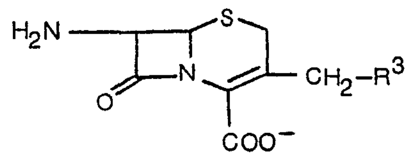

dans laquelle $R^3$ est tel que défini ci-dessus; ou son dérivé réactif sur le groupe amino ou un de ses sels, avec un composé répondant à la formule:

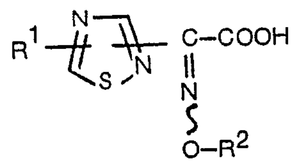

dans laquelle $R^1$ et $R^2$ sont chacun tels que définis ci-dessus; ou son dérivé réactif sur le groupe carboxy ou un de ses sels.

17. Procédé de préparation de nouveaux céphèmes répondant à la formule:

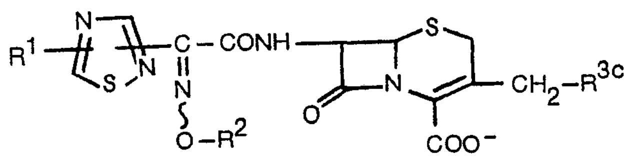

dans laquelle $R^1$ et $R^2$ sont chacun tels que définis dans la revendication 1, et $R^{3c}$ est un groupe pyridino substitué par un groupe alkyle en $C_1$ à $C_6$; ou de leurs sels pharamaceutiquement acceptables, qui consiste à soumettre un composé répondant à la formule:

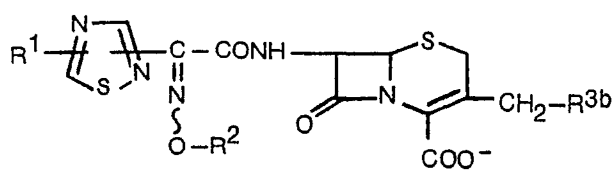

dans laquelle $R^1$ et $R^2$ sont chacun tels que définis ci-dessus et $R^{3b}$ est un groupe pyridino substitué par un groupe (amino protégé) (alkyle en $C_1$ à $C_6$), ou un de ses sels à une réaction d'élimination du groupe protecteur de l'amino.

18. Procédé de préparation de nouveaux céphèmes répondant à la formule:

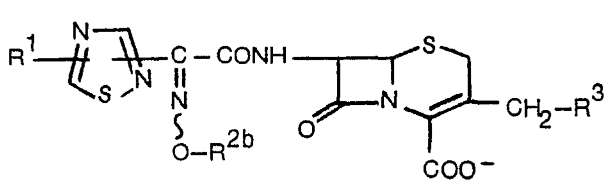

dans laquelle $R^1$ et $R^3$ sont chacun tels que définis dans la revendiction 1 et $R^{2b}$ est un groupe carboxy(alkyle en $C_1$ à $C_6$) ou de leurs sels pharmaceutiquement acceptables, qui consiste à soumettre un composé répondant à la formule:

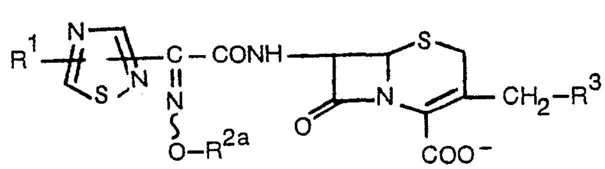

dans laquelle $R^1$ et $R^3$ sont chacun tels que définis ci-dessus et $R^{2a}$ est un groupe (carboxy protégé) (alkyle en $C_1$ à $C_6$) ou un de ses sels, à une réaction d'élimination du groupe protecteur du groupe carboxy.

19. Procédé de préparation de nouveaux céphèmes répondant à la formule:

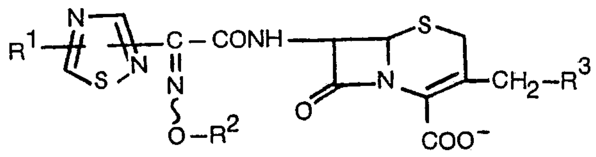

dans laquelle $R^1$, $R^2$ et $R^3$ sont chacun tels que définis dans la revendication 1, ou de leurs sels pharmaceutiquement acceptables, qui consiste à soumettre un composé répondant à la formule:

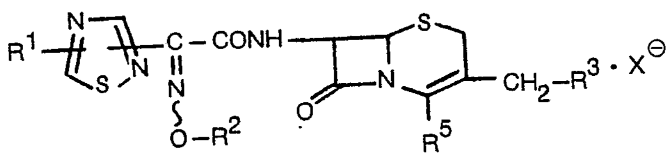

dans laquelle $R^1$, $R^2$ et $R^3$ sont chacun tels que définis ci-dessus, $R^5$ est un groupe carboxy protégé et X est un radical acide, à une réaction d'élimination du groupe protecteur du carboxy.

20. Composition pharmaceutique antibactérienne comprenant un composé selon la revendication 1 associé à un support ou excipient pratiquement non toxique, pharmaceutiquement acceptable.

21. Procédé de préparation d'une composition pharmaceutique antibactérienne qui consiste à mélanger un composé selon la revendiction 1 comme ingrédient actif avec un support inerte.

22. Composé répondant à la formule:

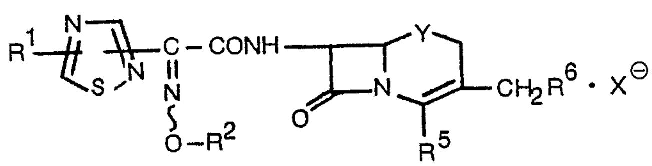

dans laquelle $R^1$ et $R^2$ sont chacun tels que définis dans la revendication 1, $R^5$ est un groupe carboxy protégé; $R^6$ est un groupe thiazolo qui peut être substitué par un ou plusieurs substituants choisis parmi des groupes alkyle en $C_1$ à $C_6$ et hydroxy(alkyle en $C_1$ à $C_6$); un groupe pyridino substitué par des substituants choisis parmi des halogènes, les groupes cyano, hydroxy, amino protégé de la manière habituelle par un groupe acyle, alcanoyle en $C_1$ à $C_6$, hydroxycarbamoyle, (alkyle en $C_1$ à $C_{14}$)carbamoyle, carboxy protégé, alkyle en $C_1$ à $C_6$, hydroxy(alkyle en $C_1$ à $C_6$), (amino protégé) (alkyle en $C_1$ à $C_6$), amino(alkyle en $C_1$ à $C_6$), carboxy (alkyle en $C_1$ à $C_6$) et hydroxyimino(alkyle en $C_1$ à $C_6$) ou un groupe pyridino substitué par un groupe amino; sous réserve que, lorsque $R^2$ est un groupe alkyle en $C_1$ à $C_6$, $R^6$ n'est pas un groupe pyridino substitué par un groupe carboxy(alkyle en $C_1$ à $C_6$) et lorsque $R^3$ est un groupe 2-carboxyprop-2-yle, $R^6$ n'est pas un groupe 3-carboxyméthylpyridino; X est un radical acide et

$$Y \text{ est } —S— \text{ ou } —\overset{\overset{O}{\uparrow}}{S}— \; ;$$

et leurs sels.

23. Procédé de préparation d'un composé répondant à la formule:

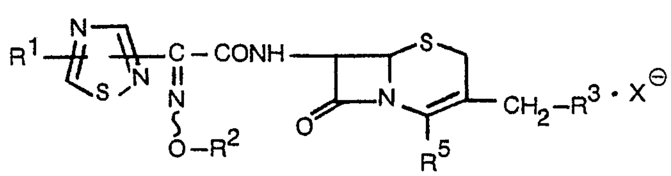

dans laquelle $R^1$, $R^2$ et $R^3$ sont chacun tels que définis dans la revendication 1; $R^5$ est un groupe carboxy protégé; et X est un radical acide; ou de ses sels; qui consiste à réduire un composé répondant à la formule:

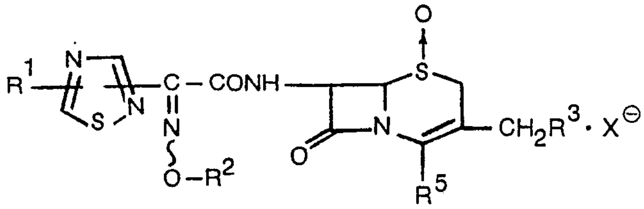

dans laquelle $R^1$, $R^2$, $R^3$, $R^5$ et X sont chacun tels que définis ci-dessus, ou un de ses sels.

24. Composé répondant à la formule de la revendication 1 pour l'utilisation comme médicament.

25. Composé répondant à la formule de la revendication 1 pour l'utilisation dans le traitement de maladies infectieuses.